# EUROPEAN PATENT APPLICATION

(11) **EP 4 606 389 A1**
(43) Date of publication of application: **27.08.2025**
(21) Application number: 25159291.1
(22) Date of filing: 21.02.2025
(51) Int. Cl.: A61K 47/60, A61P 35/00, A61P 7/00, A61P 43/00

(54) **UNIQUE PEGYLATED GRANULOCYTE COLONY STIMULATING FACTOR, METHODS OF USE AND PREPARATION THEREOF**

(30) Priority: 22.02.2024 US 202463556552 P
(71) Applicant: PharmaEssentia Corporation, Taipei 115603 (TW)
(72) Inventor: Lin, Ko-Chung, Lexington, 02421 (US); Yo, Yi-Te, 115603 Taipei (TW); Yan, Shih-Long, 115603 Taipei (TW); Lee, Shin-Jye, 115603 Taipei (TW)
(74) Representative: Hertin und Partner Rechts- und Patentanwälte PartG mbB

(57) **Abstract**

A uniquely PEGylated, stable granulocyte colony stimulating factor (G-CSF) with unexpected properties is disclosed herein. The method of making and using such GCSF are also described. The unique molecule exhibits unexpectedly longer pharmacokinetics and pharmacodynamics profile than reference standard on the market. The molecule also demonstrates safety for use.

## Description

### CROSS-REFERENCE TO RELATED APPLICATIONS

The present application claims the benefit under 35 U.S.C. § 119 to U.S. Provisional Patent Application Serial Number 63/556,552 filed on February 22, 2024, the content of which are incorporated herein by reference in its entirety.

### SEQUENCE LISTING

This application is being filed electronically via EFS-Web and includes an electronically submitted sequence listing in .xml format in ST.26 format. The sequence listing contained in this .xml file is part of the specification and is herein incorporated by reference in its entirety.

### TECHNICAL FIELD

The disclosure relates to the field of biomedicine, in particular to a unique, stable pegylated granulocyte colony stimulating factor (G-CSF) with unexpected properties.

### BACKGROUND

Patients with certain cancers may be given drugs, chemotherapy, or radiation to treat their disease. A side effect of many chemotherapy drugs and/or radiation is destruction of or delay in making immune cells that fight infection. These cells are known as white blood cells, neutrophils, or granulocytes. Neutropenia means a lack of granulocytes (infection-fighting cells). People being treated for cancer may develop neutropenia and/or fever. When this happens, treatment with antibiotics and other drugs to alleviate neutropenia is often necessary in case there is an infection.

Granulocyte colony stimulating factor ("G-CSF", "GCSF", or "filgrastim") is a molecule known in the art to assist, treat, and/or alleviate the above-mentioned issues. It is a glycoprotein which stimulates the survival, proliferation, differentiation and function of neutrophil granulocyte progenitor cells and mature neutrophils.

The human form of G-CSF was cloned by groups from Japan and the U.S.A. in 1986. The natural human glycoprotein exists in two forms, one of 175 and the other of 178 amino acids. The more abundant and more active 175 amino acid form has been used in the development of pharmaceutical products by recombinant DNA technology. A recombinant version of human G-CSF is known to be synthesized in an E. coli expression system and is called filgrastim. Nonetheless, GSCF's half-life is not long.

The attachment of synthetic polymers to the peptide backbone of filgrastim is known in the art. An exemplary polymer that has been conjugated to peptides is poly(ethylene glycol) ("PEG"). The attachment of PEG to G-CSF is known as "pegfilgrastim".

Pegfilgrastim is marketed by Amgen as NEULASTA (trademarked). At the time of filing of this disclosure, there are about 7 approved pegfilgrastim biosimilars by the U.S. Federal Drug and Food Administration (FDA). Namely, FULPHILA (Trademarked, also known as pegfilgrastim-jmdb), FYLNETRA (Trademarked, also known as pegfilgrastim-pbbk), NYVEPRIA (Trademarked, also known as pegfilgrastim-apgf), STIMUFEND (Trademarked, also known as pegfilgrastim-fpgk), UDENYCA (Trademarked, also known as pegfilgrastim-cbqv), and ZIEXTENZO (Trademarked, also known as pegfilgrastim-BMEZ), with NEULASTA being the original "name brand" drug in the market, and the remaining being mostly biosimilars.

However, NEULASTA and/or other pegfilgrastim biosimilars still suffer many drawbacks, especially in the molecular half-life requiring frequent administration to the patient which leads to patient adherence issues and high cost. Other drawbacks are that these biosimilars still cause, relate, and/or associate with major patient side effects. Examples include, but are not limited to: splenic rupture, inability to be administered in the period between 14 days before and 24 hours after administration of cytotoxic chemotherapy, potential effect on malignant cells, increased risk of MDS and AML in breast and lung cancer patients when used in conjunction with chemotherapy and/or radiotherapy, aortitis, capillary leak syndrome, sickle cell crises, leukocytosis, thrombocytopenia, immunogenicity adverse reactions, glomerulonephritis, and/or acute respiratory distress syndrome.

### SUMMARY

In an aspect, the present disclosure relates to a substantially homogenous composition comprising formula I which includes the following structure. 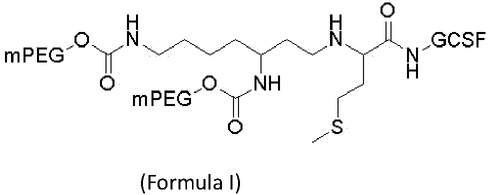 wherein each methoxy poly(ethylene glycol) includes a molecular weight of between about 19-23KDa; and wherein said composition includes a mixture of D- and L- form isomers.

In another aspect, the substantially homogenous composition mixture can include at least about 95%, 98%, 99%, 99.5%, or 100% of D- form isomers.

In one aspect, the present disclosure also relates to a pharmaceutical formulation comprising the substantially homogenous composition according to formula I, and/or any chiral mixture in any proportion thereof; and a pharmaceutically acceptable carrier.

In one aspect, the present disclosure relates to a method of increasing the white cell count in a subject or a subject in need, or preventing, alleviating or treating a condition in a subject or subject in need thereof, wherein the condition includes compromised white blood cell production in said subject or said subject in need, the method comprising administering an effective amount of the substantially homogenous composition or pharmaceutical formulation according to formula I, and/or any chiral mixture in any proportion thereof, and/or the pharmaceutical formulation as described above to the subject or the subject in need. In an aspect, the subject or a subject in need is at risk of and/or is suffering from neutropenia and/or being treated with an agent, and/or is undergoing radiation and/or chemotherapy treatment that decreases its white blood cell count.

The method described above, in an aspect, the radiation and/or chemotherapy treatment and administration of the substantially homogenous composition or the pharmaceutical formulation occurs on the same day or about the same time.

In another aspect, the subject or the subject in need described above has decreased endogenous levels of GCSF and/or is suffering from diseases including lung cancer, lymphoma, breast cancer, bone marrow transplantation, testicular cancer, AIDS-related malignancies, myleodysplastic disorders, myeloid cancer, acute leukemia, congenital and cyclic neutropenias, aplastic anemia, head and neck cancer, lung cancer, stomach cancer, colon cancer, pancreatic cancer, prostate cancer, breast cancer, kidney cancer, bladder cancer, ovary cancer, cervical cancer, melanoma, glioblastoma, myeloid leukemia, myeloma, lymphoma, leukemia, chronic and/or severe neutropenia, severe congenital neutropenia, cyclic neutropenia, febrile neutropenia, and/or severe combined neutropenia.

Yet in another aspect, any of the substantially homogenous composition and/or the pharmaceutical formulation described above can be administered subcutaneously.

In an aspect, the present disclosure relates to a method of treating a hematopoietic disorder in a subject or a subject in need, wherein the method comprises administering a therapeutically effective dose according to any of the substantially homogenous composition or pharmaceutical formulation to the subject or the subject in need, wherein the treatment (a) alleviates, slows down, lessen symptoms of, and/or halts progression of the subject or the subject in need for the hematopoietic disorder including chronic, severe neutropenia, severe congenital neutropenia, cyclic neutropenia, febrile neutropenia, and/or severe combined neutropenia, as compared to other pharmaceutical drugs; and/or alleviates, slows down, lessen symptoms of, and/or halts progression of the subject or the subject in need who is at risk of and/or is suffering from neutropenia, as compared to other pharmaceutical drugs.

In an aspect, the method described above, wherein said any of the substantially homogenous composition or pharmaceutical formulation exhibits more potent efficacy of (a) alleviating, slowing down, lessening symptoms of, and/or halting progression of the hematopoietic disorder in the subject or the subject in need, as compared to other pharmaceutical drugs; and/or (b) alleviates, slows down, lessen symptoms of, and/or halts progression of the subject or the subject in need who is at risk of and/or is suffering from neutropenia, as compared to other pharmaceutical drugs. Some non-limiting other pharmaceutical drugs can include filgrastim, pegfilgrastim, pegfilgrastim-jmdb, pegfilgrastim-pbbk, pegfilgrastim-apgf, pegfilgrastim-cbqv, pegfilgrastim-bmez, pegfilgrastim-fpgk, and/or eflapegrastim-xnst.

In another aspect, the present disclosure relates to a method for preparing a mPEG-MetHuG-CSF having the chemical Formula I I, wherein the method comprises: attaching a branched aldehyde polymer including a pair of methoxy poly(ethylene glycol) (mPEG) covalently linked to the N-terminus of a MetHuG-CSF using amine linkage, wherein each mPEG includes a molecular weight between about 19 to about 23 KDa, thereby obtaining mPEG-MetHuG-CSF, wherein mPEG-MetHuG-CSF exhibits improved pharmacodynamics, pharmacokinetics, and/or toxicokinetic profile in in vitro, in vivo, and/or ex vivo as compared to filgrastim, pegfilgrastim, and/or their biosimilars. Some non-limiting biosimilars can include pegfilgrastim-jmdb, pegfilgrastim-pbbk, pegfilgrastim-apgf, pegfilgrastim-cbqv, pegfilgrastim-bmez, pegfilgrastim-fpgk, and/or eflapegrastim-xnst. Additionally, or alternatively, each mPEG includes a molecular weight between about 19-23 KDa. The step of attaching can further include covalently coupling the branched meoxyl polyalkylene glycol to the N terminus of MetHuG-CSF under reducing alkylation conditions at a pH sufficiently acidic to activate the carbonyl group at the N terminus, thereby forming an amine bond; and the step further optionally comprises purifying and/or separating the mPEG-MetHuG-CSF from an unreacted MetHuG-CSF or any impurities.

Yet in an aspect, the present disclosure relates to a method of treating chemotherapy induced thrombocytopenia and/or chemotherapy induced neutropenia in a subject or a subject in need, wherein the method comprises administering a therapeutically effective dose according to any of the substantially homogenous composition or pharmaceutical formulation described above to the subject or the subject in need. In an aspect, said subject or said subject in need is suffering from myelosuppressive chemotherapy induced thrombocytopenia and/or chemotherapy induced neutropenia.

In an aspect, the present disclosure relates to the use of any of the substantially homogenous composition or pharmaceutical formulation described above for treating chemotherapy induced thrombocytopenia and/or chemotherapy induced neutropenia in a subject or a subject in need, and/or the use of any of an effective amount of the substantially homogenous composition or pharmaceutical formulation described above for treating chemotherapy induced thrombocytopenia and/or chemotherapy induced neutropenia in a subject or a subject in need,. In an aspect, said subject or said subject in need is suffering from myelosuppressive chemotherapy induced thrombocytopenia and/or chemotherapy induced neutropenia.

In an aspect, the myelosuppressive chemotherapy induced thrombocytopenia can be caused by a drug or any combination of drugs including Ziv-aflibercept, Brentuximab vedotin, Pralatrexate, Ganciclovir, Valganciclovir, Romidepsin, Ruxolitinib, Decitabine, Imatinib, Topotecan, Lenalidomide, Irinotecan, Interferons, Phenylhydrazine, Tamoxifen, Lipopolysaccharide, Anthracycline antibiotics, daunorubicin, doxorubicin, Gemcitabine, Cytoxan, Paclitaxel, Alkylating antineoplastic agent, DNA intercalating agent, Alkylating agent, bendamustin, mustard, Topoisomerase inhibitor, Bortezomib, Temsirolimus, Vorinostat, Ifosfamide, and/or Ixabepilone. In another aspect, the subject or the subject in need is receiving or about to receive a chemotherapeutic agent comprising one or more of cyclophosphamide, doxorubicin, etoposide, ifosfamide, mesna, cisplatin, gemcitabine, tamoxifen and/or lenalidomide. Additionally, or alternatively, the subject or the subject in need has multiple myeloma, chronic myelogenous leukemia (CML), acute myeloid leukemia, and/or myelodysplastic syndrome.

In one aspect, the present disclosure relates to uses of any of the substantially homogenous composition or pharmaceutical formulation described above for the preparation of a medicament for the treatment of one or more conditions including a subject or a subject in need having compromised white blood cell production, decreases its white blood cell count, decreased endogenous levels of GCSF, neutropenia, chronic and/or severe neutropenia, severe congenital neutropenia, cyclic neutropenia, febrile neutropenia, severe combined neutropenia, myelosuppressive chemotherapy induced thrombocytopenia, chemotherapy induced neutropenia, chemically induced neutropenia, multiple myeloma, chronic myelogenous leukemia (CML), acute myeloid leukemia, myelodysplastic syndrome, and/or alcoholic hepatitis. In an aspect, the one or more conditions can be induced by a cancer therapy or another condition, wherein said cancer of cancer therapy or another condition comprises lung cancer, lymphoma, breast cancer, hematopoietic stem cell transplantation, bone marrow transplantation, testicular cancer, AIDS-related malignancies, myleodysplastic disorders, myeloid cancer, acute leukemia, aplastic anemia, head and neck cancer, lung cancer, stomach cancer, colon cancer, pancreatic cancer, prostate cancer, breast cancer, kidney cancer, bladder cancer, ovary cancer, cervical cancer, melanoma, glioblastoma, myeloid leukemia, myeloma, lymphoma, leukemia, myelosuppressive chemotherapy, acute myeloid leukemia, bone marrow transplant, progenitor cell collection, and/or acute radiation syndrome.

Yet in another aspect, the present disclosure relates to any of the substantially homogenous composition or pharmaceutical formulation described above, characterized in that it is used to treating one or more of condition including a subject or a subject in need having compromised white blood cell production, decreases its white blood cell count, decreased endogenous levels of GCSF, neutropenia, chronic and/or severe neutropenia, severe congenital neutropenia, cyclic neutropenia, febrile neutropenia, severe combined neutropenia, myelosuppressive chemotherapy induced thrombocytopenia, chemotherapy induced neutropenia, chemically induced neutropenia, multiple myeloma, chronic myelogenous leukemia (CML), acute myeloid leukemia, myelodysplastic syndrome, and/or alcoholic hepatitis. In an aspect, the one or more of conditions can be induced by a cancer therapy and/or another condition, wherein said cancer of cancer therapy or another condition includes lung cancer, lymphoma, breast cancer, hematopoietic stem cell transplantation, bone marrow transplantation, testicular cancer, AIDS-related malignancies, myleodysplastic disorders, myeloid cancer, acute leukemia, aplastic anemia, head and neck cancer, lung cancer, stomach cancer, colon cancer, pancreatic cancer, prostate cancer, breast cancer, kidney cancer, bladder cancer, ovary cancer, cervical cancer, melanoma, glioblastoma, myeloid leukemia, myeloma, lymphoma, leukemia, myelosuppressive chemotherapy, acute myeloid leukemia, bone marrow transplant, progenitor cell collection, and/or acute radiation syndrome.

### BRIEF DESCRIPTION OF FIGURES

For a more complete understanding of the features and advantages of the present disclosure, reference is now made to the detailed description of the disclosure along with the accompanying figures and in which:
Figure 1 denotes the molecule P2203 (mPEG-MetHuG-CSF) which is uniquely (methoxy)PEGylated. In this Figure, the mPEG is in a branched format with a linker, and the average molecular weight of each mPEG is between about 19-23KDa. The covalent attachment is located at N-terminus of MetHuG-CSF via methionine. The first amino acid Met is clearly denoted.
Figure 2(a) denotes the amino acid sequence of MetHuG-CSF as used in P2203 having 175 amino acids. Figure 2(b) denotes the amino acid sequence of human GCSF with 174 amino acids which has different chemical structure and amino acid sequence. Figure 2(c) is a DNA sequence encoding MetHuG-CSF.
Figure 3(a) denotes P2203 and its two chiral centers labelled with asterisks. Figure 3(b) denotes the L-form of P2203.
Figure 4(a) denotes comparison of PK study in rats between P2203 and Neulasta (trademarked). Figure 4(b) denotes comparison of PD study in rats between P2203 and Neulasta (trademarked). Figure 4(c) denotes comparison of PK study in monkeys between P2203 and Neulasta (trademarked) at 300 µg/kg. Figure 4(d) denotes comparison of PD study in monkeys between P2203 and Neulasta (trademarked) at 300 µg/kg.
Figure 5(a) denotes various CEX-HPLC analysis of mPEG-MetHuG-CSF. Figure 5(b) denotes SE-HPLC analysis of mPEG-MetHuG-CSF. Figure 5(c) denotes various RP-HPLC analysis of mPEG-MetHuG-CSF.
Figure 6 denotes mass spectrum of mPEG-MetHuG-CSF.
Figure 7 denotes gel purification and analysis of non-reduced mPEG-MetHuG-CSF using silver stain technique. Sample details per each lane are described in the Examples section.
Figure 8 denotes gel purification and analysis of reduced mPEG-MetHuG-CSF using silver stain technique. Sample details per each lane are described in the Examples section.
Figure 9(a) denotes rats PD of MetHuG-CSF, mPEG-MetHuG-CSF (P2203), and Pegilgrastim (Neulasta^{®}) for WBC (white blood cell). Figure 9(b) denotes PD comparison of MetHuG-CSF, mPEG-MetHuG-CSF (P2203), and Pegilgrastim (Neulasta^{®}) for ANC (absolute neutrophil count).
Figure 10 denotes the mean plasma concentration-time profiles of MetHuG-CSF, Pegfilgrastim, and mPEG-MetHuG-CSF in rats after intravenous administration (n=5, except for mPEG-MetHuG-CSF 1000mcg/mL, n=10).
Figure 11 denotes the mean log plasma concentration-time profiles of all test formulation of mPEG-MetHuG-CSF in rats after intravenous administration (n=5, except for mPEG-MetHuG-CSF 1000mcg/mL n=10).
Figure 12 denotes mean concentration in ng/mL of P2203 and Neulasta^{®} vs. time in Monkey Serum.
Figure 13(a) denotes combined male and female P2203 mean serum concentrations (ng/mL) vs time (hr) in combined male and female monkeys following subcutaneous injection on Day 1. Figure 13(b) denotes combined male and female P2203 mean serum concentrations (ng/mL) vs time (hr) in combined male and female monkeys following subcutaneous injection on and Day 22.
Figure 14 denotes a human subject clinical study with 3 cohorts.

### DETAIL DESCRIPTIONS

While the making and using of various embodiments of the present disclosure are discussed in detail below, it should be appreciated that the present disclosure provides many applicable inventive concepts that can be embodied in a wide variety of specific contexts. The specific embodiments discussed herein are merely illustrative of specific ways to make and use the disclosure and do not delimit the scope of the disclosure.

To facilitate the understanding of this disclosure, a number of terms are defined below. Terms defined herein have meanings as commonly understood by a person of ordinary skill in the areas relevant to the present disclosure. Terms such as "a", "an" and "the" are not intended to refer to only a singular entity but include the general class of which a specific example may be used for illustration. The terminology herein is used to describe specific embodiments of the disclosure, but their usage does not delimit the disclosure, except as outlined in the claims.

The following terms, unless otherwise indicated, shall be understood to have the following meanings.

As used herein, "administering," means oral administration, administration as a suppository, topical contact, intravenous, intraperitoneal, intramuscular, intralesional, intranasal or subcutaneous administration, or the implantation of a slow-release device e.g., a mini-osmotic pump, to the subject or the subject in need. Administration is by any route including parenteral, and transmucosal (e.g., oral, nasal, vaginal, rectal, or transdermal). Parenteral administration includes, e.g., intravenous, intramuscular, intra-arteriole, intradermal, subcutaneous, intraperitoneal, intraventricular, and intracranial. Moreover, where injection is to treat a tumor, e.g., induce apoptosis, administration may be directly to the tumor and/or into tissues surrounding the tumor. Other modes of delivery include, but are not limited to, the use of liposomal formulations, intravenous infusion, transdermal patches, etc.

As used herein, the term "isolated molecule" refers to a molecule (where the molecule is, for example, a polypeptide, a polynucleotide, or an antibody) that by virtue of its origin or source of derivation (1) is not associated with naturally associated components that accompany it in its native state, (2) is substantially free of other molecules from the same source, e.g., species, cell from which it is expressed, library, etc., (3) is expressed by a cell from a different species, or (4) does not occur in nature. Thus, a molecule that is chemically synthesized, or expressed in a cellular system different from the system from which it naturally originates, will be "isolated" from its naturally associated components. A molecule also can be rendered substantially free of naturally associated components by isolation, using purification techniques well known in the art. Molecule purity or homogeneity may be assayed by a number of methods known in the art. For example, the purity of a polypeptide sample may be assayed using polyacrylamide gel electrophoresis and staining of the gel to visualize the polypeptide using techniques known in the art. For certain purposes, higher resolution may be provided by using HPLC or other means known in the art for purification.

As used herein, the terms "treating" or "treatment" or "to treat" or "alleviating" or "to alleviate" can be used interchangeably and refer to (1) therapeutic measures that cure, slow down, lessen symptoms of, and/or halt progression of a diagnosed pathologic condition or disorder and/or (2) prophylactic or preventative measures that prevent and/or slow the development of a targeted pathologic condition or disorder. Thus, those in need of treatment include those already with the disorder; those prone to have the disorder; and those in whom the disorder is to be prevented. In certain aspects, a subject or a subject in need is successfully "treated" according to the methods and molecules of the present disclosure if the patient shows, e.g., total, partial, or transient remission of a certain type of disorder. One ordinary skilled in the art realizes that a treatment may improve a disorder condition but may not be a complete cure for the disorder.

As used herein, the term "substantially homogenous" means that the only (branched) polymer/protein conjugate molecules observed are those having one polymer moiety, although the preparation can also contain unreacted (i.e., lacking polymer moiety) protein. Typically, the (branched) polymer is linked to the N-terminus of the protein, and the N-terminally branched mPEG pegylation can be at least about 90%, at least about 91%, at least about 92%, at least about 93%, at least about 94%, at least about 95%, at least about 96%, at least about 97%, at least about 98%, at least about 99%, and/or at least about 99.5% of the final preparation or more, and the present disclosure denotes those which are homogenous to display the unique advantages of clinical application pharmacological properties (e.g., PK/PD). As known in the art, a mixture of polymer/protein conjugate molecules with various numbers of polymer moieties attached (i.e., di-, tri-, tetra-, etc.) and combine with the mono-polymer/protein conjugate material which can cause shorter half-life and/or more severe side effects to a subject or a subject in need. The purity of the peptide conjugates is typically determined by one or more methods known to those of skill in the art, e.g., liquid chromatography-mass spectrometry (LC-MS), matrix assisted laser desorption mass time of flight spectrometry (MALDI-TOF), capillary electrophoresis, and/or the like.

The terms "polypeptide", "peptide" and "protein" are used interchangeably herein to refer to chains of amino acids of any length. The chain may be linear or branched, it may comprise modified amino acids, and/or may be interrupted by non-amino acids. The terms also encompass an amino acid chain that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art. It is understood that the polypeptides can occur as single chains or associated chains.

The terms "subject" and "individual" are used interchangeably herein, referring to an animal, including humans and non-human animals such as rats, mice, rabbits, sheep, horses, cats, dogs, cows, pigs, non-human primates, and non-mammals. A subject may be healthy, free from a condition or disorder, or suffering from a condition or disorder.

The term "patient" or "subject in need" are used interchangeably and refer to an individual suffering from or prone to a condition that can be prevented or treated by administration of a molecule such as P2203 as provided herein.

As used herein, "branched" in reference to the geometry or overall structure of a polymer can refer to a polymer having 2 or more arms.

A "host cell" includes an individual cell or cell culture that can be or has been a recipient for vector(s) for incorporation of polynucleotide inserts. Host cells include progeny of a single host cell, and the progeny may not necessarily be completely identical (in morphology or in genomic DNA complement) to the original parent cell due to natural, accidental, or deliberate mutation. Host cells include cells transfected in vivo with a polynucleotide(s) of this disclosure. One example is *E. Coli.*

As used herein, any concentration range, percentage range, ratio range or integer range is to be understood to include the value of any integer as indicated *per se,* as well as within the recited range and, when appropriate, fractions thereof (such as one tenth and one hundredth of an integer), unless otherwise indicated.

As used herein, an "effective dosage" or "effective amount" of drug, compound, or pharmaceutical composition is an amount sufficient to affect any one or more beneficial or desired results. In more specific aspects, an effective amount prevents, alleviates, ameliorates symptoms of disease, and/or prolongs the survival of the subject or the subject in need being treated. For prophylactic use, beneficial or desired results include, but not limited to eliminating or reducing the risk, lessening the severity, or delaying the outset of the disease, including biochemical, histological and/or behavioral symptoms of the disease, its complications and intermediate pathological phenotypes presenting during development of the disease. For therapeutic use, beneficial or desired results include, but not limited to clinical results such as reducing one or more symptoms of a disease such as cancer including, for example without limitation, neutropenia caused by typical treatment of cancer, decreasing the dose of other medications required to treat the cancer and/or neutropenia disease, enhancing the effect of another medication, and/or delaying the progression of the cancer/neutropenia in patients. An effective dosage can be administered in one or more administrations. For purposes of this disclosure, an effective dosage of drug, compound, or pharmaceutical composition is an amount sufficient to accomplish prophylactic or therapeutic treatment either directly or indirectly. As is understood in the clinical context, an effective dosage of a drug, compound, or pharmaceutical composition may or may not be achieved in conjunction with another drug, compound, or pharmaceutical composition. Thus, an "effective dosage" or "effective amount" may be considered in the context of administering one or more therapeutic agents, and a single agent may be considered to be given in an effective amount if, in conjunction with one or more other agents, a desirable result may be or is achieved.

As used herein, the term "per dose", "dosage", or "dose" means administering a given numeric amount of drug to a subject or a subject in need. Per dose can be administered in separate injections at about the same time and/or different time, so long as a subject or a subject in need receives the drug amount.

As used herein, the term "per injection" means administering the entire numeric amount of drug to a subject or a subject in need for a given dose in a single injection.

As used herein, "substantially" or "substantially all" means more than about 50%, for example, about 60%, 70%, 80%, 90% ,92.5%, 95%, 97.5%, 98%, 99%, 99.5%, 99.95% or 100% of the amount, purity, or sequence identity of a molecule, depending on the context.

As used herein, "vector" or "expression vector" means a construct, which is capable of delivering, and expressing one or more gene(s) or sequence(s) of interest in a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells.

As used herein, the term "substantially homologous" can also mean that the component or molecule can be at least about 90%, at least about 91%, at least about 92%, at least about 93%, at least about 94%, at least about 95%, at least about 96%, at least about 97%, at least about 98%, at least about 99%, and/or at least about 99.5% of the sequence of a peptide component or molecule.

The term "PEG" generally refers to a polyalkylene glycol compound or derivative thereof, with or without linkers or activating moieties. The term PEG as used herein includes, but is not limited to, polyethylene glycol homopolymers, copolymers of ethylene glycol with propylene glycol and derivatives and equivalents thereof, wherein said homopolymers and copolymers are unsubstituted or substituted, for example, at one end with an alkyl group. The PEG polymers for use with the present disclosure can be linear, branched, comb, and/or star-shaped with a wide range of molecular weights. The average molecular weight of the PEG for use with embodiments of the present disclosure can range from about 5 to about 100 kDa. Fpr example, about 5 to about 90 kDa, about 5 to about 80 kDa, about 5 to about 70 kDa, about 5 to about 60 kDa, about 10 to about 50 kDa, about 15 to about 40 kDa, about 15 to about 40 kDa, about 15 to about 30 kDa, about 19 to about 30 kDa, and/or about 19 to about 23 kDa.The term "mPEG" refers to the methoxy version of any of the above wherein PEG is capped with a methoxy group at the end.

As used herein, "pharmaceutically acceptable carrier" or "pharmaceutical acceptable excipient" includes any material which, when combined with an active ingredient, allows the ingredient to retain biological activity or stability and is non-reactive with the subject's or a subject in need's immune system. Examples include, but are not limited to, any of the standard pharmaceutical carriers such as a phosphate buffered saline solution, water, emulsions such as oil/water emulsion, and various types of wetting agents. Preferred diluents for aerosol or parenteral administration are phosphate buffered saline (PBS) or normal (0.9%) saline. Compositions comprising such carriers are formulated by known methods.

The term "half-life" or "t½", as used herein in the context of administering a peptide-polymer drug to a patient, is defined as the time required for plasma concentration of a drug in a subject or a subject in need to be reduced by one half. There may be more than one half-life associated with the peptide-polymer drug depending on clearance mechanisms, redistribution, and other mechanisms known in the art.

The term "intradermal administration", "i.d.", or "administered intradermally," in the context of administering a substance to a mammal including a human, refers to the delivery of the substance into the dermis layer of the skin of the mammal. The skin of a mammal is composed of an epidermis layer, a dermis layer, and a subcutaneous layer. The epidermis is the outer layer of the skin. The dermis, which is the middle layer of the skin, contains nerve endings, sweat glands and oil (sebaceous) glands, hair follicles, and blood vessels. The subcutaneous layer is made up of fat and connective tissue that houses larger blood vessels and nerves. In contrast with intradermal administration, "s.c.", or "subcutaneous administration", refers to the administration of a substance into the subcutaneous layer, and "topical administration" refers to the administration of a substance onto the surface of the skin.

The term "preventing" or "prevent" refers to (a) keeping a disorder from occurring or (b) delaying the onset of a disorder or onset of symptoms of a disorder such as cancer and/or neutropenia.

As used herein, "about" means within an acceptable error range for the particular value as determined by one of ordinary skill in the art, which will depend in part on how the value is measured or determined, i.e., the limitations of the measurement system. For example, "about" can mean within 1 or more than 1 standard deviation per the practice in the art. Alternatively, "about" can mean a range of up to 5%, 7.5%, 10%, 12.5%, 15%, 17.55, 20%, 22.5% 25%, 27.5%, 30%, 32.5%, 35%, 37.5%, or 40% of difference in either direction (positive or negative) compared to a reference value. Furthermore, particularly with respect to biological systems or processes, the terms can mean up to an order of magnitude or up to 1, 2, 3, 4, or 5-folds of a value. When particular values are provided in the application and claims, unless otherwise stated, the meaning of "about" should be assumed to be within an acceptable error range for that particular value. Reference to "about" a value or parameter herein includes (and describes) embodiments that are directed to that value or parameter *per se.* For example, description referring to "about X" includes description of "X". Numeric ranges are inclusive of the numbers defining the range.

The term "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

As used herein, "ADME" is an abbreviation in pharmacokinetics and pharmacology for "absorption, distribution, metabolism, and excretion", and describes the disposition of a pharmaceutical compound within an organism.

As used herein, "pharmacodynamics", or "PD" are used interchangeably. Pharmacodynamics is the study of the action of a drug on the organism. Pharmacodynamics describes the interaction of drugs with target tissues.

As used herein, "pharmacokinetics", or "PK" are used interchangeably. Pharmacokinetics is the study of the effect an organism has on a drug. Pharmacokinetics represents the absorption, distribution, metabolism, and/or elimination of drugs from the body. Pharmacokinetics (PK) studies how the body interacts with administered substances for the entire duration of exposure (medications for the sake of this disclosure).

An "adverse event" or "AE" as used herein is any unfavorable and generally unintended, even undesirable, sign (including an abnormal laboratory finding), symptom, or disease associated with the use of a medical treatment. For example, an adverse event may be associated with activation of the immune system or expansion of immune system cells (e.g., T cells) in response to a treatment. A medical treatment may have one or more associated AEs and each AE may have the same or different level of severity. Reference to methods capable of "altering adverse events" means a treatment regime that decreases the incidence and/or severity of one or more AEs associated with the use of a different treatment regime.

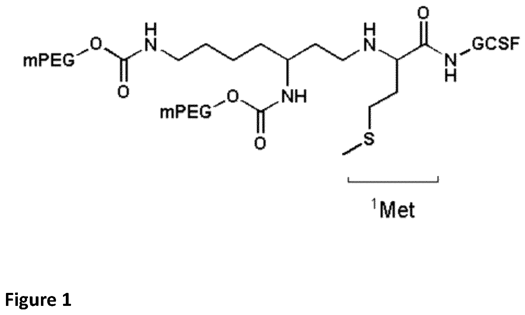

As used herein, the term "P2203", "P-2203", and "mPEG-MetHuG-CSF" are used interchangeably and refer to the present disclosure's methoxy PEGylated MetHuG-CSF with uniquely properties and the structure denoted in Figure 1.

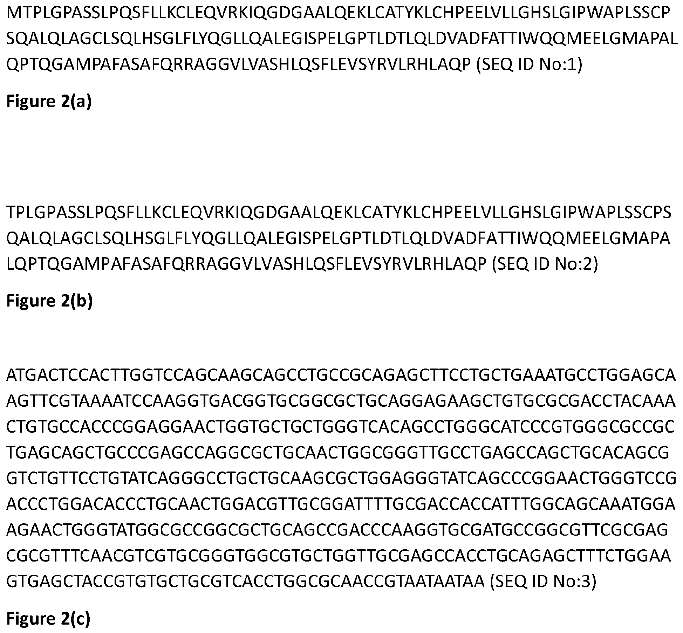

As used herein, "MetHuG-CSF" means a version of human G-CSF with 174 amino acids with an additional N-terminal Met, totaling 175 amino acids as denoted in Figure 2(a) having SEQ ID NO:1.

As used herein, "GCSF" refers to human granulocyte colony stimulating factor G-CSF with 174 amino acids as denoted in Figure 2(b) having SEQ ID NO:2.

As used herein, the terms "PEG-filgrastim" and "Pegfilgrastim" are used interchangeably and refers to commercially available PEGylated form of GCSF which is market by the company Amgen with brand name Neulasta (trademarked). The structure and property of Pegfilgrastim are known in the art.

As used herein, the term "branched aldehyde polymer" includes two or more branches of polymers linked by a linker.

### i. Peptide/Nucleotide Sequence of MetHuG-CSF

In an embodiment, the present disclosure includes a biologics molecule having 175 amino acids, and one particular example is denoted in Figure 2(a) as SEQ ID No.:1. The biologics molecule includes MetHuG-CSF and is structurally unique. In an embodiment, MetHuG-CSF is attached to a branched mPEG with 2 arms as shown in Figure 1. In particular, it is bacterially produced with an extra N-terminal methionine (Met) and is not glycosylated, and the Met is not removed. Natural G-CSF or CHO (Chinese hamster ovary) cell-derived G-CSF has 174 amino acids without the Met at the N-terminus (SEQ ID NO.:2) and is glycosylated with an O-linked glycan. More particularly human version of GCSF has an O-glycosylation site at the Thr-133 position. Typically, hGCSF (human GCSF) also has additional signal peptide at the N-terminus.

In an embodiment, MetHuG-CSF can have one or more PEG or mPEG covalently attached at the N terminus Met instead of lysine. In yet another embodiment, the PEG can have methoxy group capping at the end, and one or more PEG (e.g., mPEG) molecules can be attached to MetHuG-CSF via a branched structure such as via a linker, thereby forming mPEG-MetHuG-CSF.

The nucleotide sequences encoding the polypeptides disclosed herein can vary such as using codon optimization, if needed, and depending on which host cell is used. Such method is known in the art. The nucleotide sequence can be cloned into a vector and transfect into a host cell for MetHuG-CSF expression by methods also known in the art. One embodiment is denoted in Figure 2(c) as SEQ ID NO.:3.

### ii. Method of Making

The present disclosure also provides methods of generating and making P2203. One method is provided by this disclosure in the examples' procedures.

Briefly, and in general, vectors encoding P2203 are transfected to host cells such as *E. Coli.* Then, the host cells are first inoculated, and cultured in a fermenter. Induction is conducted with IPTG (Isopropyl β-D-1-thiogalactopyranoside) to induce protein product in the host cell, and then the cells are harvested. The cell pellet is then stored at a proper temperature for long term storage. When in need, cells can then be thawed, and the cell pellet be homogenized to obtain a crude lysate which contains the protein P2203. The crude lysate protein is then refolded and clarified to obtain crude refolded protein solution. The crude solution is then concentrated and subjected to filtration, cationic chromatography, hydrophobic interaction chromatography, and 0.2µm filter steps to obtain purified MetHuG-CSF in bulk.

In an embodiment, the pegylation procedure includes first chemically synthesizing an mPEG aldehyde. The pegylation can be accomplished by covalently attaching an aldehyde group to a methionine via an amine bond.

For example, the bulk ingredient as described can be pegylated with a branched molecule which has two of between about 19-23KDa activated methoxy polyethylene glycol aldehyde (using reducing agent and reaction buffer. The mixture can then be buffer-changed and subjected to a cationic column to remove impurities such as non-pegylated MetHuG-CSF intermediate. The purified, pegylated MetHuG-CSF and intermediate fractions can then be collected and buffer-exchanged to formulation buffer by a Tangential Flow Filtration (TFF) system to obtain purified drug substance of P2203.

In an embodiment, the present disclosure includes an mPEG size per single branch of about 5KDa, about 7.5KDa, about 10KDa, about 12.5KDa, about 15KDa, about 17.5KDa, about 20KDa, about 22.5KDa, about 25KDa, about 27.5KDa, about 30KDa, about 32.5KDa, about 35KDa, about 37.5KDa, or about 40KDa. This makes the final branched mPEG moiety with two mPEGs having about 10KDa, about 15KDa, about 20KDa, about 25KDa, about 35KDa, about 40KDa, about 45KDa, about 50KDa, about 55KDa, about 60KDa, about 65KDa, about 70KDa, about 75KDa, or about 80KDa.

### iii. Chiral Mixtures

In an embodiment, P2203 has chiral centers and/or geometric isomeric centers (E-and Z-isomers), and it is to be understood that the disclosure encompasses all such optical, diastereoisomers and geometric isomers that possess the same property and/or better efficacy with lesser side effects. It is to be understood that the P2203 of the present disclosure encompasses racemic, optically active, regioisomeric and stereoisomeric forms, or combinations thereof that possess the therapeutically useful properties described herein. Preparation of optically active forms is achieved in any known manner, including as non-limiting examples, by resolution of the racemic form by recrystallization techniques, by synthesis from optically active starting materials, by chiral synthesis, or by chromatographic separation using a chiral stationary phase. In some embodiments, mixtures of one or more isomers are utilized as the therapeutic compound described herein. In certain embodiments, P2203 described herein contains one or more chiral centers. These compounds are prepared by any known means, including enantioselective synthesis and/or separation of a mixture of enantiomers and/or diastereomers. Resolution of compounds and isomers thereof is achieved by known methods including, by way of non-limiting examples, chemical processes, enzymatic processes, fractional crystallization, distillation, chromatography, and the like.

In an embodiment, the disclosure also relates to all possible isomers of the compounds of the invention such as optical and/or geometrical, pure or as a mixture, in any proportions as a mixture, of said P2203 and possible tautomeric forms.

In another embodiment, P2203 of the present disclosure may possess asymmetric carbon atoms (optical or chiral centers) or double bonds; the enantiomers, racemates, diastereomers, tautomers, geometric isomers, stereoisometric forms that may be defined, in terms of absolute stereochemistry, as (R)-or (S)- or, as D- or L- for amino acids, and individual isomers are encompassed within the scope of the present disclosure. The compounds of the present disclosure do not include those which are known in art to be too unstable to synthesize and/or isolate. The present disclosure is meant to include compounds in racemic, scalemic, and optically pure forms. Optically active (R)- and (S)-, or D- and L-isomers may be prepared using chiral synthons or chiral reagents or resolved using conventional techniques. When the compounds described herein contain olefenic bonds or other centers of geometric asymmetry, and unless specified otherwise, it is intended that the compounds include both E and Z geometric isomers.

Unless otherwise stated, structures depicted herein are also meant to include all stereochemical forms of the structure, i.e., the R and S configurations for each asymmetric center. Therefore, single stereochemical isomers as well as enantiomeric and diastereomeric mixtures of the present compounds are within the scope of the disclosure.

Yet in an embodiment, P2203 can exist in tautomeric forms, and all such tautomeric forms of the compounds being within the scope of the disclosure. The term "tautomer," as used herein, refers to one of two or more structural isomers which exist in equilibrium, and which are readily converted from one isomeric form to another.

Yet in another embodiment, P2203 exists in individual isomeric, e.g., enantiomeric or diastereomeric form or as mixtures of individual forms, e.g., racemic/diastereomeric mixtures. Any isomer may be present in which the asymmetric center is in the (R)-, (S)-, or (R,S)- configuration. This disclosure is to be understood as embracing both individual optically active isomers as well as mixtures (e.g., racemic/diastereomeric mixtures) thereof. Accordingly, P2203 may be a racemic mixture or it may be predominantly, e.g., in pure, or substantially pure, isomeric form, e.g., greater than about 50% enantiomeric/diastereomeric excess ("ee"), greater than about 55% ee, greater than about 60% ee, greater than about 65% ee, greater than about 70% ee, greater than about 80% ee, greater than about 90% ee, greater than about 95% ee, greater than about 98% ee, greater than about 99% ee, greater than about 99.5% ee, or 100% in either L- or D- form. The purification of said isomers and the separation of said isomeric mixtures may be accomplished by standard techniques known in the art (e.g., column chromatography, preparative TLC, preparative HPLC, simulated moving bed and the like).

Geometric isomers by nature of substituents about a double bond or a ring may be present in cis (Z) or trans (E) form, and both isomeric forms are encompassed within the scope of this invention.

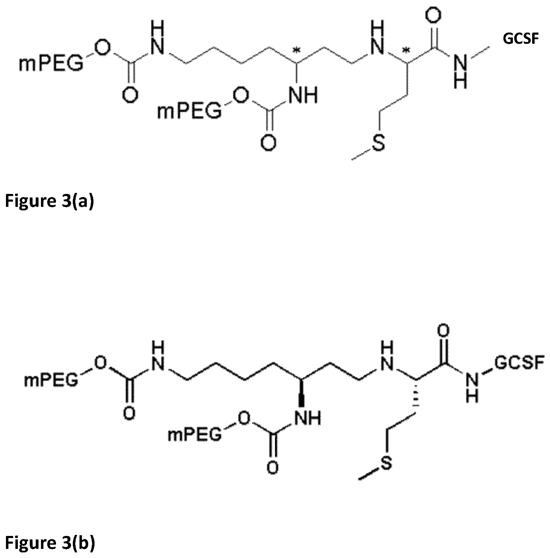

In an embodiment, P2203 includes 2 chiral centers as shown in Figure 3(a). In one embodiment, P2203 is 100% L-, 100% D, or includes a mixture of L- and D- isomers that includes substantially L-form. The L-form is denoted in Figure 3(b).

In another embodiment, P2203 drug substance can include a mixture of L and D forms. Yet in an embodiment, the drug substance can include substantially about 50% of L-form, substantially about 60% of L-form, substantially about 60% of L-form, substantially about 70% of L-form, substantially about 80% of L-form, substantially about 90% of L-form, substantially about 95% of L-form, substantially about 97.5% of L-form, substantially about 99% of L-form, substantially about 99.5% of L-form, or 100% of L-form.

### iv. Pharmacokinetic (PK) and pharmacodynamic (PD)

Pharmacokinetic (PK) and pharmacodynamic (PD) provide information from the scientific basis of modern pharmacotherapy. Pharmacokinetics describes the drug concentration-time courses in body fluids resulting from administration of a certain drug dose, whereas pharmacodynamics relates to the observed effect resulting from a certain drug concentration. The rationale for PK/PD-modelling is to link pharmacokinetics and pharmacodynamics in order to establish and evaluate dose-concentration-response relationships and subsequently describe and predict the effect-time courses resulting from a drug dose. Under pharmacokinetic steady-state conditions, concentration-effect relationships can be described by several relatively simple pharmacodynamic models, which comprise the fixed effect model, the linear model, the long-linear model, the Emax-model and the sigmoid Emax-model. Under non-steady-state conditions, more complex integrated PK/PD-models are necessary to link and account for a possible temporal dissociation between the plasma concentration and the observed effect. Four basic attributes may be used to characterize PK/PD-models: first, the link between measured concentration and the pharmacologic response mechanism that mediates the observed effect, direct vs. indirect link; second, the response mechanism that mediates the observed effect, direct vs. indirect response; third, the information used to establish the link between measured concentration and observed effect, hard vs. soft link; and fourth, the time dependency of the involved pharmacodynamic parameters, time-variant vs. time-invariant. In general, PK/PD-modelling based on the underlying physiological process should be preferred whenever possible. The expanded use of PK/PD-modelling is assumed to be highly beneficial for drug development as well as applied pharmacotherapy and will most likely improve the current state of applied therapeutics.

In an embodiment, the present disclosure's P2203 surprisingly has improved PK/PD profile in rats and monkeys or in a subject or subject in need thereof as compared to existing Pegfilgrastim or other Pegfilgrastim biosimilars, even when there are numerous pegfilgrastim biosimilars approved for marketing by the Food and Drug Administration in the U.S. In another embodiment, P2203 lasts longer than existing Pegfilgrastim and/or its biosimilars, which reduces the overall AD, side effects, number of times or frequency of administration and/or required dosage to achieve substantially similar efficacy as Pegfilgrastim and/or Pegfilgrastim biosimilars.

### v. Pharmaceutically Acceptable Carriers

The composition used in the present disclosure, whether P2203 alone, or with any combinations of other active ingredients (e.g., pegfilgrastim or its biosimilars) thereof, can further comprise pharmaceutically acceptable carriers, excipients, or stabilizers, in the form of lyophilized formulations or aqueous solutions. Acceptable carriers, excipients, or stabilizers can be nontoxic to recipients at the dosages and concentrations, and can comprise buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrans; chelating agents such as EDTA; sugars such as sucrose, mannitol, trehalose or sorbitol; salt-forming counter-ions such as sodium; metal complexes (e.g. Zn-protein complexes); and/or nonionic surfactants such as TWEEN (trademarked), PLURONICS (trademarked) polyethylene glycol (PEG), or methoxy polyethylene glycol (mPEG).

### vi. Dosage

In an embodiment, a subject or subject in need can be administered with about 0.005mg, about 0.075mg, about 0.1mg, about 0.2mg, about 0.3mg, about 0.4mg, about 0.5mg, about 0.6mg, about 0.7mg, about 0.8mg, about 0.9mg, about 1mg, about 2mg, about 3mg, about 4mg, about 5mg, about 6 mg, about 7 mg, about 8 mg, about 9 mg, about 10 mg, about 11 mg, about 12 mg, about 13 mg, or more of P2203 subcutaneously per single injection, per dose, per multiple injections and/or per chemotherapy cycle. Yet in another embodiment, subjects or subjects in need acutely exposed to myelosuppressive doses of radiation can be administered with one or more doses, individually or multiple injections totaling between about 0.05 mg to 14 mg, about 0.05 mg, about 0.075 mg, about 0.1 mg, about 0.15 mg, about 0.2 mg, about 0.25 mg, about 0.3 mg, about 0.35 mg, about 0.4 mg, about 0.45 mg, about 0.5 mg, about 0.55 mg, about 0.6 mg, about 0.65 mg, about 0.7 mg, about 0.75 mg, about 0.8 mg, about 0.85 mg, about 0.9 mg, about 0.95 mg, about 1 mg, about 1.5 mg, about 2 mg, about 2.5 mg, about 3 mg, about 3.5 mg, about 4 mg, about 4.5 mg, about 5 mg, about 5.5 mg, or about 6 mg, about 6.5 mg, about 7 mg, about 8 mg, about 9 mg, about 10 mg, about 11 mg, about 12 mg, about 13 mg, about 14 mg, or more of P2203, where each dosage and/or injection can be administered about once per 3 days, about once per week, about once per 10 days, about once per two weeks, about once per 17 days, about once per three weeks, about once per 24 days, about once per four weeks, about once per 30 or 31 days, about once per five weeks, about once per 39 days, or about once per six weeks apart. In an embodiment, the administration can be any of the above dosage amount administered at once in a single shot instead of multiple shot in a single or multiple visits by a subject to a care facility. In the event that the administration is s.c., any dosage above can be administered in a single shot.

Yet in another embodiment, the first dose/injection can be administered as soon as possible after suspected and/or confirmed exposure to myelosuppressive doses of chemotherapy and/or radiation, and a second dose/injection at about once every 3 days, at about once every 5 days, at about once a week, at about once every 10 days, about once every two weeks, at about once every 17 days, about once every three weeks, at about once every 25 days, about once every four weeks, at about once every 30 or 31 days, about once every five weeks, at about once every 39 days, about once every six weeks, about once every 45 days, or about once every three months thereafter. The injection can contain P2203 at a concentration of between about 0.05 mg to 14 mg, about 0.05 mg, about 0.075 mg, about 0.1 mg, about 0.15 mg, about 0.2 mg, about 0.25 mg, about 0.3 mg, about 0.35 mg, about 0.4 mg, about 0.45 mg, about 0.5 mg, about 0.55 mg, about 0.6 mg, about 0.65 mg, about 0.7 mg, about 0.75 mg, about 0.8 mg, about 0.85 mg, about 0.9 mg, about 0.95 mg, about 1 mg, about 1.5 mg, about 2 mg, about 2.5 mg, about 3 mg, about 3.5 mg, about 4 mg, about 4.5 mg, about 5 mg, about 5.5 mg, about 6 mg, about 7 mg, about 8 mg, about 9 mg, about 10 mg, about 11 mg, about 12 mg, about 13 mg, about 14 mg, or more in about 0.6 mL solution in a single-dose in a single injection prefilled syringe administered in a single injection of any given amount.

In another embodiment, about 6 mg P2203 can be administered in a subject or subject in need for Chemotherapy-induced Neutropenia in a regimen using a single subcutaneous injection or about 6mg per dose or per separate injections administered once per chemotherapy cycle. Alternatively, or additionally, P2203 can be administered to a subject or subject in need for Chemotherapy-induced Neutropenia in a regimen using any known method of administration at between about 0.05 mg to 14 mg, about 0.05 mg, about 0.075 mg, about 0.1 mg, about 0.15 mg, about 0.2 mg, about 0.25 mg, about 0.3 mg, about 0.35 mg, about 0.4 mg, about 0.45 mg, about 0.5 mg, about 0.55 mg, about 0.6 mg, about 0.65 mg, about 0.7 mg, about 0.75 mg, about 0.8 mg, about 0.85 mg, about 0.9 mg, about 0.95 mg, about 1 mg, about 1.5 mg, about 2 mg, about 2.5 mg, about 3 mg, about 3.5 mg, about 4 mg, about 4.5 mg, about 5 mg, about 5.5 mg, about 6 mg, about 6.5 mg, about 7 mg, about 8 mg, about 9 mg, about 10 mg, about 11 mg, about 12 mg, about 13 mg, about 14 mg, or more, about once, about twice, about three, about four or about five times per chemotherapy cycle.

In another embodiment, P2203 can be administered at about 5 mcg/kg/day subcutaneously until engraftment Median Time to neutrophil engraftment in between about 9-16 days, for example, about 12 days for treating HSC transplantation Post HSCT neutrophil engraftment. Alternatively, or additionally, P2203 can be administered at between about 1 to 13 mcg/kg/day, or about 1, about 2, about 3, about 4, about 5, about 6, about 7, about 8, about 9, about 10, about 11, about 12, about 13 mcg/kg/day, or more using known methods of administration until engraftment Median Time to neutrophil engraftment in between about 8 to 16, about 8, about 9, about 10, about 11, about 12, about 13, about 14, about 15, about 16 days or later for treating HSC transplantation Post HSCT neutrophil engraftment.

Yet in another embodiment, P2203 can be administered about 2 days prior to 1^{st} dose of AZA (azacitidine) and about 2 days prior to the 6^{th} dose of AZA at about 5 µg/kg of P2203 for treating subjects or subjects in need with high risk MDS. Alternatively, or additionally, P2203 can be administered between about 1 to 5, about 1, about 2, about 3, about 4, or about 5 days prior to 1^{st} dose of AZA (azacitidine) and about 1, about 2, about 3, about 4, or about 5 days prior to the 6^{th} dose of AZA at about 1 to 10, about 1, about 2, about 3, about 4, about 5, about 6, about 7, about 8, about 9, about 10 µg/kg or more for treating subjects or subjects in need with high risk MDS.

In another embodiment, all of the above recited dosages can be administered per dose, and/or per injection. Non-limiting examples include administering between about 6 to 15 mg of drug using between about 2 to 5 mg per each of about 1-3 injections at about the same time and/or different times, so long as the subject or the subject in need receives a total of about 6 mg, about 7 mg, about 8 mg, about 9 mg, about 10 mg, about 11 mg, about 12 mg, about 7 mg, about 8 mg, about 9 mg, about 10 mg, about 11 mg, about 12 mg, about 13 mg, about 14 mg, about 15 mg, or between about 1 to 2.5 mg per injection about 6 times at about the same time and/or different times. The combination can be picked and chosen depending on patient or subject convenience and/or medical need. In another non-limiting example, the entire amount of between about 6 to 15 mg can be all administered in a single injection (per injection) at a single time point.

In one embodiment, P2203 can be used via s.c. or i.v. with between about 5-10 mcg/kg/day with at least 24 hours after chemo cycle, wherein the number of days varies by the subject's or a subject in need's cancer indication.

Yet in another embodiment, P2203 dosage can be used via s.c. with dosage about 6mg/0.6mL administered at least 24 hours after a chemo cycle.

### vii. Administration Methods

In an aspect, P2203 can be used for subcutaneous injections to a subject or subject in need. Alternative, or additionally, P2203 can be administered via topical contact, intravenous, intraperitoneal, intramuscular, intralesional, intranasal or the implantation of a slow-release device e.g., a mini-osmotic pump, to a subject or a subject in need. Other administration is by any route including parenteral, and transmucosal (e.g., oral, nasal, vaginal, rectal, or transdermal). Parenteral administration includes, e.g., intravenous (i.v.), intramuscular, intra-arteriole, intradermal, intraperitoneal, intraventricular, and/or intracranial. Moreover, where injection is to treat a tumor, e.g., induce apoptosis, administration may be directly to the tumor and/or into tissues surrounding the tumor. Other modes of delivery include, but are not limited to, the use of liposomal formulations, intravenous infusion, transdermal patches.

In certain embodiments, P2203 is administered once daily, about once weekly, about once every two weeks, about once every three weeks, about once every four weeks, about once every five weeks, about once every six weeks, or about once every three months. P2203 can also be administered within about twelve hours or within about twenty-four hours of a dose of chemotherapy and/or radiation therapy. In certain embodiments, P2203 is administered at least once between about 3 to 21, about 3, about 7, about 10, about 14, about 17 or about 21 days before a dose of chemotherapy and/or radiation therapy. In an embodiment, P2203 can be administered to a patient immediately after, at about the same time, and/or any time during chemo and/or radiation therapy.

### viii. Uses

P2203 is a methoyl pegylated form of modified (with extra Met) GCSF which has a surprising higher serum half-life than wild type human GCSF, pegfilgrastim (Neulasta, trademarked), and/or other pegfilgrastim biosimilars. P2203 is administered to patients with cancer to accelerate recovery from chemotherapy induced neutropenia.

In one embodiment, P2203 of present disclosure can be used on subjects or subject in need who has chemotherapy-induced febrile neutropenia, acute myeloid leukemia, subjects or a subjects in need who receive bone marrow transplant, peripheral blood progenitor cell collection or engraftment, and/or subjects or subjects in need with severe chronic neutropenia.

In certain embodiment, P2203 of present disclosure can be used to decrease the incidence of a subject's or a subject in need's infection of any kind, as manifested by febrile neutropenia, in patients with non-myeloid malignancies receiving myelosuppressive anti-cancer drugs associated with a clinically significant incidence of febrile neutropenia. Alternative, and/or additionally, it can increase survival in subjects or subjects in need acutely exposed to myelosuppressive doses of radiation (Hematopoietic Subsyndrome of Acute Radiation Syndrome). Additionally, or alternatively, P2203 can be used to treat CIN, which reduces the incidence of infections following cytotoxic chemotherapy

In further embodiments, P2203 can mobilize hematopoietic stem cells. It can also treat subjects or a subjects in need who underwent transplantation of hematopoietic stem and/or progenitor cells, or treat neutropenia after chemotherapy, Acute myeloid leukemia, Myelodysplastic syndrome (MDS), and/or Aplastic anemia. Yet in another embodiment, P2203 can treat persistent neutropenia in advanced HIV infection, and/or subjects or subjects in need with radiation-induced myelosuppression following a radiological/nuclear incident (H-ARS), which increases survival in patients with acute radiation exposure.

Other non-limiting exemplary uses of P2203 utilize the surprising fact that the molecule can further decrease the incidence of infection, as compared to FDA approved pegfilgrastim and/or its biosimilars, as manifested by febrile neutropenia, in individuals with non-myeloid malignancies receiving myelosuppressive anti-cancer drugs in one or more of the following categories: 1. Cancer individuals receiving myelosuppressive chemotherapy, 2. individuals with acute myeloid leukemia (AML) receiving induction or consolidation chemotherapy, 3. Cancer individuals receiving bone marrow transplantation (BMT), 4. individuals undergoing Peripheral Blood Progenitor Cell Collection and Therapy, 5. individuals with Severe Chronic Neutropenia, and/or 6. individuals who undergo high-risk MDS (Myelodysplastic syndrome) and hematopoietic stem cell (HSC) transplantation (also known as post HSCT neutrophil engraftments). In the embodiment of BMT, P2203 can reduce in the duration of neutropenia following myeloablative therapy before BMT.

In one embodiment, P2203 can be used for cancer subjects or subject in need using Myelosuppressive Chemotherapy, Acute Myeloid Leukemia, Bone Marrow Transplant, Progenitor Cell Collection, Severe Chronic Neutropenia, and/or Acute Radiation Syndrome, all associated with febrile neutropenia, showing surprisingly fewer, lesser, or subtler side effect as compared to FDA approved pegfilgrastim or any of its biosimilars.

In one other embodiment, P2203 can be used to treat neutropenia, a condition where the body makes too few white blood cells. More specifically, P2203 can surprisingly decrease the incidence of infection, as manifested by febrile neutropenia, in subjects or subjects in need with non-myeloid malignancies receiving myelosuppressive anti-neoplastic drugs with surprisingly higher efficacy as compared to FDA approved pegfilgrastim and/or its biosimilars.

In yet another embodiment, in the case of subjects or subject in need using leukapheresis, which is a procedure healthcare providers use to remove white blood cells from blood. Providers may do leukapheresis to ease symptoms that happen when you have abnormal white blood cells that are multiplying uncontrollably. P2203 can mobilize hematopoietic progenitor cells for collection.

In another embodiment, P2203 can surprisingly decrease the incidence of infection, as manifested by febrile neutropenia, in subjects or subject in need with non-myeloid malignancies receiving myelosuppressive anti-cancer drugs associated with a clinically significant incidence of febrile neutropenia. In some instances, this results in a surprisingly increased survival in subjects or subjects in need acutely exposed to myelosuppressive doses of radiation (Hematopoietic Subsyndrome of Acute Radiation Syndrome) as compared to other FDA approved pegfilgrastim and/or its biosimilars.

In certain embodiments, P2203 can be provided in a single dose during a course of chemotherapy, and/or provided as multiple doses over the course of chemotherapy. P2203 surprisingly provides much greater dosing flexibility than is the case with the commercially available FDA approved pegfilgrastim and/or any of its biosimilars.

Yet in another embodiment, P2203 requires fewer administration or lower dosage per administration for all of the aforementioned conditions as compared to pegfilgrastim and/or any of its biosimilars.

In an embodiment, the present disclosure relates to the use of the substantially homogenous composition or pharmaceutical formulation of P2203 for the preparation of a medicament for the treatment of one or more conditions including but not limited to compromised white blood cell production, decreased white blood cell count, decreased endogenous levels of GCSF, neutropenia, chronic and/or severe neutropenia, severe congenital neutropenia, cyclic neutropenia, febrile neutropenia, severe combined neutropenia, myelosuppressive chemotherapy induced thrombocytopenia, chemotherapy induced neutropenia, chemically induced neutropenia, multiple myeloma, chronic myelogenous leukemia (CML), acute myeloid leukemia, myelodysplastic syndrome, and/or alcoholic hepatitis. In some embodiments, the one or more conditions can be induced by a cancer therapy or another condition, wherein said cancer of cancer therapy or another condition include, but is not limited to: lung cancer, lymphoma, breast cancer, hematopoietic stem cell transplantation, bone marrow transplantation, testicular cancer, AIDS-related malignancies, myelodysplastic disorders, myeloid cancer, acute leukemia, congenital and cyclic neutropenia, aplastic anemia, head and neck cancer, lung cancer, stomach cancer, colon cancer, pancreatic cancer, prostate cancer, breast cancer, kidney cancer, bladder cancer, ovary cancer, cervical cancer, melanoma, glioblastoma, myeloid leukemia, myeloma, lymphoma, leukemia, myelosuppressive chemotherapy, acute myeloid leukemia, bone marrow transplant, progenitor cell collection, and/or acute radiation syndrome. In an embodiment, the substantially homogenous composition or pharmaceutical formulation of P2203 includes between about 80% to about 99.99% sequence identity to P2203. For example, at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, and/or at least about 99.5% of the sequence identity to P2203.

In another embodiment, the present disclosure relates to a substantially homogenous composition or pharmaceutical formulation of P2203, characterized in that it is used to treat one or more of conditions including but not limited to: compromised white blood cell production, decreased white blood cell count, decreased endogenous levels of GCSF, neutropenia, chronic and/or severe neutropenia, severe congenital neutropenia, cyclic neutropenia, febrile neutropenia, severe combined neutropenia, myelosuppressive chemotherapy induced thrombocytopenia, chemotherapy induced neutropenia, chemically induced neutropenia, multiple myeloma, chronic myelogenous leukemia (CML), acute myeloid leukemia, myelodysplastic syndrome, and/or alcoholic hepatitis. Yet in some embodiments, the one or more of conditions can be induced by a cancer therapy or another condition, wherein said cancer of cancer therapy or another condition include, but is not limited to: lung cancer, lymphoma, breast cancer, hematopoietic stem cell transplantation, bone marrow transplantation, testicular cancer, AIDS-related malignancies, myelodysplastic disorders, myeloid cancer, acute leukemia, congenital and cyclic neutropenia, aplastic anemia, head and neck cancer, lung cancer, stomach cancer, colon cancer, pancreatic cancer, prostate cancer, breast cancer, kidney cancer, bladder cancer, ovary cancer, cervical cancer, melanoma, glioblastoma, myeloid leukemia, myeloma, lymphoma, leukemia, myelosuppressive chemotherapy, acute myeloid leukemia, bone marrow transplant, progenitor cell collection, and/or acute radiation syndrome.

### iv. Surprising Efficacy, Advantages and Improvement

In an embodiment, the present disclosure describes mPEG-MetHuG-CSF having surprising and unexpected improved *in vitro, in vivo, ex vivo* and clinical pharmacokinetic, pharmacodynamic, and/or toxicokinetic profiles as compared to pegfilgrastim. In some embodiments, mPEG-MetHuG-CSF or a substantially homogenous composition comprising mPEG-MetHuG-CSF can have one or more characteristics and/or profiles shown in Tables 2-6 and 10-21 below.

In another embodiment, the present disclosure describes mPEG-MetHuG-CSF having surprising and unexpected improved *in vitro, in vivo, ex vivo* and clinical pharmacokinetic, pharmacodynamic, and/or toxicokinetic profiles as compared to filgrastim.

Yet in an embodiment, the present disclosure describes mPEG-MetHuG-CSF having surprising and unexpected improved *in vitro, in vivo, ex vivo* and clinical pharmacokinetic, pharmacodynamic, and/or toxicokinetic profiles as compared to pegfilgrastim, filgrastim, pegfilgrastim-jmdb, pegfilgrastim-pbbk, pegfilgrastim-apgf, pegfilgrastim-cbqv, pegfilgrastim-bmez, pegfilgrastim-fpgk, and/or eflapegrastim-xnst.

In an embodiment, the present disclosure describes mPEG-MetHuG-CSF having surprising and unexpected improved *in vitro, in vivo, ex vivo* and clinical pharmacokinetic profiles as compared to pegfilgrastim by at least between about 15% to 95%, at least about 15%, at least about 20%, at least about 30%, at least about 40%, at least about 50%, at least about 60%, at least about 70%, at least about 80%, at least about 90%, at least about 95%, at least between about 1 to 5 folds, at least about one fold, at least about 1.5 fold, at least about 2 fold, at least about 2.5 fold, at least about 3 fold, at least about 4 fold, and/or at least about 5 fold.

In an embodiment, the present disclosure describes mPEG-MetHuG-CSF having surprising and unexpected improved *in vitro, in vivo, ex vivo* and clinical pharmacodynamic profiles as compared to pegfilgrastim by at least between about 15% to 95%, at least about 15%, at least about 20%, at least about 30%, at least about 40%, at least about 50%, at least about 60%, at least about 70%, at least about 80%, at least about 90%, at least about 95%, at least between about 1 to 5 fold, at least about one fold, at least about 1.5 fold, at least about 2 fold, at least about 2.5 fold, at least about 3 fold, at least about 4 fold, and/or at least about 5 fold.

In an embodiment, the present disclosure describes mPEG-MetHuG-CSF having surprising and unexpected improved *in vitro, in vivo, ex vivo* and clinical toxicokinetic profiles (making it safer) as compared to pegfilgrastim by at least between about 15% to 95%, at least about 15%, at least about 20%, at least about 30%, at least about 40%, at least about 50%, at least about 60%, at least about 70%, at least about 80%, at least about 90%, at least about 95%, at least between about 1 to 5 fold, at least about one fold, at least about 1.5 fold, at least about 2 fold, at least about 2.5 fold, at least about 3 fold, at least about 4 fold, and/or at least about 5 fold.

In another embodiment, the present disclosure describes mPEG-MetHuG-CSF having surprising and unexpected improved *in vitro, in vivo, ex vivo* and clinical pharmacokinetic profiles as compared to filgrastim by at least between about 15% to 95%, at least about 15%, at least about 20%, at least about 30%, at least about 40%, at least about 50%, at least about 60%, at least about 70%, at least about 80%, at least about 90%, at least about 95%, at least between about 1 to 5 fold, at least about one fold, at least about 1.5 fold, at least about 2 fold, at least about 2.5 fold, at least about 3 fold, at least about 4 fold, and/or at least about 5 fold.

In another embodiment, the present disclosure describes mPEG-MetHuG-CSF having surprising and unexpected improved *in vitro, in vivo, ex vivo* and clinical pharmacodynamic profiles as compared to filgrastim by at least between about 15% to 95%, at least about 15%, at least about 20%, at least about 30%, at least about 40%, at least about 50%, at least about 60%, at least about 70%, at least about 80%, at least about 90%, at least about 95%, at least between about 1 to 5 fold, at least about one fold, at least about 1.5 fold, at least about 2 fold, at least about 2.5 fold, at least about 3 fold, at least about 4 fold, and/or at least about 5 fold.

In another embodiment, the present disclosure describes mPEG-MetHuG-CSF having surprising and unexpected improved *in vitro, in vivo, ex vivo* and clinical toxicokinetic profiles (making it safer) as compared to filgrastim by at least between about 15% to 95%, at least about 15%, at least about 20%, at least about 30%, at least about 40%, at least about 50%, at least about 60%, at least about 70%, at least about 80%, at least about 90%, at least about 95%, at least between about 1 to 5 fold, at least about one fold, at least about 1.5 fold, at least about 2 fold, at least about 2.5 fold, at least about 3 fold, at least about 4 fold, and/or at least about 5 fold.

Yet in an embodiment, the present disclosure describes mPEG-MetHuG-CSF having surprising and unexpected improved *in vitro, in vivo, ex vivo* and clinical pharmacokinetic profiles as compared to pegfilgrastim, filgrastim, pegfilgrastim-jmdb, pegfilgrastim-pbbk, pegfilgrastim-apgf, pegfilgrastim-cbqv, pegfilgrastim-bmez, pegfilgrastim-fpgk, and/or eflapegrastim-xnst by at least between about 15% to 95%, at least about 15%, at least about 20%, at least about 30%, at least about 40%, at least about 50%, at least about 60%, at least about 70%, at least about 80%, at least about 90%, at least about 95%, at least between about 1 to 5 fold, at least about one fold, at least about 1.5 fold, at least about 2 fold, at least about 2.5 fold, at least about 3 fold, at least about 4 fold, and/or at least about 5 fold..

Yet in an embodiment, the present disclosure describes mPEG-MetHuG-CSF having surprising and unexpected improved *in vitro, in vivo, ex vivo* and clinical pharmacodynamic profiles as compared to pegfilgrastim, filgrastim, pegfilgrastim-jmdb, pegfilgrastim-pbbk, pegfilgrastim-apgf, pegfilgrastim-cbqv, pegfilgrastim-bmez, pegfilgrastim-fpgk, and/or eflapegrastim-xnst by at least between about 15% to 95%, at least about 15%, at least about 20%, at least about 30%, at least about 40%, at least about 50%, at least about 60%, at least about 70%, at least about 80%, at least about 90%, at least about 95%, at least between about 1 to 5 fold, at least about one fold, at least about 1.5 fold, at least about 2 fold, at least about 2.5 fold, at least about 3 fold, at least about 4 fold, and/or at least about 5 fold..

Yet in an embodiment, the present disclosure describes mPEG-MetHuG-CSF having surprising and unexpected improved *in vitro, in vivo, ex vivo* and clinical toxicokinetic profiles (making it safer) as compared to pegfilgrastim, filgrastim, pegfilgrastim-jmdb, pegfilgrastim-pbbk, pegfilgrastim-apgf, pegfilgrastim-cbqv, pegfilgrastim-bmez, pegfilgrastim-fpgk, and/or eflapegrastim-xnst by at least between about 15% to 95%, at least about 15%, at least about 20%, at least about 30%, at least about 40%, at least about 50%, at least about 60%, at least about 70%, at least about 80%, at least about 90%, at least about 95%, at least between about 1 to 5 fold, at least about one fold, at least about 1.5 fold, at least about 2 fold, at least about 2.5 fold, at least about 3 fold, at least about 4 fold, and/or at least about 5 fold.

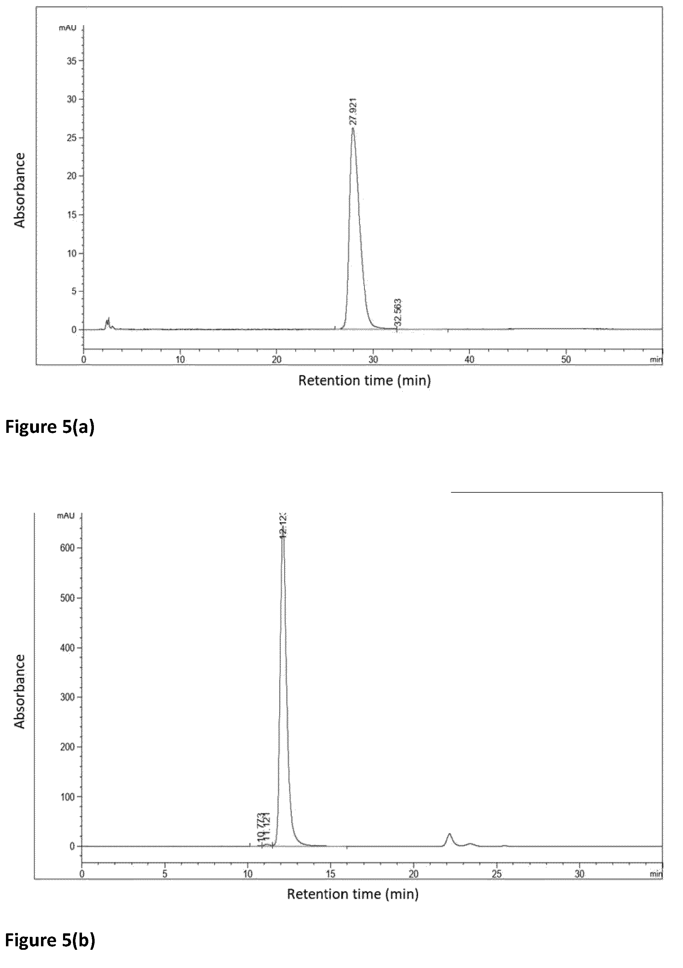

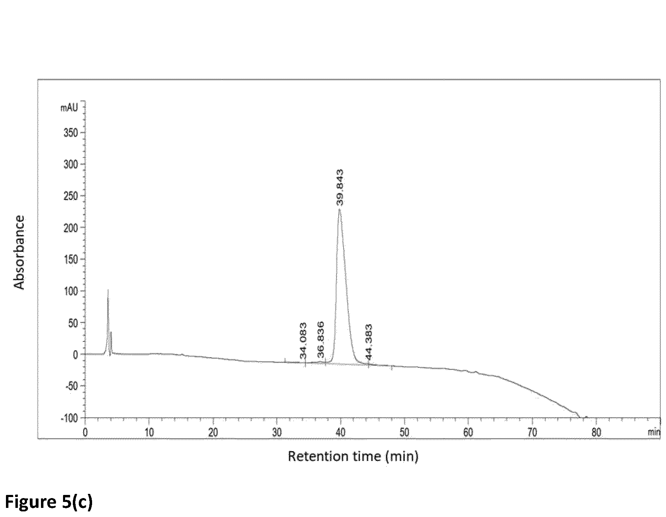

In one embodiment, certain surprising and unexpected improved properties of P2203 as compared to Pegfilgrastim (Neulasta, trademarked) are illustrated in Figure 5, where the PK in rats, PD in rats, and PD in rats and monkey comparison are plotted out. In particular, Figure 5(a) denotes comparison of PK study in rats between P2203 and Neulasta (trademarked). Figure 5(b) denotes comparison of PD study in rats between P2203 and Neulasta (trademarked). Figure 5(c) denotes comparison of PK study in monkeys between P2203 and Neulasta (trademarked) using dosage at about 300µg/kg. Figure 5(d) denotes comparison of PD study in monkeys between P2203 and Neulasta (trademarked) using dosage at about 300µg/kg. P2203 has surprising and unexpected improvement as compared to Neulasta (trademarked).

One of the many reasons for the improved properties of mPEG-MetHuG-CSF is that the unique structure contributes to improved stability in storage, *in vitro, in vivo, ex vivo,* pre-clinical and/or clinical settings.

### EXAMPLES

### Example 1

### Making of P2203

### Construction of Expression Plasmid and Cloning of MetHuG-CSF

The host cells used are *E.coli* BLR (DE3) with (or without RIL) plasmid from *E.coli* BL21-CodonPlus (DE3)-RIL strain, which is not codon optimized for *E.coli.*

The DNA sequence of MetHuG-CSF is illustrated in Figure 2(c) as SEQ ID No.:3. The gene of MetHuG-CSF was cloned into the protein expression vector with multiple cloning sites. The restriction enzyme cleavage sites were used for MetHuG-CSF. Following the transformation of E. coli, the resulting plasmid was identified and isolated, and sequenced to confirm its identity.

MetHuG-CSF gene was cloned by polymerase chain reaction (PCR) using purchased human cDNA as templates. The oligonucleotides with additional methionine-proline residue on the 5' primer were synthesized based on the flanking sequences of GCSF. The derived PCR products were cloned into protein expression vector pET-24, a T7 RNA polymerase promoter driven vector, using restriction cloning sites. The high expression clones were selected by transforming the pET24 containing MetHuG-CSF intermediate gene sequence into *E.coli* BLR (DE3) strain with co-transformation of RIL plasmid from *E.coli* BL21-CodonPlus (DE3)-RIL strain, and maintained in the presence of kanamycin and chloramphenicol. Thus, the BLR cell contains two plasmids. Optionally, the RIL plasmid may be removed.

The transformed *E.coli* BLR (DE3) ± RIL cells were plated on agar plates for antibiotic resistance against kanamycin and chloramphenicol. The expression of *E.coli* cells containing MetHuG-CSF intermediate with or without RIL plasmid on agar plates was confirmed by colonies resistant to kanamycin and chloramphenicol. One colony resistant to kanamycin and chloramphenicol was selected, expanded into 5 mL LB medium containing kanamycin and chloramphenicol for IPTG induction testing. Expression of MetHuG-CSF intermediate was confirmed by 13% reducing SDS-PAGE analysis of IPTG induced culture. The small culture expressing MetHuG-CSF intermediate was identified. The small culture was aliquotted with final 10% glycerol into pre-Master Cell Bank cryovials.

The remaining overall manufacturing process is described below.

### Seed Culture

A master cell bank vial of recombinant *E.coli* BLR (DE3) with Methionine-Proline N-terminally modified GCSF gene is obtained. This plasmid is codon-optimized for *E.coli* expression. The *E.coli* from Master Cell Bank were inoculated into SYN medium, which is contained in a sterilized Erlenmeyer flask supplemented with kanamycin and chloramphenicol. Cells were then cultured at 37° C in a shaker incubator for an overnight culture period of between about 8 to about 24 hours.

### Fermentation

After cultivation, the preculture was then transferred into a sterilized polypropylene seeding bottle, which was then transferred to a basic medium. Fed-batch medium was used to determine and record the zero-time bacterial colonies by OD₆₀₀ at about 3 minutes after inoculation. The bacterial density was then taken and recorded at about every 2 h intervals as batch record after the initial optical density determination. The dissolved oxygen (DO) level was maintained at between about 30-40% of air saturation. The expression of MetHuG-CSF was achieved by the addition of IPTG into the fermenter. After induction, the optical density of the fed-batch culture was taken about every 1 hour and recorded.

### Cell Processing

The fed-batch culture is harvested after IPTG induction into separate centrifuge bottle and centrifuged at between about 6000-8000 rpm for about 15 minutes with a centrifuge. Supernatant was discarded after centrifugation. Cell viability has also been tested using BD Cell Viability Kit and BD FACS Canto flowcytometer for detection of *E.coli* viability. The viability of the *E.coli* used for production of MetHuG-CSF.

### Homogenization

Cells were thawed at 4°C for about 16-24 h, then suspended in TD Buffer (50mM Tris-base, about 1mM DTT with pH at about 8) and homogenized for at least about 3 cycles (@1000 bar). The material was centrifuged (at between about 8,000-10,000 rpm) for about 20 min at 4°C. The pellet was collected and resuspended in TD buffer. Centrifuged pellets were collected and resuspended in TEDD buffer (50mM Tris-base, 5mM EDTA, 1% DOC, 5mM DTT pH 9.0). The material was centrifuged (at between about 8,000-10,000 rpm, for between about 20-40 min, 4°C) and the pellet (inclusion bodies) was resuspended in TS buffer (50mM Tris-base, 2% Sarkosyl pH 8.0). The solubilized inclusion bodies were agitated for between about 20-40 min, at room temperature, then centrifuged (at between about 8,000-10,000 rpm, for 20min, at 4°C). The supernatant was collected and analyzed for total protein concentration by the BCA (Bicinchoninic acid assay) assay.

### Refolding

The inclusion bodies (IBs) were diluted with refolding buffer (20mM Tris-base, with between about 20-40uM CuSO₄ with pH at between about 7-8). The refolding proceeded at room temperature and can take between about 12-24 hrs. A basic column was prepared, and the column was washed with MilliQ water for multiple column volumes (CV). The column was equilibrated with refolding buffer. The MetHuG-CSF was filtered through a 0.2 µM filter and then loaded onto the column. The refolded MetHuG-CSF was loaded onto the column and eluted with refolding buffer at room temperature. The fractions were collected and stored at 4°C.

### Purification

The supernatant was then purified using methods know in the art, in particular, using Ion Exchange - Sepharose Fast Flow (SP FF) column, size exclusion (SE) column, and/or by Hydrophobic Interaction Chromatography. Figure 5(a) denotes various ionic exchange-HPLC analysis of MetHuG-CSF. Figure 5(b) denotes SE-HPLC analysis of MetHuG-CSF. Figure 5(c) denotes various reverse phase HPLC (RP-HPLC) analysis of MetHuG-CSF.

### Ionic exchange Chromatography

An ionic column was prepared, and the column was washed with MQ water or WFI for about 10 column volumes (CV). Next, the column was equilibrated with refolding buffer (about 6-8 CV). The refolded MetHuG-CSF was filtered through a 0.2 µM filter and loaded onto the ionic exchange column then eluted with refolding buffer at room temperature (flow rate at between about 80-100 mL/min). The fractions were collected and stored at 4°C.

The samples collected were adjusted to between about pH 5.0 to pH 5.5 by 50% acetic acid and held for between about 10-15 min with stirring. Then the sample was filtered by depth filter. Before filtration, the filter was flushed with WFI and buffer (CM-A, 20 mM NaOAc, pH 5.2) at a flow rate at between about 500-600 LMH. Then, the sample was loaded onto the filter with the flow rate at between about 100-150 LMH (about 6 L/H/filter) and the pressure held at about ≤ 2.0 bar.

### Second Ion Exchange Chromatography

Resin packed column was washed with MQ water for between about 3-5 CV at a flow rate at between about 150-200 mL/min then equilibrated with equilibration buffer between about 3-5 CV at a flow rate at between about 150-200mL/min. The column elution was programed by AKTA Unicorn 7.6 Axi-CM for purification. The MetHuG-CSF was loaded onto the column at a flow rate between about 150-200 mL/min. To remove unbound material, the column was eluted with Buffer A for between about 3-5 CV. Then, the impurities and target product were separated by 0.0~50.0% gradient elution with Buffer B (20 mM NaOAc, 0.5M NaCl, pH 5.2) for about 15 CV. Finally, the column was cleaned using 100% Buffer B for about 3 CV. The target fractions were collected and analyzed.

### Hydrophobic Interaction Chromatography

The target fractions obtained above were determined for protein concentration for the next procedure. The target pooled fractions were stirred and combined with ammonium sulfate to final concentration of between about 0.4-0.6M and stood for between about 8-10 min. The solution was then filtered through a 0.2 µM filter.

A hydrophobic interaction packed column was washed with MQ water between about 3-5 CV at flow rate between about 300-350 mL/min. The column was then equilibrated with Buffer A (20 mM NaOAc, 0.6M ammonium sulfate, pH 5.2), at between about 3-5 CV at flow rate at between about 290-310 mL/min. The MetHuG-CSF sample was loaded onto the column with flow rate at between 290-310 mL/min. The unbound material was washed away with Buffer A for about 2 CV and the impurities was washed by the second washing buffer (100% A buffer) for about 4 CV. The collection of fractions began when UV 280 nm absorbance exceeded 80 mAU. The target material was eluted with about 40% Buffer B for about 10 CV and stopped collection when the UV absorbance was below 20 mAU. Finally, the column was cleaned with 100% Buffer B for about 2 CV.

### Ultrafiltration/Diafiltration I (UF/DF)

The TFF system was washed with about 20L MQ water or (WFI) and then equilibrated with about 0.5L of PEGylation buffer (about 20 mM NaOAc, at about pH 5.4). The sample was subjected to ultrafiltration to obtain a protein concentration within between about 3.5-4.5 mg/mL and then proceeded to diafiltration with about 6-7 times of the UF sample volume by PEGylation buffer. The sample was then filtered through a 0.2 µM filter.

### PEGylation

To an aqueous solution of 40K-mPEG-aldehyde in WFI, the PEGylation buffer (2M sodium phosphate) was added. After stirring for between about 1-3 min, a solution of the MetHuG-CSF intermediate was then added to the reaction vessel followed by the addition of the reducing agent (about 0.4M sodium cyanoborohydride). The pH was adjusted to between about 5.0-6.0 with 6N NaOH (or 6N HCl) and the oxygen (or air) was removed by purging the vessel with nitrogen. The reaction vessel was allowed to stir in the dark at room temperature for between about 15-20h. The crude material was applied to the UF/DF step for the next purification.

### UltraFiltration/Diafiltration II (UF/DF II)

The TFF system was washed with 20L MQ water or (WFI) and then equilibrated with 0.5L of MSP buffer (20 mM NaOAc, at about pH 5.4). The sample was subjected to ultrafiltration to obtain a protein concentration within about 2 mg/mL and then proceeded to diafiltration with between about 6-7 times of the UF sample volume by PEGylation buffer. The sample was then filtered through a 0.2 µM filter. The supernatant was collected and diluted with MSP Buffer A to a concentration of about 0.5 mg/mL. The sample was stored at 4°C.

### Ion Exchange Chromatography

Packed column was washed with MQ water with between about 3-5 CV at a flow rate at between about 150-170 mL/min and then equilibrated with equilibration buffer between about 3-5 CV at the same flow rate. The unbound material was washed away with Buffer A (about 20 mM NaOAc, at about pH 4.5) for 2 CV and the impurities were washed away with between about 6.0-8.0% Buffer B (about 20 mM NaOAc, 0.5M NaCl, at about pH 4.5) for about 8 CV. A gradient elution between about 8.0%-18.8% Buffer B for 1.5 CV was applied and followed by elution with between about 15-20% Buffer B for between about 4-5 CV to collect the desired target protein. The column was then cleaned with 100% Buffer B for 3 CV. The collected target fractions were analyzed for protein concentration.

### UltraFiltration/Diafiltration III (UF/DF III)

The TFF system (Merck Pellicon 3, with 2x 0.11 m² 10K cassettes) was washed with 20L MQ water or (WFI) and then equilibrated with about 0.5L of UDIII buffer (10 mM Acetic acid, at about pH 4). The sample was subjected to ultrafiltration to obtain a protein concentration at about >10 mg/mL and then proceeded to diafiltration with 6-7 times of the UF sample volume by PEGylation buffer. The sample was then filtered through a 0.2 µM filter and stored at 4°C.

### Formulation

According to the protein concentration analyzed by UV₂₈₀, the stock solution of formulation buffer and 0.8% (w/v) Polysorbate 80 (PS80) were added to collected sample. The final protein concentration was about at 10 mg/ml and containing 0.004% PS80. The protein solution was then filtered into a glass bottle through 0.2 µm filter. Approximately 500 mL of filtrate was obtained and stored at 4°C.

### Example 2

### P2203 Characterization

### P2203 Peptide mapping

Peptide mapping was used to compare the P2203 reference standard and the test samples. Peptides were generated by digesting the samples with endoproteinase Glu-C and separated by RP-HPLC. The chromatogram shows the peaks of the characteristic peptides eluted at specific retention times, serving as a peptide map for confirmation of P2203 identity. The method of peptide mapping is known in the art. Table 1 below denotes the results.

**Table 1**

| **Peptide** | **Residue** | **Sequence** | **Monoisotopic Mass (cal.)** | **Monoisotopic Mass (obs.)¹** | |
|---|---|---|---|---|---|
| | | | | **23FB-G003** | **23FB-G005** |
| G1² | 1-20 | MTPLGPASSLPQSFLLKCLE | 2131.11 | ***2131.09*** | ***2131.09*** |
| G2-3 | 21-34 | QVRKIQGDGAALQE | 1511.81 | ***1511.79*** | ***1511.79*** |
| G4 | 35-46 | KLCATYKLCHPE | 1404.69 | ***1404.68*** | ***1404.67*** |
| G5 | 47 | E | 147.05 | ND | ND |
| G4-5 | 35-47 | KLCATYKLCFIPEE | 1533.73 | ***1533.72*** | **1533.72** |
| G6 | 48-94 | LVLLGHSLGIPWAPLSSCPSQALQLAGCLSQLHS GLFLYQGLLQALE | 4941.63 | ***4941.64*** | ***4941.64*** |
| G7 | 95-99 | GISPE | 501.24 | ***501.24*** | ***501.24*** |
| G8 | 100-105 | LGPTLD | 614.33 | ***614.32*** | ***614.32*** |
| G8-9 | 100-110 | LGPTLDTLQLD | 1184.63 | ***1184.62*** | ***1184.62*** |
| G8-12 | 100-124 | LGPTLDTLQLDVADFATTIWQQMEE | 2834.37 | ***2834.38*** | ***2834.38*** |
| G9 | 106-110 | TLQLD | 588.31 | ***588.31*** | ***588.31*** |
| G9-12 | 106-124 | TLQLDVADFATTIWQQMEE | 2238.05 | ***2238.04*** | ***2238.04*** |
| G10 | 111-113 | VAD | 303.14 | ND | ND |
| G10-12 | 111-124 | VADFATTIWQQMEE | 1667.75 | ***1667.74*** | ***1667.74*** |
| G11 | 114-123 | FATTIWQQME | 1253.58 | ND | ND |
| G12 | 124 | E | 147.05 | ND | ND |
| G13 | 125-163 | LGMAPALQPTQGAMPAFASAFQRRAGGVLVASH LQSFLE | 4025.08 | ***4025.09*** | ***4025.09*** |
| G14 | 164-175 | VSYRVERHLAQP | 1437.82 | ***1437.81*** | ***1437.81*** |

Size-exclusion high-performance liquid chromatography (SE-HPLC) was used to resolve monomer of P2203 from its aggregates. Agilent 1260 VWD HPLC system was operated with G3000SWXL column at 25°C. The system was set to run at about 0.5 mL/min with isocratic gradient. The run last for about 35 min and retention time was about 12 min. The species were detected at 215 nm. The purity was determined as the percentage of monomers present in the total proteins including high molecular weight aggregates and monomers. The percentage of each peak is expressed as integrated areas under individual peaks to total peak areas obtained for either test sample or reference sample. The purity of the main monomer peak was greater than 95.0%, the sum of high molecular aggregates was less than about 4.5%, and any other impurity (post peaks) were less than about 1%.

### RP-HPLC

A reversed-phase high-performance liquid chromatography (RP-HPLC) method was used for quantitative evaluation of the purity of P2203 drug substance for in-process control, drug substance release testing, and stability testing. The method uses a high-performance silica-based C18 column, and the separation is based on the protein's hydrophobic properties. A HPLC system was used and operated with C18 column at 45°C. The system was set to run at about 0.8 mL/min with gradient. The run lasted for about 90 min and retention time was about 39 min. The species were detected at 214 nm. The RP-HPLC method was validated for testing P2203 purity and impurity levels. Results were expressed as percent of peak P2203 recovered versus P2203 related impurities, including the oxidized form and unspecified forms. The purity of the P2203 main peak was greater than about 92.0% and the sum of related impurities (total others) were less than about 8%.

### Molecular Mass

The molecular mass of the MetHuG-CSF was measured using an electrospray ionization Q-Exactive mass analyzer. The intact molecular mass was determined to be 18,798 Dalton, which matches the calculated mass of the full-length GCSF drug intermediate with two intramolecular disulfide bonds. There were two minor species centered at 18,814 and 18,812 detected.

### MS-TOF

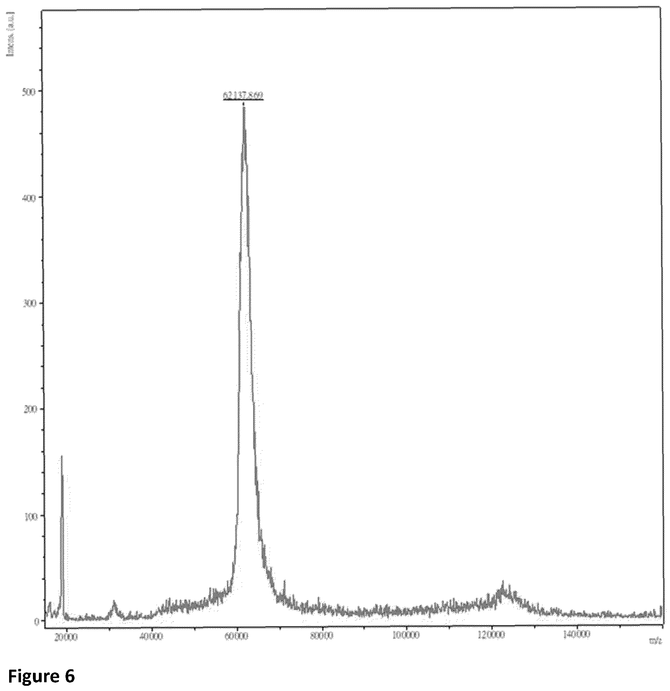

Figure 6 denotes a mass spectrometry (MS) result while determining molecular mass for P2203. P2203 was analyzed by an ESI-Q-TOF MS. The m/z and the number of charges were indicated. The transformed mass spectrum showed a predominant product centered at about 62,138.

### N-terminal Sequence Analysis

N-terminal sequencing was performed according to the automated Edman degradation chemistry. Sample was applied directly onto a Polybrene treated glass fiber filter and subjected to a sequencer. The resulting phenylthiohydantoin (PTH)-amino acids were separated with a Reversed-Phase column and analyzed on-line. Sequencing showed matching amino acid sequence as designed.

### SDS-PAGE with Silver Staining

After protein manufacturing and SPR binding, gel purification SDS-PAGE followed by silver staining were performed using methods known in the art on P2203 compared with marker protein.

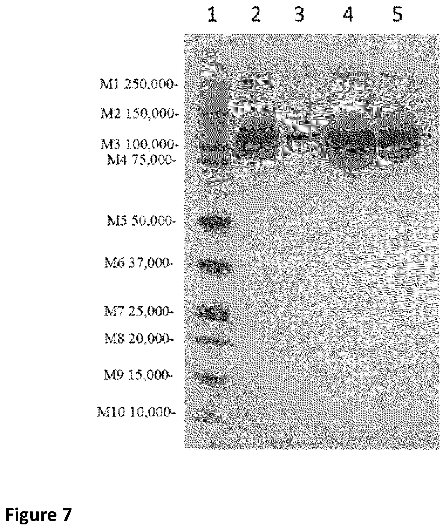

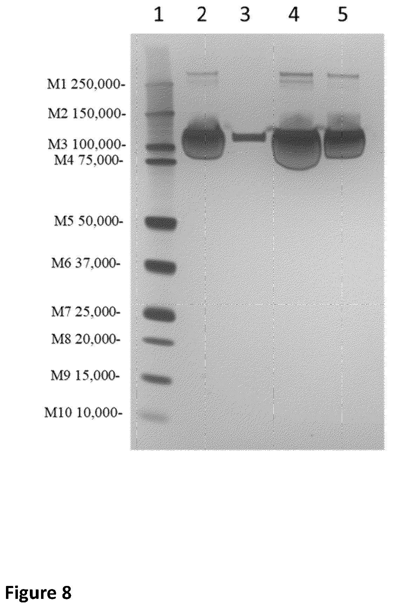

The results are shown in Figure 7 and Figure 8, where Figure 7 is the non-reduced form and Figure 8 is reduced form of P2203. Each lane identifications are denoted below, and the Figures show that all samples of P2203 match the size as compared to the marker protein, which is the reference standard.

| **Lane** | **Sample Name** | **Loading (µg)** |
|---|---|---|
| 1 | Marker Protein | 3.5 µL |
| 2 | P2203 lot 1, 100% | 1 |
| 3 | P2203 lot 1, 1% | 0.01 |
| 4 | P2203 lot 1, 300% | 3 |
| 5 | P2203 lot 2 | 1 |

### Stability

Here, drug substance refers to the active pharmaceutical ingredient, P2203, which is often not the only ingredient of a final drug product. A "drug product" refers to the finished dosage form.

Stability of P2203 was evaluated at different temperatures for 3 months (0 to 3). In the Tables blow, HMW stands for high molecular weight materials that are not pure P2203. Table 2 shows the stability of drug substance at between 2-8°C.

**Table 2.**

| Parameter | | | Acceptance Criteria | Month | | | |
|---|---|---|---|---|---|---|---|
| | | | | 0 | 1 | 2 | 3 |
| Appearance | Clarity and colouration | | Clear, colorless liquid | Complies | Complies | Complies | Complies |
| Identity | Glu-C peptide map (RP-HPLC) | | The profile is comparable to reference standard. | Complies | Complies | Complies | Complies |
| | 4-12% SDS-PAGE (Non-reduced) Silver stain | | The main band in the sample solution migrates to the same position as reference solution A | Complies | Complies | Complies | Complies |
| | 4-12% SDS-PAGE (Reduced) Silver stain | | | Complies | Complies | Complies | Complies |
| Purity and Impurities | SE-HPLC | Main peak | ≥ 95.0% | 99.40% | 99.40% | 99.40% | 99.50% |
| | | Front peak (Total HMW species) | ≤ 4.5% | 0.60% | 0.60% | 0.60% | 0.50% |
| | | Post peak | ≤ 1.0% | ND | ND | ND | ND |
| | RP-HPLC | Main peak | ≥ 92.0% | 98.40% | 98.40% | 98.30% | 98.30% |
| | | Total others | ≤ 8.0% | 1.60% | 1.60% | 1.70% | 1.70% |
| | IEX-HPLC | Main peak | ≥ 95.0% | 99.80% | 99.90% | 99.90% | 99.80% |
| | | Total others | ≤ 5.0% | 0.20% | 0.10% | 0.10% | 0.20% |
| | Host cell DNA (HCD) | | ≤ 500 pg/mg | < LOQ LOQ=0. 497 pg/mg) | ------- | ------- | ------- |
| | Host cell protein (HCP) | | ≤ 100 ng/mg | < LOQ (LOQ=1 4.91 ng/mg) | ------- | ------- | -------- |
| Microbiolog ical Quality | Bacterial endotoxins | | ≤ 10 EU/mg | < LOQ (LOQ=0. 497 EU/mg) | ------- | ------- | ------- |
| | Bioburden | | ≤ 1 CFU/mL | < 1 CFU/mL | ------ | ------ | ------- |
| Quantity | Protein content (UV 280) | | 10.0 ±1.0 mg/mL | 10.06 mg/mL | 10.04 mg/mL | 9.98 mg/mL | 9.94 mg/mL |
| Potency | Proliferation bioassay | | 60%-140% relative potency of the Reference Standard | 94% | 101% | 100% | 107% |
| General Tests | pH | | 4.0 ± 0.5 | 4.1 | 4.1 | 4.1 | 4.1 |
| | Osmolality | | 300 ± 40 mOsm/kg | 307 mOsm/k g | 310 mOsm/k g | 311 mOsm/k g | 308 mOsm/kg |

| | | | | | | | |
|---|---|---|---|---|---|---|---|
| ND: Not detected LOQ: Limit Of Quantitation | | | | | | | |

Table 3 shows the stability study of drug substance at about 25°C.

**Table 3.**

| Parameter | | | Acceptance Criteria | Month | | | |
|---|---|---|---|---|---|---|---|
| | | | | 0 | 1 | 2 | 3 |
| Appearance | Clarity and colouration | | Clear, colorless liquid | Complies | Complies | Complies | Complies |
| Identity | Glu-C peptide map (RP-HPLC) | | The profile is comparable to reference standard. | Complies | Complies | Complies | Complies |
| | 4-12% SDS-PAGE (Non-reduced) Silver stain | | The main band in the sample solution migrates to the same position as reference solution A | Complies | Complies | Complies | Complies |
| | 4-12% SDS-PAGE (Reduced) Silver stain | | | Complies | Complies | Complies | Complies |
| Purity and Impurities | SE-HPLC | Main peak | ≥ 95.0% | 99.40% | 99.30% | 99.40% | 99.20% |
| | | Front peak (Total HMW species) | ≤ 4.5% | 0.60% | 0.70% | 0.60% | 0.70% |
| | | Post peak | ≤ 1.0% | ND | ND | ND | 0.10% |
| | RP-HPLC | Main peak | ≥ 92.0% | 98.40% | 98.30% | 98.20% | 98.20% |
| | | Total others | ≤ 8.0% | 1.60% | 1.70% | 1.80% | 1.80% |
| | IEX-HPLC | Main peak | ≥ 95.0% | 99.80% | 99.90% | 99.80% | 99.80% |
| | | Total others | ≤ 5.0% | 0.20% | 0.10% | 0.20% | 0.20% |
| | Host cell DNA | | ≤ 500 pg/mg | < LOQ (LOQ=0.497 pg/mg) | ------ | ------ | ------ |
| | Host cell protein | | ≤ 100 ng/mg | < LOQ (LOQ=14.91 ng/mg) | ------ | ------ | ------ |
| Microbiological Quality | Bacterial endotoxins | | ≤ 10 EU/mg | < LOQ (LOQ=0.497 EU/mg) | ------ | ------ | ------ |
| | Bioburden | | ≤ 1 CFU/mL | < 1 CFU/mL | ------ | ------ | ------ |
| Quantity | Protein content (UV 280) | | 10.0 ±1.0 mg/mL | 10.06 mg/mL | 10.10 mg/mL | 10.23 mg/mL | 10.26 mg/mL |
| Potency | Proliferation bioassay | | 60%-140% relative potency of the Reference Standard | 94% | 94% | 103% | 102% |
| General Tests | pH | | 4.0 ± 0.5 | 4.1 | 4.1 | 4.1 | 4.1 |
| | Osmolality | | 300 ± 40 mOsm/kg | 307 mOsm/kg | 310 mOsm/kg | 312 mOsm/kg | 305 mOsm/kg |

| | | | | | | | |
|---|---|---|---|---|---|---|---|
| ND: Not detected LOQ: Limit Of Quantitation | | | | | | | |

Table 4(a) shows the stability study of drug product at about 2-8 °C.

**Table 4 (a).**

| Parameter | | | Acceptance Criteria | Month | |
|---|---|---|---|---|---|
| | | | | 0 | 1 |
| Appearance | Clarity and colouration | | Clear, colorless liquid | Complies | Complies |
| Identity | Glu-C peptide map (RP-HPLC) | | The profile is comparable to reference standard. | Complies | Complies |
| | 4-12% SDS-PAGE (Non-reduced) Silver stain | | The main band in the sample solution migrates to the same position as reference solution A | Complies | Complies |
| | 4-12% SDS-PAGE (Reduced) Silver stain | | | Complies | Complies |
| Purity and Impurities | SE-HPLC | Main peak | ≥ 95.0% | 99.50% | 99.30% |
| | | Front peak (Total HMW species) | ≤ 4.5% | 0.50% | 0.70% |
| | | Post peak | ≤ 1.0% | ND | ND |
| | RP-HPLC | Main peak | ≥ 92.0% | 98.40% | 98.30% |
| | | Total others | ≤ 8.0% | 1.60% | 1.70% |
| | IEX-HPLC | Main peak | ≥ 95.0% | 99.80% | 99.70% |
| | | Total others | ≤ 5.0% | 0.20% | 0.30% |
| | Host cell DNA | | ≤ 500 pg/mg | < LOQ (LOQ=0.497 pg/mg) | ----- |
| | Host cell protein | | ≤ 100 ng/mg | < LOQ (LOQ=14.91 ng/mg) | ----- |
| Microbiological Quality | Bacterial endotoxins | | ≤ 10 EU/mg | <LOQ (LOQ=0.502 EU/mg) | ----- |
| | Sterility | | Sterile | Sterile | ----- |
| Quantity | Protein content (UV 280) | | 10.0 ±1.0 mg/mL | 9.97 mg/mL | 10.12 mg/mL |
| Potency | Proliferation bioassay | | 60%-140% relative potency of the Reference Standard | 114% | 96% |
| General Tests | pH | | 4.0 ± 0.5 | 4.1 | 4.1 |
| | Osmolality | | 300 ± 40 mOsm/kg | 308 mOsm/kg | 312 mOsm/kg |
| | Particulate matter | 10 um | 6000/container | 3/container | ----- |
| | | 25 um | 600/container | 0/container | ----- |
| | Extractable volume | | ≥ 0.6 mL delivered | ≥ 0.6 mL delivered | ----- |
| | Container closure integrity Test | | No leakage | No leakage | ----- |

| | | | | | |
|---|---|---|---|---|---|
| ND: Not detected LOQ: Limit Of Quantitation | | | | | |

Table 4(b) shows the stability of drug product at about 25°C

**Table 4(b)**

| Parameter | | | Acceptance Criteria | Month | |
|---|---|---|---|---|---|
| | | | | 0 | 1 |
| Appearance | Clarity and colouration | | Clear, colorless liquid | Complies | Complies |
| Identity | Glu-C peptide map (RP-HPLC) | | The profile is comparable to reference standard. | Complies | Complies |
| | 4-12% SDS-PAGE (Non-reduced) Silver stain | | The main band in the sample solution migrates to the same position as reference solution A | Complies | Complies |
| | 4-12% SDS-PAGE (Reduced) Silver stain | | | Complies | Complies |
| Purity and Impurities | SE-HPLC | Main peak | ≥ 95.0% | 99.50% | 99.30% |
| | | Front peak (Total HMW species) | ≤ 4.5% | 0.50% | 0.70% |
| | | Post peak | ≤ 1.0% | ND | ND |
| | RP-HPLC | Main peak | ≥ 92.0% | 98.40% | 98.20% |
| | | Total others | ≤ 8.0% | 1.60% | 1.80% |
| | IEX-HPLC | Main peak | ≥ 95.0% | 99.80% | 99.80% |
| | | Total others | ≤ 5.0% | 0.20% | 0.20% |
| | Host cell DNA | | ≤ 500 pg/mg | < LOQ (LOQ=0.497 pg/mg) | ---- |
| | Host cell protein | | ≤ 100 ng/mg | < LOQ (LOQ=14.91 ng/mg) | ---- |
| Microbiological Quality | Bacterial endotoxins | | ≤ 10 EU/mg | <LOQ (LOQ=0.502 EU/mg) | ---- |
| | Sterility | | Sterile | Sterile | ---- |
| Quantity | Protein content (UV 280) | | 10.0 ±1.0 mg/mL | 9.97 mg/mL | 10.07 mg/mL |
| Potency | Proliferation bioassay | | 60%-140% relative potency of the Reference Standard | 114% | 104% |
| General Tests | pH | | 4.0 ± 0.5 | 4.1 | 4.1 |
| | Osmolality | | 300 ± 40 mOsm/kg | 308 mOsm/kg | 312 mOsm/kg |
| | Particulate matter | 10 um | 6000/container | 3/container | - |
| | | 25 um | 600/container | 0/container | - |
| | Extractable volume | | 0.6 mL delivered | 0.6 mL delivered | - |
| | Container closure integrity Test | | No leakage | No leakage | - |

| | | | | | |
|---|---|---|---|---|---|
| ND: Not detected LOQ: Limit Of Quantitation | | | | | |

Stability data of the first available P2203 lot for clinical uses were stored at 5°C ± 3°C for the long-term condition and at 25°C ± 2°C/60% RH ± 5% RH for the accelerated condition.

The stability data and two lots for reference standard are also presented. The stabilities of P2203 are supported by the real-time data met within the specifications set at the time of testing. All samples are stable.

### Example 3

Surface Plasmon Resonance (SPR) binding for P2203, MetHuG-CSF, and Pegfilgrastim against G-CSF receptor binding assay

One aim of this example is to demonstrate surface plasmon resonance (SPR) binding properties for the determination of the equilibrium dissociation constant (K_{D}) between granulocyte colony-stimulating factor receptor (G-CSFR) and three testing samples, Filgrastim Reference Standard (Filgrastim RS, or Neulasta), MetHuG-CSF and mPEG-MetHuG-CSF (P2203). The binding strength of the three molecules are demonstrated by direct comparison.

In this example, G-CSFR (GCSF receptors) were immobilized onto the Sensor Chip CM5 (Biacore, trademarked) as the ligand. In each testing samples, Filgrastim RS(reference standard), MetHuG-CSF, and mPEG-MetHuG-CSF, as the analyte, were flowed through the ligand on CM5 chip respectively. The titration range of test sample analytes used in the assay was 40.5, 20.2, 20, 10.1, 5.1, 2.5, 1.3 and 0.6 nM. Formulation buffers of testing samples were also tested as negative controls. The binding between the ligand and the analyte was monitored through SPR sensorgram. As slow-on and slow-off sensorgrams were observed, the kinetics fit model ("1:1 binding") was used to obtain association constant (ka) and dissociation constant (kd), and K_{D} was calculated by kd/ka.

In Table 5 below, K_{D} values of three testing samples towards GCSFR were determined by SPR and summarized in the unit of molar concentration (M). For the K_{D} ratio, the K_{D} value of each testing sample was normalized against Filgrastim.

**Table 5.**

| | Filgrastim | MetHuG-CSF | P2203 |
|---|---|---|---|
| K_{D} (M) | 5.95E-10 | 7.13E-10 | 9.82E-10 |
| K_{D} ratio | 1.00 | 1.20 | 1.65 |

The binding affinity of testing proteins was determined using the G-CSFR SPR binding assay.

In Table 6 below, K_{D} values of three testing samples towards G-CSFR were determined by SPR and summarized in the unit of molar concentration (M). For the K_{D} ratio, the K_{D} value of each testing sample was normalized with K_{D} of Filgrastim RS.

**Table 6**

| **Sample** | **Fit model (kinetics)** | **ka (1/Ms)** | **kd (1/s)** | **K_{D} (M)** | **K_{D} ratio** |
|---|---|---|---|---|---|
| Filgrastim RS | 1:1 binding | 7.47E+05 | 4.44E-04 | 5.95E-10 | 1.00 |
| MetHuG-CSF | 1:1 binding | 6.57E+05 | 4.69E-04 | 7.13E-10 | 1.20 |
| mPEG-MetHuG-CSF | 1:1 binding | 4.08E+05 | 4.01E-04 | 9.82E-10 | 1.65 |

The SPR study aims to determine the binding affinities of G-CSFR for the Filgrastim Reference Standard (Filgrastim RS), MetHuG-CSF, and P2203. The equilibrium dissociation constant (KD) values of Filgrastim RS, MetHuG-CSF, and P2203 bound GCSFR were 0.595 nM, 0.713 nM, and 0.982 nM, respectively, indicating that all three testing samples bound GCSFR with high affinities.

In summary, the head-to-head comparison of the present disclosure's P2203 outperforms MetHuG-CSF alone or Filgrastim RS for Ka, Kd, with a final K_{D} by a factor of at least about 1.65.

### Example 4

### Direct Comparison of P2203 Single Dose Pharmacokinetic in Rats against MetHuG-CSF, and Filgrastim

This Example demonstrates the systemic exposure, pharmacokinetic (PK), pharmacodynamics (PD) and immunogenicity profile in Sprague Dawley (SD) rats following a single intravenous administration of P2203, MetHuG-CSF, and Filgrastim.

Animals were randomly assigned into 7 groups before dosing. The body weight variation of animals used was not exceeding ± 20 percent of the mean weight of all animals. Groups of 5 or 10 male SD rats received single intravenous administration of P2203, Neulasta (trademarked) or MetHuG-CSF. Examinations of these animals were conducted with respect to mortality, body weight and clinical observation. The results showed that no animal was found dead and no test article related clinical signs were observed in all study groups. The detailed results of examinations are shown in Table 7. Blood samples were collected for PD analysis, PK analysis and immunogenicity analysis.

The time points for blood sampling are presented below. No anti-GCSF antibody and anti-PEG antibody were observed in all animals in this study.

**Table 7**

| **Items** | **Groups** | **Time points** |
|---|---|---|
| PK analysis | 1-4 | Pre-dose*, 15-min (± 1 min), 1-h (± 10 mins), 2-h (± 10 mins), 4-h (± 10 mins), 8-h (± 10 mins), 24-h (± 15 mins), 48-h (± 15 mins), 72-h (± 15 mins), 120-h (± 15 mins), 168-h (± 15 mins), 240-h (± 15 mins) post the end of dosing |
| | 5-7 | Pre-dose*, 15-min (± 1 min), 1-h (± 10 mins), 2-h (± 10 mins), 4-h (± 10 mans), 6-h (± 10 mins), 8-h (± 10 mins), 24-h (± 15 mins) post the end of dosing |
| PD analysis | 1⁻7 | Pre-dose*, 24-h (± 15 mins), 48-h (± 15 mins), 72-h (± 15 mins), 96-h (± 15 mins), 120-h (± 15 mins), 168-h (± 15 mins) post the end of dosing |
| Immunogenicity analysis | 1-7 | Pre-dose*, 336-h (± 15 mins), 672.h (± 15 mins) post the end of dosing |

Animals were randomly assigned into 7 groups before dosing. The body weight variation of animals used was not exceeding ± 20 percent of the mean weight of all animals. The basic design is presented in Table 8 below.

**Table 8**

| Group | Dose Level (mcg/kg) | Sample Used | Dose Concentration (mcg/mL) | Dose Volume (mL/kg) | No of animals (M: Male) |
|---|---|---|---|---|---|
| 1 | 300 | P2203 | 300 | 1 | 5M |
| 2 | 1000 | P2203 | 1000 | 1 | 10M |
| 3 | 300 | Pegfilgrastim | 300 | 1 | 5M |
| 4 | 1000 | Pegfilgrastim | 1000 | 1 | 5M |
| 5 | 300 | MetHuG-CSF | 300 | 1 | 5M |
| 6 | 1000 | MetHuG-CSF | 1000 | 1 | 5M |
| 7 | 0 | N/A | 0 | 1 | 5M |

### Preparation of dose formulations

The dose formulations were prepared by diluting the test articles with appropriate formulation buffer. The dose formulations for intravenous injection were freshly prepared.

### Dose formulation analysis

On the dosing day, a volume of 2 mL dosing solutions were sampled from the middle layer of Group 1-6. The samples were stored at 2-8°C.

All animals were dosed once with the design listed above. The dosing day was denoted as Study Day 1 (Day 1). All substance were administered by intravenous injection.

For Mortality/Moribundity, animal status was recorded twice daily (once daily on the weekend or holidays). For Body weights, all animals were recorded before randomization and on Day 1 (prior to dosing).

For clinical observation, the clinical observation was performed once daily during the study period. Any abnormal findings, local/systemic and behavioral abnormalities were recorded and documented. For Blood sampling and analysis, Blood sampling time points were conducted as shown in Table 9 below.

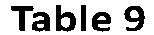

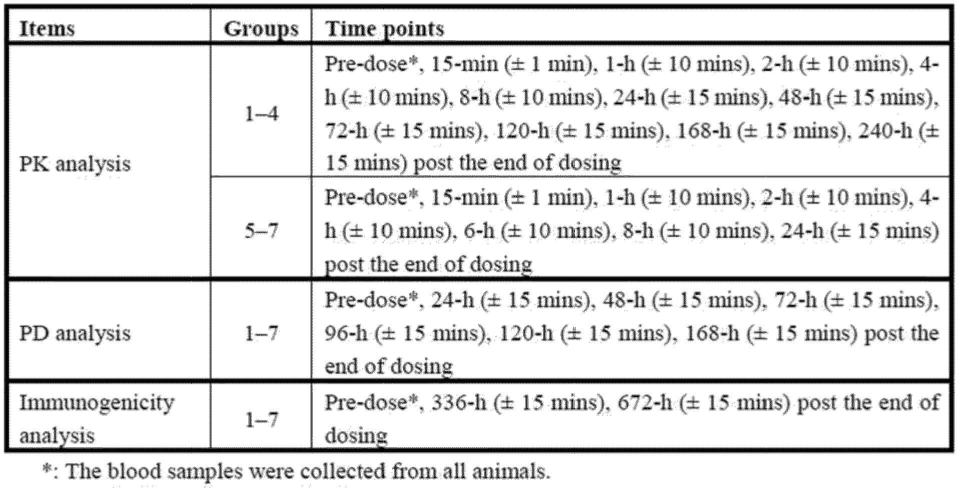

All PK samples were analyzed at the test site. A developed ELISA method was used to analyze serum samples. The PK parameters (C₀, Tmax, t_{1/2}, AUC [area under curve], etc.) were analyzed.

### PK Results

Mean pharmacokinetic parameters of 300 µg/kg and 1000 µg/kg of P2203, Pegfilgrastim, and MetHuG-CSF to rats after intravenous administration were analyzed. Data expressed as mean ± standard deviation are shown below in Table 10.

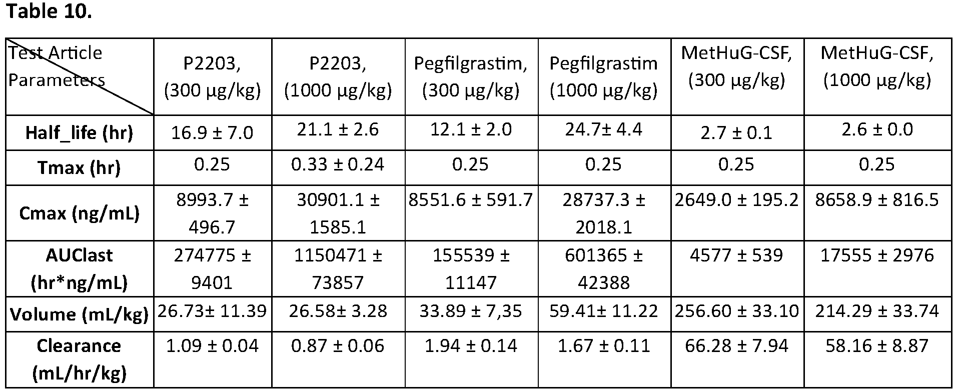

### PD analysis

Hematology was performed for all surviving animals after blood collection for PD analysis. On the scheduled sampling days, blood samples were obtained through tail vein and collected into tubes containing K₂ EDTA for complete blood count analysis. Analyzed items include White blood cell counts (WBC), WBC differential (%), Neutrophils (NEU), Eosinophils (EOS), Basophils (BAS), Monocytes (MON), and Lymphocytes (LYM).

All measured parameters were calculated and expressed as mean ± standard deviation or percentage. Comparisons of parametric data collected from treated and control groups of PD analysis were performed by One-Way ANOVA method, followed by Dunnett's method.

Table 11 denotes Results of White Blood Cell (WBC) Counts of P2203, Neulasta, and MetHuG-CSF.

**Table 11**

| **WBC (10³/µL)** | | | | | | | |
|---|---|---|---|---|---|---|---|
| **Group** | **Predose** | **Day 2 (24hr)** | **Day 3 (48hr)** | **Day 4 (72hr)** | **Day 5 (96hr)** | **Day 6 (120hr)** | **Day 8 (168hr)** |
| Vehicle | 11.164 ± 3.303 | 16.498 ± 3.351 | 19.426 ± 3.443 | 14.446 ± 2.551 | 15.670 ± 2.954 | 14.304 ± 2.734 | 14.216 ± 2.981 |
| P2203-300 | 9.792 ± 2.212 | 45.760 ± 7.133* | 58.274 ± 12.320* | 73.944 ± 14.197* | 61.502 ± 11.956*^{a} | 34.072 ± 6.977*^{a} | 14.206 ± 3.027 |
| P2203-1000 | 10.520 ± 2.495 | 44.951 ± 5.381* | 51.125 ± 4.711*^{b} | 68.549 ± 9.698*^{b} | 83.612 ± 11.425* | 98.890 ± 12.706*^{b} | 33.452 ± 4.651*^{b} |
| Neulasta-300 | 10.734 ± 1.908 | 47.030 ± 6.521* | 56.776 ± 7.913* | 60.536 ± 9.756* | 32.572 ± 8.124* | 20.766 ± 5.560* | 13.430 ± 1.395 |
| Neulasta-1000 | 10.800 ± 2.469 | 51.702 ± 6.417* | 67.794 ± 12.591* | 83.990 ± 12.795* | 88.830 ± 9.349* | 51.660 ± 6.693* | 19.148 ± 2.312* |
| MetHuG-CSF-300 | 7.824 ± 1.929 | 32.742 ± 8.676*^{a} | 13.864 ± 3.364*^{a} | 10.820 ± 1.289*^{a} | 11.488 ± 1.144*^{a} | 11.744 ± 1.424^{a} | 10.154 ± 0.779*^{a} |
| MetHuG-CSF-1000 | 10.606 ± 1.488 | 46.650 ± 6.806* | 22.796 ± 2.320^{b} | 14.516 ± 1.744^{b} | 16.104 ± 1.958^{b} | 15.484 ± 2.952^{b} | 15.288 ± 2.791^{b} |

| | | | | | | | |
|---|---|---|---|---|---|---|---|
| *: p ≦ 0.05 (One-Way ANOVA, compared to the vehicle group) ^{a}: p ≦ 0.05 (Student t-test, P2203-300 or MetHuC-CSF-300 compared to the Neulasta-300 group) ^{b}: p ≦ 0.05 (Student t-test, P2203-1000 or MetHuG-CSF-1000 compared to the Neulasta-1000 group) | | | | | | | |

Table 12 shows Results of Absolute Neutrophil Counts (ANC) of P2203, Neulasta, and MetHuG-CSF.

**Table 12**

| **ANC (10³/µL)** | | | | | | | |
|---|---|---|---|---|---|---|---|
| **Group** | **Predose** | **Day 2 (24hr)** | **Day 3 (48hr)** | **Day 4 (72hr)** | **Day 5 (96hr)** | **Day 6 (120hr)** | **Day 8 (168hr)** |
| Vehicle | 1.410 ± 0.583 | 2.770 ± 1.053 | 3.066 ± 0.756 | 2.490 ± 1.142 | 2.818 ± 0.582 | 2.518 ± 0.492 | 2.314 ± 0.577 |
| P2203-300 | 1.130 ± 0.290 | 28.400 ± 5.237* | 32.528 ± 8.582* | 41.564 ± 11.683* | 40.312 ± 8.244*^{a} | 19.444 ± 3.803*^{a} | 5.042 ± 2.235* |
| P2203-1000 | 1.205 ± 0.306 | 27.211 ± 3.480*^{b} | 27.993 ± 2.030*^{b} | 33.857 ± 5.622*^{b} | 54.654 ± 7.642* | 68.507 ± 10.210*^{b} | 19.520 ± 4.140*^{b} |
| Neulasta-300 | 1.668 ± 0.320 | 28.290 ± 4.263* | 31.060 ± 5.255* | 32.864 ± 8.307* | 16.918 ± 6.362* | 8.930 ± 4.374* | 4.012 ± 1.514 |
| Neulasta-1000 | 1.694 ± 0.430 | 32.222 ± 4.825* | 36.810 ± 6.597* | 44.420 ± 8.490* | 59.600 ± 7.639* | 32.142 ± 5.169* | 7.860 ± 2.292* |
| MetHuG-CSF-300 | 1.302 ± 0.336 | 18.920 ± 6.553*^{a} | 3.678 ± 1.365^{a} | 1.946 ± 0.486^{a} | 2.446 ± 0.403^{a} | 2.320 ± 0.356^{a} | 1.920 ± 0.f300^{a} |
| MetHuG-CSF-1000 | 1.734 ± 0.378 | 27.924 ± 6.888* | 7.060 ± 1.839*^{b} | 2.816 ± 0.617^{b} | 3.570 ± 0.561*^{b} | 3.282 ± 0.483^{b} | 3.460 ± 0.994^{b} |

| | | | | | | | |
|---|---|---|---|---|---|---|---|
| *: p ≦ 0.05 (One-Way ANOVA, compared to the vehicle group) ^{a}: p ≦ 0.05 (Student t-test, P2203-300 or MetHuG-CSF-300 compared to the Neulasta-300 group) ^{b}: p ≦ 0.05 (Student t-test, P2203-1000 or MetHuG-CSF-1000 compared to the Neulasta-1000 group) | | | | | | | |

All rats survived to the scheduled sacrifice day and no adverse clinical signs occurred during the study. The hematology of blood samples was performed for PD analysis.

The WBC counts between Group 1 and Group 3 was significantly different at 96h and 120h. It was also significantly different between Group 2 and Group 4 at 48h, 72 h, 120h and 168h. The ANC between Group 1 and Group 3 was significantly different at 96h and 120h. It was also significantly different between Group 2 and Group 4 at 24h, 48h, 72h, 120h and 168h. Analysis items and results are summarized above. No anti-GCSF antibody and anti-PEG antibody were observed in all groups of animals.

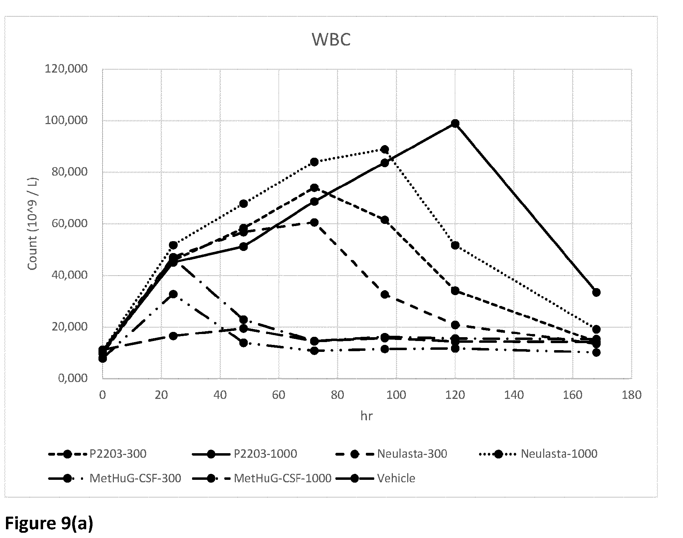

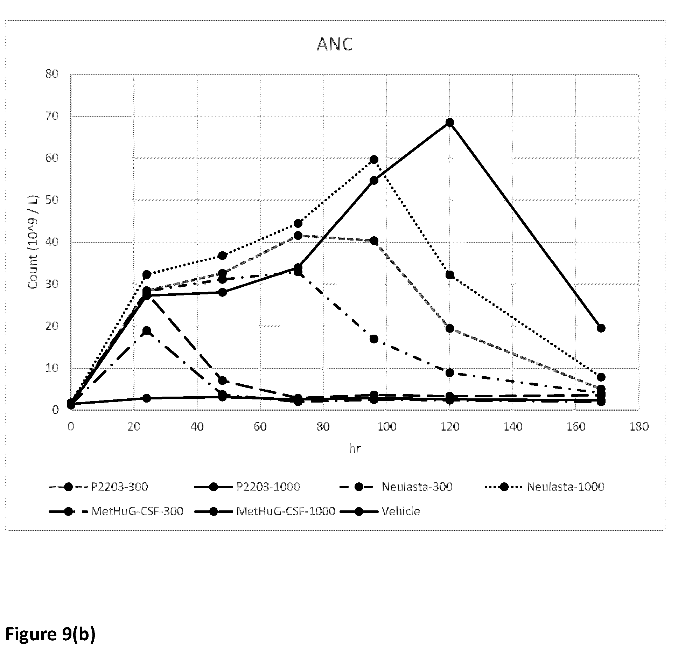

Figures 9(a) and 9(b) summarize rat PD. In particular, Figure 9(a) shows rats PD of MetHuG-CSF, mPEG-MetHuG-CSF and Pegilgrastim (Neulasta) for WBC (white blood cell). Figure 9(b) shows PD comparison of MetHuG-CSF, mPEG-MetHuG-CSF and Pegilgrastim (Neulasta) for ANC (absolute neutrophil count).

### Example 5

### P2203 Multi-Dose Toxicokinetic in Rats

One purpose of this example was to evaluate the toxicity of the test article, P2203, administered once weekly via subcutaneous (s.c.) injection to CD (trademarked) (Sprague Dawley) IGS rats for a total of 4 times during consecutive 28 days and to assess the reversibility, persistence, or delayed occurrence of toxic effects following a 4-week recovery period. The toxicokinetic (TK) profile and immunogenicity of the test article were also determined. A total of 142 animals were randomly assigned to four groups, and doses were administered as indicated in Table 13. Each group includes a toxicity (Tox) portion and a toxicokinetic (TK) portion.

**Table 13**

| **Group** | **Dose Level (µg/kg/week) ^{f}** | **No. of Animals ^{b}** | | | | |
|---|---|---|---|---|---|---|
| | | **Toxicity (Tox)** | | **Toxicokinetic (TK)** | | |
| | | **Main study** | **Recovery** | **Sub 3 ^{c}** | **Sub 4 ^{d}** | **Sub 5 ^{e}** |
| | | **Sub 1** | **Sub 2** | | | |
| 1 (Control) ^{a} | 0 | 10 M + 10 F | 5M+5F | 3M+3F | - | - |
| 2 (Low-dose) | 100 | 10 M + 10 F | - | - | 3M+3F | 3M+3F |
| 3 (Mid-dose) | 300 | 10 M + 10 F | - | - | 3M+3F | 3M+3F |
| 4 (High-dose) | 1000 | 10 M + 10 F | 5 M + 5 F | - | 3M+3F | 3M+3F |
| ^{a} P2203 FB. | | | | | | |
| ^{b} M = male; F = female; Sub = subgroup. | | | | | | |
| ^{c} Sub 3 animals (control animals): For blood sampling after the 1^{st} and the last dosing. | | | | | | |
| ^{d} Sub 4 animals: For Days 1-7 blood sampling after the 1^{st} dosing. Animals were be sacrificed after Day 7 blood sampling. | | | | | | |
| ^{e} Sub 5 animals: For Days 8-57 blood sampling. Animals were sacrificed after Day 57 blood sampling. | | | | | | |
| ^{f} Acceptable ranges of calculated dose levels during preparation are ± 1%. | | | | | | |

Parameters evaluated in this example included mortality, body weights, food consumption, clinical observations, local tolerance, ophthalmology, central nervous system (CNS) evaluation (motor activity and functional observation battery), clinical pathology (hematology, coagulation, serum chemistry, and urinalysis), gross observation, organ weights, and histopathology evaluation. Blood samples were collected for bioanalysis, toxicokinetic evaluations, and immunogenicity analysis (detection of anti-drug antibodies (ADA)).

Animals were randomly assigned into four groups before dosing. The body weight variation of animals used fell within an interval ± 20 percent of the mean weight for each sex. The basic design is presented in Table 14 below.

**Table 14**

| **Group** | **Dose Level (µg/kg/week) ^{f}** | **Dose Conc. (µg/mL) ^{f}** | **Dose Volume (mL/kg)** | **No. of Animals ^{b}** | | | | |
|---|---|---|---|---|---|---|---|---|
| | | | | **Toxicity (Tox)** | | **Toxicokinetic (TK)** | | |
| | | | | **Main study** | **Recovery** | **Sub 3 ^{c}** | **Sub 4 ^{d}** | **Sub 5 ^{e}** |
| | | | | **Sub 1** | **Sub 2** | | | |
| 1 (Control) ^{a} | 0 | 0 | 0.5 | 10 M + 10 F | 5 M + 5 F | 3M+3F | - | - |
| 2 (Low-dose) | 100 | 200 | 0.5 | 10 M + 10 F | - | - | 3M+3F | 3M+ 3F |
| 3 (Mid-dose) | 300 | 600 | 0.5 | 10 M + 10 F | - | - | 3M+3F | 3M+ 3F |
| 4 (High-dose) | 1000 | 2000 | 0.5 | 10 M + 10 F | 5 M + 5 F | - | 3M+3F | 3M+ 3F |
| ^{a} P2203 FB. | | | | | | | | |
| ^{b} M = male; F = female; Sub = subgroup. | | | | | | | | |
| ^{c} Sub 3 animals (control animals): For blood sampling after the 1^{st} and the last dosing. | | | | | | | | |
| ^{d} Sub 4 animals: For Days 1-7 blood sampling after the 1^{st} dosing. Animals were be sacrificed after Day 7 blood sampling. | | | | | | | | |
| ^{e} Sub 5 animals: For Days 8-57 blood sampling. Animals were sacrificed after Day 57 blood sampling. | | | | | | | | |
| ^{f} Acceptable ranges of calculated dose levels during preparation are ± 1%. | | | | | | | | |

Subcutaneous injections (s.c.) was performed at animals' dorsal neck area with the volume of 0.5 mL/kg. Before each injection, the hair of animals was removed from the proposed injection area (at least 3 cm × 3 cm) with a small animal clipper. The accuracy of 0.3-mL syringes (needle size: 31G) used in this study is nearest 0.005 mL. After injection, the injection site was pressed tightly for around 30 seconds to prevent leakage of the injected liquid.

All animals were dosed four times (Days 1, 8, 15 and Day 22) with the designated dose levels listed. The first dosing day was denoted as Study Day 1 (Day 1). The first recovery day was denoted as Recovery Day 1 (RD 1). All measured parameters were calculated and expressed as mean ± standard deviation or percentage.

Comparisons of parametric data collected from treated and control groups were performed by One-Way ANOVA method, followed by Dunnett's method (Pristima (trademark) Version 7.5.0 Build 15.0, Xybion Medical System Corporation). In FOB evaluation, Kruskal-Wallis test (except respiration rate, grip strength, and absolute body temperature were analyzed by ANOVA, SPSS (trademark), version 12.0) was employed. Parameters in motor activity were analyzed by ANOVA, SPSS (trademark), version 22.0. In urinary sediment examination, Kruskal-Wallis test was employed. Probability of 0.05 (p ≦ 0.05) was used as the criterion of significance.

### Results

The summary of TK parameters of P2203 in male rats (Mean ± SD, n = 3) are shown in Table 15 below.

**Table 15**

| **Dose level (µg/kg/week)** | **Dosing Day** | **Cₘₐₓ (pg/mL)** | **AUC₍₀₋ₜ₎ (hr*pg/mL)** | **AUC_{(0-∞)} (hr*pg/mL)** | **Tₘₐₓ (hr)*** | **T_{1/2} (hr)** | **CL/F (mL/hr/kg)** | **D22/D1 ratio for AUC₍₀₋ₜ₎** |
|---|---|---|---|---|---|---|---|---|
| 100 | 1 | 224797 ± 70729 | 6429500 ± 2621082 | 6439692 ± 2621052 | 24 | 6.81 ± 2.31 | 17.08 ± 5.75 | 0.3 |
| | 22 | 72983 ± 16271 | 1932411 ± 458146 | 1950592 ± 466009 | 12 | 12.52 ± 1.08 | 53.64 ± 14.86 | |
| 300 | 1 | 1171655 ± 26999 | 41933582 ± 2383592 | 41940793 ± 2385700 | 24 | 5.34 ± 0.13 | 7.17 ± 0.41 | 0.7 |
| | 22 | 893939 ± 567437 | 27275686 ± 19431181 | 27289222 ± 19432424 | 12 | 16.59 ± 1.54 | 16.85 ± 13.53 | |
| 1000 | 1 | 4033140 ± 1344294 | 219390554 ± 93124141 | 219397253 ± 93123744 | 24 | 6.19 ± 0.41 | 5.07 ± 1.84 | 0.5 |
| | 22 | 2842763 ± 814413 | 104311911 ± 28948331 | 104325822 ± 28947301 | 24 | 18.05 ± 1.13 | 10.05 ± 2.55 | |
| * Tₘₐₓ presented as median. | | | | | | | | |

The summary of TK parameters of P2203 in female rats (Mean ± SD, n = 3) are shown in Table 16 below.

**Table 16**

| **Dose level (µg/kg/ week)** | **Dosing Day** | **Cₘₐₓ (pg/mL)** | **AUC₍₀₋ₜ₎ (hr*pg/mL)** | **AUC_{(0-∞)} (hr*pg/mL)** | **Tₘₐₓ (hr)*** | **T_{1/2} (hr)** | **CL/F (mL/hr/kg )** | **D22/D1 ratio for AUC₍₀₋ₜ₎** | **F/M AUC ₍₀₋ₜ₎ ratio** |
|---|---|---|---|---|---|---|---|---|---|
| 100 | 1 | 269319 ± 86862 | 7499999 ± 2604795 | 7510697 ± 2605697 | 12 | 6.86 ± 1.56 | 14.55 ± 5.46 | 0.7 | 1.2 |
| | 22 | 247814 ± 50924 | 5240322 ± 775499 | 5262143 ± 778981 | 12 | 12.24 ± 1.32 | 19.30 ± 2.96 | | 2.7 |
| 300 | 1 | 1107374 ± 184624 | 43917107 ± 9880443 | 43925385 ± 9881053 | 24 | 5.26 ± 0.25 | 7.05 ± 1.50 | 0.7 | 1.0 |
| | 22 | 1013015 ± 417627 | 30603966 ± 14196148 | 30623831 ± 14200958 | 24 | 13.41 ± 1.12 | 12.14 ± 7.60 | | 1.1 |
| 1000 | 1 | 3707717 ± 220644 | 196004677 ± 20870474 | 196010323 ± 20870482 | 24 | 5.95 ± 0.79 | 5.14 ± 0.52 | 1.0 | 0.9 |
| | 22 | 4833593 ± 93086 | 204245392 ± 9248566 | 204260007 ± 9244458 | 24 | 11.41 ± 3.99 | 4.90 ± 0.23 | | 2.0 |
| Tₘₐₓ presented as median. | | | | | | | | | |
| F/M: Female/Male. | | | | | | | | | |

### Day 1 (single dose) analysis

After the first administration, the Cₘₐₓ and AUC₍₀₋ₜ₎ increased in a greater than dose-proportional manner with the three dose levels in both male and female rats. The clearance (CL/F) decreased from 17.08 to 5.07 mL/hr/kg in males and from 14.55 to 5.14 mL/hr/kg in females when administered doses elevated from 100 to 1000 µg/kg/week. There was no apparent sex difference in the AUC₍₀₋ₜ₎. The half-life of P2203 after the first administration ranged from 5.26 to 6.86 hours.

### Day 22 (repeated dose) analysis

After the treatment with weekly repeated dose for four consecutive weeks, Cₘₐₓ values (repeated dose vs. single dose) were 72983 vs. 224797, 893939 vs. 1171655, and 2842763 vs. 4033140 pg/mL in male rats and were 247814 vs. 269319, 1013015 vs. 1107374, and 4833593 vs. 3707717 pg/mL in female rats at 100, 300, and 1000 µg/kg/week, respectively. AUC₍₀₋ₜ₎ values (repeated dose vs. single dose) were 1932411 vs. 6429500, 27275686 vs. 41933582, and 104311911 vs. 219390554 hr*pg/mL in males and were 5240322 vs. 7499999, 30603966 vs. 43917107, and 204245392 vs. 196004677 hr*pg/mL in females at 100, 300, and 1000 µg/kg/week, respectively.

After four-week repeated dosing, Cₘₐₓ and AUC₍₀₋ₜ₎ increased in a greater than dose-proportional manner with the three dose levels in both male and female rats.

The clearance (CL/F) after four-week repeated dosing decreased from 53.64 to 10.05 mL/hr/kg in males and from 19.30 to 4.90 mL/hr/kg in females when administered doses elevated from 100 to 1000 µg/kg/week. The half-life of P2203 after the fourth dosing ranged from 11.41 to 18.05 hours.

Generally, P2203 did not accumulate in both sexes following the repeated dosing. AUC₍₀₋ₜ₎ on Day 1 showing no apparent sex difference. However, female AUC₍₀₋ₜ₎ was higher than male AUC₍₀₋ₜ₎ on Day 22 with Female/Male AUC₍₀₋ₜ₎ ratios of 2.7, 1.1 and 2.0 at 100, 300 and 1000 µg/kg/week, respectively.

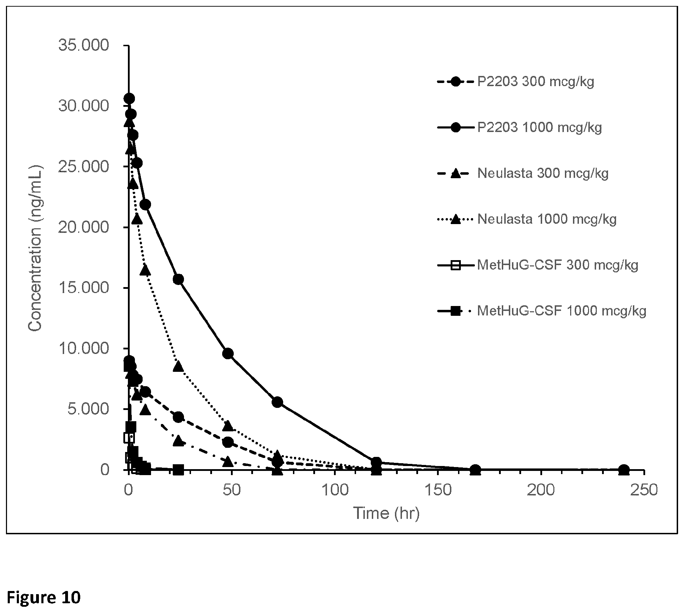

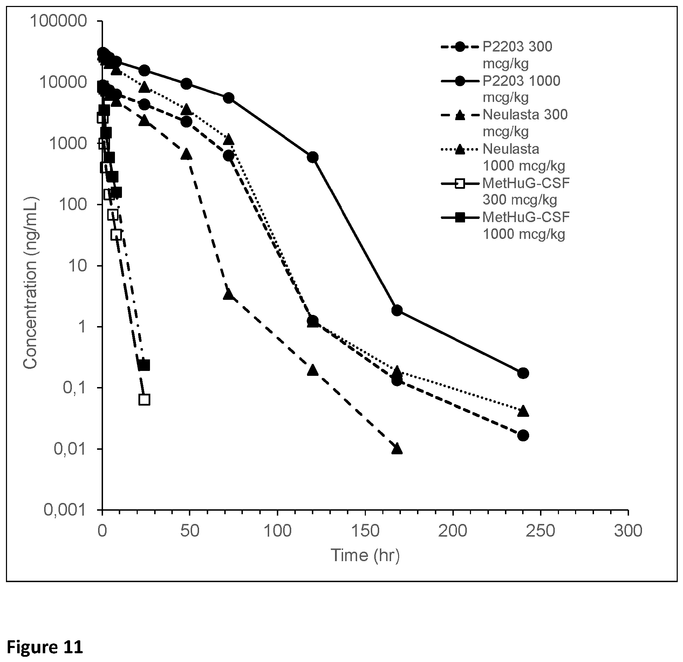

Plasma concentrations were evaluated in rats. Figure 10 shows the mean plasma concentration-time profiles of MetHuG-CSF, PEGfilgrastim, and mPEG-MetHuG-CSF in rats after intravenous administration (n=5, except for mPEG-MetHuG-CSF 1000mcg/mL, n=10). Figure 11 shows the mean log plasma concentration-time profiles of all test formulation of mPEG-MetHuG-CSF in rats after intravenous administration (n=5, except for mPEG-MetHuG-CSF 1000mcg/mL n=10).

### Immunogenicity analysis

No anti-PEG antibody was detected by the confirmatory assay.

Overall, positive ADA results for GCSF were detected in a few animals from the test article-treated groups during the dosing phase on Days 7 or 29, but with low incidences. The exposure (AUC₀₋ₜ) values of P2203 were lower on Day 22 than on Day 1, except for the data of female Group 4 animals. The decrease in exposures on Day 22 may have been influenced by the presence of ADA, but no connection or evidence in the presence of ADAs with P2203 exposures.

During the evaluation, no animal was found dead or early sacrificed in all groups. No test article-related findings were noted in body weight, body weight gains, food consumption, clinical observation, local tolerance, ophthalmologic examination, CNS evaluation, coagulation, and urinalysis.

Test article-related hematology effects, including increases in the values of RET, WBC, NEU, LYM (female only), MON, EOS and BAS, were noted in Group 3 and Group 4 main study animals, which were generally dose dependent. These findings were correlated with histopathologic findings including hypercellularity in the bone marrow and increased extramedullary hematopoiesis in the spleen and liver. Test article-related serum chemistry effect, increased ALP, was noted in Groups 3 and 4 main study animals. These changes were considered pharmacological effects of the P2203 administration and exhibited reversibility in recovery animals.

In histopathologic evaluation of main study animals, hypercellularity in the bone marrow and increased extramedullary hematopoiesis in the spleen and liver were noted in all test article-treated groups in both sexes with dose dependence and correlated with gross or hematologic changes. Femur bone marrow smear examination results indicated an increased M:E (Myeloid:Erythroid) ratio in Group 3 and Group 4 main study animals. In the recovery animals, there was a low severity and incidence of hypercellularity in the bone marrow and extramedullary hematopoiesis in the spleen, suggesting partial reversibility. Complete recovery of extramedullary hematopoiesis in the liver was noted. As a result, they were considered test article-related pharmacological effects, not of toxicological significance.

The systemic exposures (Cₘₐₓ and AUC₍₀₋ₜ₎) of P2203 increased in a greater than dose-proportional manner in both sexes on Day 1 and Day 22. The P2203 AUC₀₋ₜ values were generally lower on Day 22 than on Day 1, except for the data of female Group 4 animals, indicating no accumulation of P2203 after repeated dosing. The decrease in AUC₀₋ₜ on Day 22 may have been influenced by the presence of ADA, but no connection or evidence in the presence of ADAs with P2203 systemic exposures was found.

Based on the results on hematology, organ weights and histopathology, the NOAEL (no-observable-adverse-effect-level) of P2203 in this study was the high-dose (1000 µg/kg/week) in both sexes. This dose level corresponded to male and female mean maximum observed concentration (Cₘₐₓ) of 2842763 pg/mL and 4833593 pg/mL, respectively, and area under the concentration time curve (AUC₀₋ₜ) of 104311911 hr*pg/mL and 204245392 hr*pg/mL, respectively, on Day 22 of the dosing phase.

In this rat example, no P2203-related effects on the function of the CNS (functional observational battery and motor activity included) at all dose levels (0.1, 0.3, or 1 mg/kg) were noted. Positive ADA results for MetHuG-CSF were detected in a few animals from the P2203-treated groups during the dosing phase on Days 7 or 29, but with low incidences. The exposure (AUC₀₋ₜ) values of P2203 were lower on Day 22 than on Day 1, except for the data of female animals administered 1000 µg/kg/week. The decrease in exposures on Day 22 may have been influenced by the presence of ADA, but no strong connection or evidence of the presence of ADAs with P2203 exposures. No anti-PEG antibody was detected by the confirmatory assay.

### Example 6

### Pharmacokinetic in Monkey of P2203 and pegfilgrastim

One purpose of this example was to assess the pharmacokinetics of P2203 when administered to Cynomolgus monkeys compared with pegfilgrastim following a single subcutaneous injection at 0.1 mg/kg, 0.3 mg/kg and 1.0 mg/kg.

A total of 20 monkey (10/sex) were assigned into 5 groups, 2/sex/group: P2203 FB group, P2203 0.1 mg/kg group, P2203 0.3 mg/kg group, P2203 1.0 mg/kg group and pegfilgrastim 0.3 mg/kg group with subcutaneous administration. Blood samples for bioanalysis from each group were collected from 15 time points. Blood samples for anti-drug antibody from each group were collected from 3 time points. The anti-drug antibody in serum was determined using validated ELISA method. Pharmacokinetic parameters were determined using non-compartmental modelling analysis module of WinNonlin software.

There is no obvious sex difference in the main pharmacokinetic parameters in each dose group. Therefore, the mean pharmacokinetic parameters are presented using the combined value of male and female as below Table 17.

**Table 17**

| | | | | | | | | |
|---|---|---|---|---|---|---|---|---|
| **Group** | **2** | | **3** | | **4** | | **5** | |
| **Test Article** | **P2203** | | **P2203** | | **P2203** | | **pegfilgrastim** | |
| **Dose Route** | **SC** | | **SC** | | **SC** | | **SC** | |
| **Dose level (mg/kg)** | **0.1 mg/kg** | | **0.3 mg/kg** | | **1.0 mg/kg** | | **0.3 mg/kg** | |

| **PK parameters** | **Mean** | **SD** | **Mean** | **SD** | **Mean** | **SD** | **Mean** | **SD** |
|---|---|---|---|---|---|---|---|---|
| **Cₘₐₓ (ng/mL)** | 696 | 297 | 2840 | 420 | 11300 | 745 | 2050 | 789 |
| **Tₘₐₓ (h)** | 13.5 | 7.00 | 30.0 | 12.0 | 48.0 | 0.00 | 13.5 | 7.00 |
| **T_{1/2} (h)** | 10.1 | 2.19 | 10.2 | 2.12 | 10.4 | 1.63 | 6.73 | 1.28 |
| **Vd (L/kg)** | 0.0911 | 0.100 | 0.0243 | 0.00745 | 0.0144 | 0.00204 | 0.0363 | 0.0122 |
| **CL (mL/min/kg)** | 0.0964 | 0.0958 | 0.0271 | 0.00356 | 0.0162 | 0.00305 | 0.0616 | 0.0156 |
| **AUC₀₋ₗₐₛₜ (ng'h/mL)** | 27700 | 14400 | 187000 | 28300 | 938000 | 280000 | 79800 | 20700 |
| **AUC_{0-inf} (ng'h/mL)** | 27800 | 14400 | 187000 | 28300 | 1050000 | 193000 | 84900 | 22200 |

After single subcutaneous injection of P2203 at three dose levels (0.1 mg/kg, 0.3 mg/kg and 1.0 mg/kg), the sex combined mean Cₘₐₓ of P2203 were 696 ng/mL, 2840 ng/mL and 11300 ng/mL, the sex combined mean AUC₀₋ₗₐₛₜ were 27700 hr*ng/mL, 187000 hr*ng/mL and 938000 hr*ng/mL, respectively. After single subcutaneous injection of pegfilgrastim at 0.3 mg/kg, the sex combined mean Cₘₐₓ and AUC₀₋ₗₐₛₜ of pegfilgrastim were 2050 ng/mL and 79800 hr*ng/mL respectively.

The Cₘₐₓ of P2203 increased proportionally as dose levels increased within the dose range from 0.1 mg/kg to 1.0 mg/kg. The AUC₀₋ₗₐₛₜ of P2203 increased higher than the increase of dose level. The AUC₀₋ₗₐₛₜ of P2203 was higher than that of pegfilgrastim at 0.3 mg/kg.

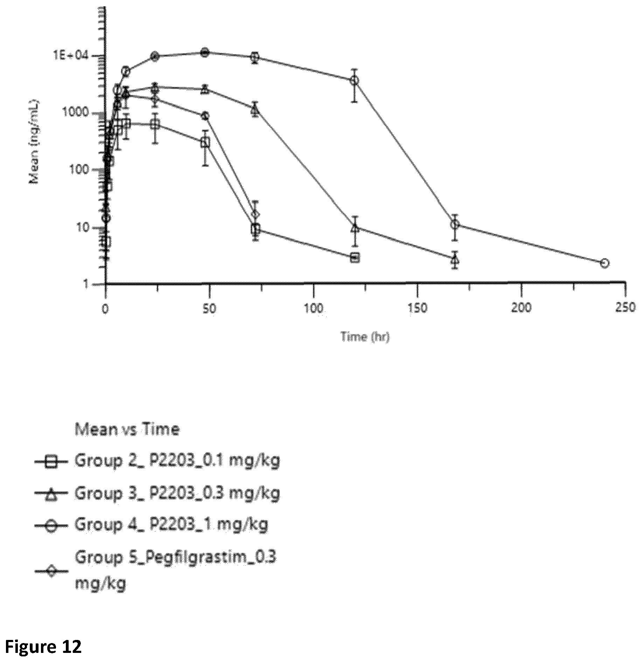

Following single subcutaneous injection of P2203 at 0.1, 0.3, and 1.0 mg/kg and pegfilgrastim at 0.3 mg/kg, the incidence of anti-GCSF antibody ratios were 1/4 (25.0%), 1/4 (25.0%), 2/4 (50.0%) and 2/4 (50.0%), respectively. The incidence of anti-PEG antibody in each group were 0/4 (0.00%), 3/4 (75.0%), 3/4 (75.0%) and 4/4 (100%), respectively. ADA did not show apparent impact on overall PK behavior evaluation. Figure 12 shows a graph of Pharmacokinetic profile of P2203 and pegfilgrastim following a single SC Injection asMean serum concentrations (ng/mL) vs. Time of P2203 and pegfilgrastim in monkey serum.

### Example 7

### Pharmacodynamics Comparison in Monkey of P2203 and pegfilgrastim

This study includes P2203 0.1 mg/kg group, P2203 0.3 mg/kg group, P2203 1 mg/kg group and pegfilgrastim 0.3 mg/kg group with single SC injection. Blood samples were collected at predose, 24, 48, 72, 120, and 168 hours postdose to measure the white blood cell (WBC) and absolute neutrophil counts (ANC), serving as the PD markers. Results of White Blood Cell (WBC) Counts of P2203 and pegfilgrastim are shown in Table 18.

**Table 18.**

| **WBC (10³/µL)** | | | | | | |
|---|---|---|---|---|---|---|
| **Group** | **Predose** | **Day 2 (24hr)** | **Day 3 (48hr)** | **Day 4 (72hr)** | **Day 5 (120hr)** | **Day 8 (168hr)** |
| Vehicle | 11.283 ± 5.312 | 9.398 ± 2.761 | 8.528 ± 1.778 | 7.88 ±2.253 | 8.098 ± 1.152 | 10.225 ± 3.843 |
| P2203, 0.1 mg/kg | 12.348 ± 4.672 | 43.365 ± 13.743* | 42.055 ± 10.827* | 39.348 ± 16.34* | 28.163 ± 12.384* | 25.225 ± 9.018* |
| P2203, 0.3 mg/kg | 12.57 ± 3.873 | 46.12 ± 10.894* | 62.48 ± 14.925* | 73.95 ± 19.276* | 55.858 ± 14.543*^{a} | 45.668 ± 17.327* |
| P2203, 1 mg/kg | 13.448 ± 3.551 | 40.358 ± 21.524* | 42.393 ± 24.695* | 52.728 ± 22.922* | 103.21 ± 20.132* | 73.167 ± 22.44* |
| pegfilgrastim, 0.3 mg/kg | 17.115 ± 5.971 | 47.245 ± 8.941* | 50.878 ± 16.791* | 66.485 ± 20.221* | 36.475 ± 9.836* | 33.953 ± 9.922* |

| | | | | | | |
|---|---|---|---|---|---|---|
| *: p ≦ 0.05 (One-Way ANOVA, compared to the vehicle group) ^{a}: p ≦ 0.05 (Student t-test; P2203, 0.3 mg/kg compared to the pegfilgrastim, 0.3 mg/kg group) | | | | | | |

Results of Absolute Neutrophil Counts (ANC) of P2203 and pegfilgrastim are shown in Table 19.

**Table 19.**

| **ANC (10³/µL)** | | | | | | |
|---|---|---|---|---|---|---|
| **Group** | **Predose** | **Day 2 (24hr)** | **Day 3 (48hr)** | **Day 4 (72hr)** | **Day 5 (120hr)** | **Day 8 (168hr)** |
| Vehicle | 4.32 ± 4.4 | 4.17 ± 2.142 | 2.528 ± 0.761 | 3.558 ± 1.85 | 3.915 ± 0.678 | 5.453 ± 3.238 |
| P2203, 0.1 mg/kg | 4.543 ± 2.289 | 36.728 ± 12.217* | 32.99 ± 9.59* | 27.73 ± 12.428* | 19.243 ± 9.057* | 17.1 ± 6.104* |
| P2203, 0.3 mg/kg | 4.165 ± 2.549 | 39.618 ± 9.856* | 51.505 ± 12.484* | 60.498 ± 16.242* | 39.763 ± 13.824* ^{a} | 32.593 ± 14.451 * |
| P2203, 1 mg/kg | 5.023 ± 2.241 | 35.94 ± 19.97* | 34.905 ± 22.026* | 42.35 ± 21.028* | 90.01 ± 20.546* | 59.953 ± 19.129* |
| pegfilgrastim, 0.3 mg/kg | 6.478 ± 4.639 | 40.205 ± 8.719* | 39.495 ± 16.1* | 51.178 ± 15.879* | 20.723 ± 6.443* | 21.838 ± 8.174* |

| | | | | | | |
|---|---|---|---|---|---|---|
| *: p ≦ 0.05 (One-Way ANOVA, compared to the vehicle group) ^{a}: p ≦ 0.05 (Student t-test; P2203, 0.3 mg/kg compared to the pegfilgrastim, 0.3 mg/kg group) | | | | | | |

Animals administered P2203 at doses of 0.1, 0.3, and 1 mg/kg or pegfilgrastim at 0.3 mg/kg exhibited markedly increased in WBC and neutrophil counts from Day 2 throughout the study period compared to the vehicle control animals. Pharmacodynamic responses were dose-related for P2203.

There were similar WBC and ANC values between the 0.3 mg/kg of P2203 and pegfilgrastim groups on Day 2; however, P2203 showed higher WBC and ANC counts from Day 3 to Day 8. These results indicated that P2203 has a better capability to stimulate WBC and ANC production than pegfilgrastim and can extend the duration of its therapeutic effects at the same dose levels.

### Summary

The PK data from the rat study showed linear exposures (Cₘₐₓ and AUCₗₐₛₜ) of P2203 for the IV route at dose levels of 300 and 1000 mcg/kg. Furthermore, P2203 demonstrated 1.8-fold or 1.9-fold higher in AUCₗₐₛₜ values than pegfilgrastim at 300 or 1000 mg/kg doses, respectively.

The PK data of the monkey study showed a nonlinear response, with Cₘₐₓ and AUC₀₋ₗₐₛₜ increasing more than in a dose-proportional manner for SC injection of P2203 from 0.1 mg/kg to 1 mg/kg. No obvious sex difference was observed. The ratio of low (0.1 mg/kg), middle (0.3 mg/kg), and high (1 mg/kg) dose level was 1: 3: 10, and the corresponding ratios for Cₘₐₓ and AUC₀₋ₗₐₛₜ were 1:4.08:16.2 and 1:6.75:33.9 respectively. In addition, the ratio of Cₘₐₓ and AUC₀₋ₗₐₛₜ comparison of P2203 and pegfilgrastim at 0.3 mg/kg were 1.39 and 2.34 respectively.

P2203's PK/PD are dose dependent in three dose level groups. The AUC of P2203 is approximately 2 folds higher than pegfilgrastim in 300 mcg/kg group. The WBC and ANC were increased after administration of P2203/pegfilgrastim in all groups. The WBC and ANC were significantly different (p<0.05) between P2203 and pegfilgrastim in 300 mcg/kg group at 120h, and the PD profiles are similar to rat study.

It was demonstrated that the time to reach the peak of WBC and ANC responses from P2203 is one day later than that of pegfilgrastim at 1000 mcg/kg, which represents P2203's superior and surprising capacity to stimulate WBC and ANC production compared to pegfilgrastim at the same dose levels with an extended duration of therapeutic effects.

### Example 8

### A 4-Week Subcutaneous Injection Toxicity and Toxicokinetic Study with P2203 in Cynomolgus Monkeys

In this example, P2203 was administered once weekly via SC injection to male and female cynomolgus monkeys for 4 weeks (total of 5 doses) at a dose level of 0.1, 0.3, or 1 mg/kg/dose.

No P2203-related mortality occurred. One female administered 0.1 mg/kg/dose was sacrificed in a moribund condition on Day 29 of the dosing phase; the cause of the moribund condition was undetermined based on anatomic pathology and considered not P2203 related because similar outcomes were not noted in any other animals on study, particularly at higher dose levels. Red or dark red semifluid content was observed macroscopically in the stomach and colon, without microscopic correlates, but a correlate with the clinical pathology finding of internal hemorrhage changes included moderately to markedly decreased red cell mass. The microscopic findings were consistent with similar P2203-related findings in terminal sacrifice animals administered ≥0.1 mg/kg/dose. P2203-related clinical pathology effects in this female were similar (i.e., affected parameters and magnitude of changes) to those observed in other scheduled sacrifice animals administered 0.1 mg/kg/dose. All other animals survived to their scheduled sacrifice.

No P2203-related clinical observations; changes in body weights or food consumption; ophthalmic observations; dose site observations; changes in body temperature, blood pressure, respiration rate, or pulse oximetry; neurobehavioral observations; coagulation or urinalysis; or effects on immunotoxicology (blood immunophenotyping and cytokines) were noted in scheduled sacrifice animals.

P2203 was associated with higher mean heart rate (up to +35 bpm) and shorter mean QT interval (as short as -36 msec) from 48 through 67 hours postdose on Days 1 and 22 of the dosing phase in males given 1 mg/kg/dose. Females administered 1 mg/kg/dose showed higher mean heart rate on Day 22 of the dosing phase. These changes were compared with controls. While not consistently observed in all individual animals, the alterations in heart rate and QT interval were reversed during the recovery phase. The higher heart rates and shorter QT intervals on Days 1 and 22 in males and higher heart rates on Day 22 in females administered 1 mg/kg/dose were attributed to P2203 due to the magnitude of effect and the dose dependence.

P2203 had no effect on heart rate in animals administered 0.1 or 0.3 mg/kg/dose or on QT interval in males administered 0.1 or 0.3 mg/kg/dose or females administered 0.1, 0.3, or 1 mg/kg/dose. No P2203-related changes in PR interval, QRS duration, or corrected QT (QTc) interval were observed in animals administered 0.1, 0.3, or 1 mg/kg/dose. No abnormal waveforms or arrhythmias were attributed to P2203 during the qualitative assessment of the ECGs.

P2203-related hematology effects observed on Days 3, 8, 15, 24, and 30 in scheduled sacrifice animals administered ≥0.1 mg/kg/dose were generally dose-dependent and consistent with the pharmacologic effects of P2203. These effects included mildly to markedly increased white blood cell, neutrophil, monocyte, basophil, and large unstained cell counts. These effects were generally of greatest magnitude on Day 24 of the dosing phase. These white blood cell effects correlated with microscopic findings of increased intravascular leukocytes in the spleen, lung, and/or other tissues and increased cellularity in the sternum bone marrow. Minimally to mildly increased lymphocyte count was observed on Days 8 and 15 (females only) of the dosing phases in animals administered ≥0.1 mg/kg/dose. Additional hematology effects included mildly to moderately decreased red cell mass (i.e., red blood cell count, hemoglobin concentration, and hematocrit) and increased red cell distribution width on Day 15 of the dosing phase in males administered 1 mg/kg/dose and on Days 24 and 30 of the dosing phases in animals administered ≥0.1 mg/kg/dose. Mildly decreased reticulocyte count (compared to controls) was observed on Days 8 and 15 (females only) of the dosing phases in animals administered ≥0.1 mg/kg/dose, followed by mildly increased reticulocyte count on Day 24 of the dosing phase in animals administered 1 mg/kg/dose and on Day 30 of the dosing phase in animals administered ≥0.3 mg/kg/dose. Increased reticulocyte count correlated with microscopic findings of increased cellularity in the sternum bone marrow and increased extramedullary hematopoiesis in the spleen, mandibular lymph node, liver, kidney, pancreas, and/or oviduct. An additional hematology effect was mildly decreased platelet count on Day 8 of the dosing phase in males administered 1 mg/kg/dose and females administered ≥0.3 mg/kg/dose. P2203-related clinical chemistry effects on Day 30 of the dosing phase were mild in magnitude and included decreased albumin concentration in females administered 1 mg/kg/dose and increased globulin concentration and decreased albumin:globulin ratio in males administered ≥0.3 mg/kg/dose. These hematology and clinical chemistry effects exhibited evidence of reversibility by Day 29 of the recovery phase.

At the end of the example procedure, P2203-related findings were noted in the thymus, spleen, and sternum bone marrow; in addition, extramedullary hematopoiesis was noted in some tissues. Minimally to slightly decreased lymphocytes in the cortex of the thymus were noted in females administered 1 mg/kg/dose and correlated with reduced organ weights and the macroscopic finding of small thymus in one female. Slight hypertrophy in the adrenal cortex was noted in one female administered 1 mg/kg/dose. These effects on the thymus and adrenal were considered secondary to stress, although a direct effect of P2203 could not be completely excluded. Minimally to slightly increased intravascular leukocytes in the spleen and lung were noted in animals administered ≥0.1 mg/kg/dose and correlated with increased spleen weights and with the clinical pathology finding of increased white blood cells. Minimally to slightly increased cellularity in the sternum bone marrow was noted in animals administered ≥0.1 mg/kg/dose and correlated with clinical pathology findings of increased white blood cell and reticulocyte counts. Minimally to slightly increased extramedullary hematopoiesis in the spleen, lymph node (mandibular), liver, kidney, pancreas, and/or oviduct was noted in males administered 1 mg/kg/dose and females administered ≥0.3 mg/kg/dose and correlated with the clinical pathology finding of increased reticulocyte count. These effects on the blood, bone marrow, spleen, lymph node, liver, kidney, pancreas, and oviduct were consistent with the expected pharmacology and considered nonadverse due to their low severity. No P2203-related organ weight effects or macroscopic or microscopic findings were noted at the recovery sacrifice, indicating full reversal. Results are summarized in the below Table 20.

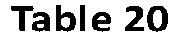

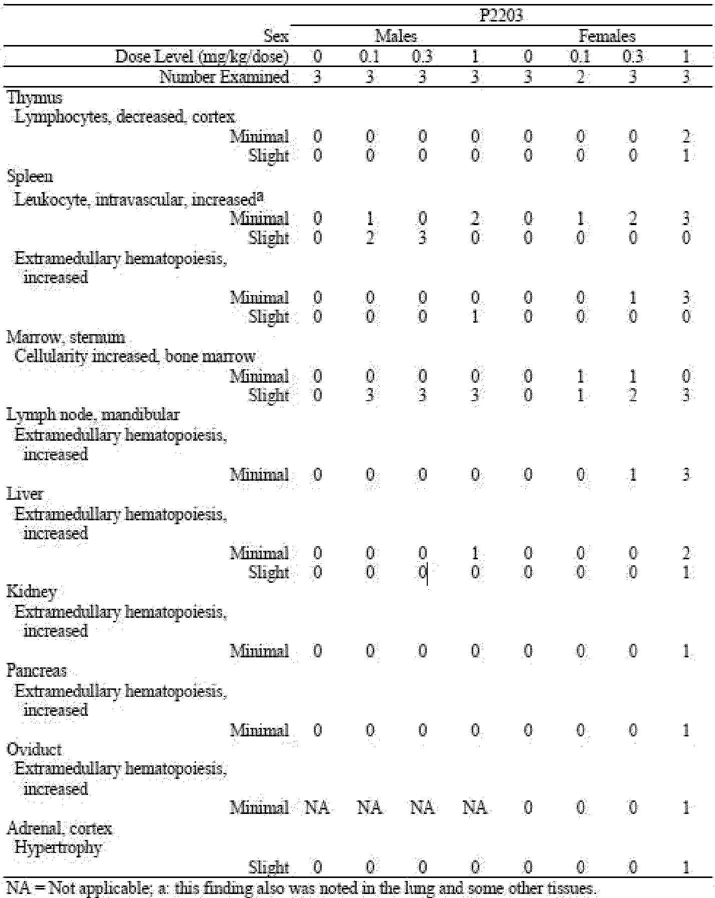

The TK profile showed sex differences in P2203 mean Cₘₐₓ and AUC₀₋₁₆₈ values being less than 2-fold, except sex differences in P2203 mean Cₘₐₓ values was little greater than 2-fold for group 4 on Day 22 (the ratio was 0.491); Results and discussion were based on combined sex values.

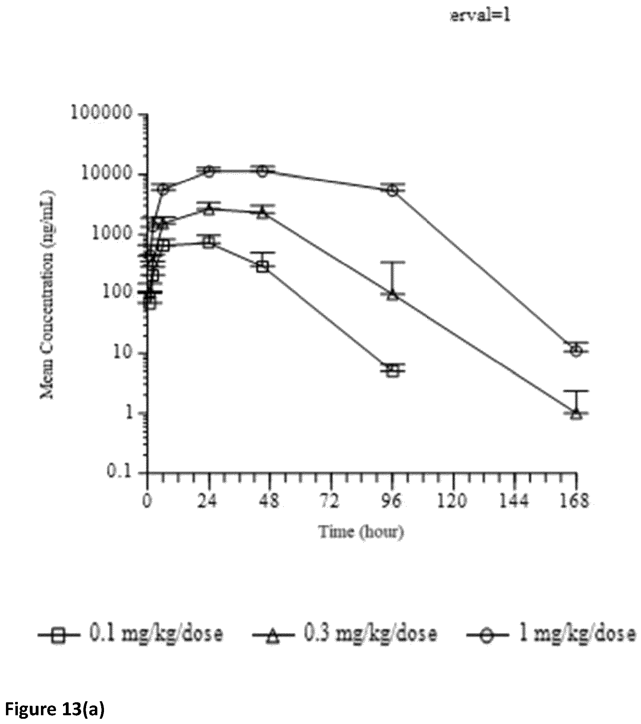

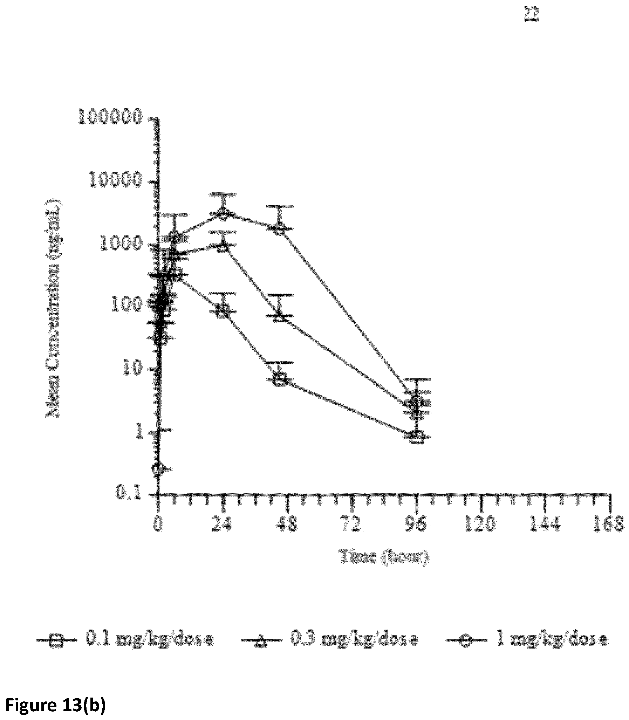

Figure 13(a) and Figure 13(b) show combined male and female P2203 mean serum concentrations (ng/mL) vs time (hr) in combined male and female monkeys following subcutaneous injection on Day 1 and Day 22, respectively.

The mean concentration-time profiles for combined sexes showed that mean concentrations of P2203 generally increased with the increase in dose level from 0.1 to 1 mg/kg/dose and were generally lower after multiple doses compared to a single dose.

The summary of the mean P2203 TK parameters are shown in Table 21 below.

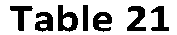

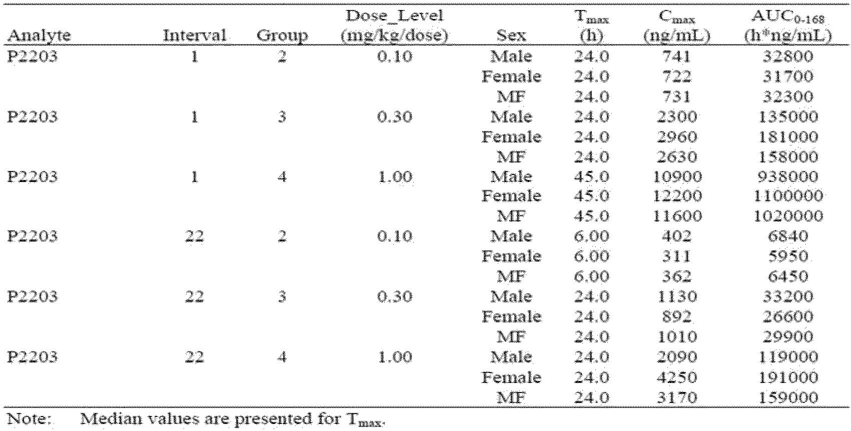

Exposure, as assessed by P2203 mean Cₘₐₓ and AUC₀₋₁₆₈ values, increased with the increase in dose level from 0.1 to 1 mg/kg/dose. The increases in P2203 mean Cₘₐₓ and AUC₀₋₁₆₈ values were generally dose-proportional except, increases in P2203 mean AUC₀₋₁₆₈ values were greater-than-dose proportional for animals administered 1 mg/kg/dose compared to animals administered 0.1 mg/kg/dose.

P2203 mean Cₘₐₓ and AUC₀₋₁₆₈ values were generally lower on Day 22 than on Day 1, indicating no accumulation of P2203 after multiple doses. Mean accumulation ratio values ranged from 0.264 to 0.599 for Cₘₐₓ and from 0.155 to 0.262 for AUC₀₋₁₆₈.

In conclusion, the NOAEL was 1 mg/kg/dose. This dose level corresponded to mean sex combined mean maximum observed concentration (Cₘₐₓ) and area under the concentration time curve (AUC₀₋₁₆₈) of 3170 µg/mL and 159000 hour*µg/mL, respectively, on Day 22 of the dosing phase.

Positive ADA result of PEG was detected for all groups in dosing phase, and only for dosing groups in recovery phase. The titer values which ranged from MRD (50) to 167, were in a lower level for control group, and no strong evidence connected titer level to doses. The ADA result of MetHuG-CSF on Day 29 of the dosing phase indicates that all animals tested positive, except 2 animals: 1 male administered 0.3 mg/kg/dose and 1 female administered 0.1 mg/kg/dose, and the ADA induction and titer values were greatly increased between Days 15 and 29. On Day 22, the exposures of all animals were decreased compared with those of the same animal on Day 1. Based on the exposures on Day 22 and the ADA results of MetHuG-CSF on Day 29, the decreased exposures on Day 22 may have been influenced by the ADA.

In sum, in the monkey repeated dose toxicity study, male and female cynomolgus monkeys were administered vehicle control article or 0.1, 0.3, or 1 mg/kg/dose P2203 via SC injection once weekly for 4 weeks (total five doses). One female administered 0.1 mg/kg/dose was sacrificed in a moribund condition on Day 29 of the dosing phase and was considered not P2203-related. P2203-related clinical pathology findings were generally dose-dependent, consistent with the pharmacologic effects of P2203, and reversible during recovery phase. P2203-related anatomic pathology findings were consistent with the expected pharmacology and considered non-adverse due to their low severity and were reversible during recovery phase. Due to the mild severity of findings and the lack of impact on the health and wellbeing of animals, the NOAEL was determined to be 1 mg/kg/dose.

### Example 9

### Overall pre-clinical summary

Overall, the in vitro pharmacology study shows that P2203 binds to human GCSFR specifically, with binding kinetics comparable to Filgrastim RS and MetHuG-CSF. The *in vivo* pharmacology study shows that P2203 and Neulasta generally elicited significantly higher WBC and ANC values compared to MetHuG-CSF and demonstrated a notable capability to extend their therapeutic effects over time, surpassing the efficacy observed with MetHuG-CSF. Notably, it was observed that P2203 demonstrated a surprising and superior capacity to stimulate WBC and ANC production compared to pegfilgrastim at the same dose levels with an extended duration of therapeutic effects.

No clinically relevant findings in safety pharmacology endpoints that examined the potential effects of P2203 on the CV system, respiratory system, and CNS were noted.

The PK data from the rat study showed linear exposures (Cₘₐₓ and AUCₗₐₛₜ) of P2203 for the IV route at dose levels of 300 and 1000 mcg/kg. In the monkey PK study, it showed a nonlinear PK, with Cₘₐₓ and AUC₀₋ₗₐₛₜ increasing more than in a dose-proportional manner for SC injection of P2203 from 0.1 mg/kg to 1 mg/kg. In general, the P2203 exposures increased as dose levels increased when P2203 was administered via IV injection in rats or SC injection in monkeys, and P2203 demonstrated higher in AUC₀₋ₗₐₛₜ values than pegfilgrastim.

In repeated dose toxicity studies, P2203-related clinical pathology findings were generally dose-dependent, consistent with the pharmacologic effects of P2203, and reversible during recovery phase. P2203-related anatomic pathology findings were consistent with the expected pharmacology and considered nonadverse. No other P2203-related adverse effects were observed in the two toxicity studies. The NOAEL was determined to be 1 mg/kg/dose for rats and cynomolgus monkeys.

Collectively, these nonclinical studies demonstrate that P2203 exhibits a high binding affinity to the GCSF Receptor. *In vivo* efficacy was observed in rats and monkeys with a linear PK profile noted in rats and a nonlinear PK profile in monkeys.

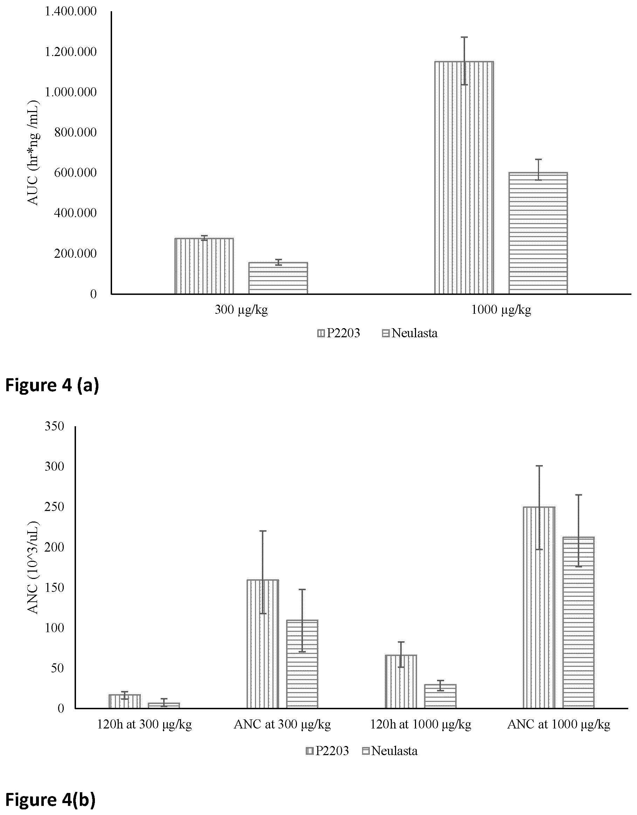

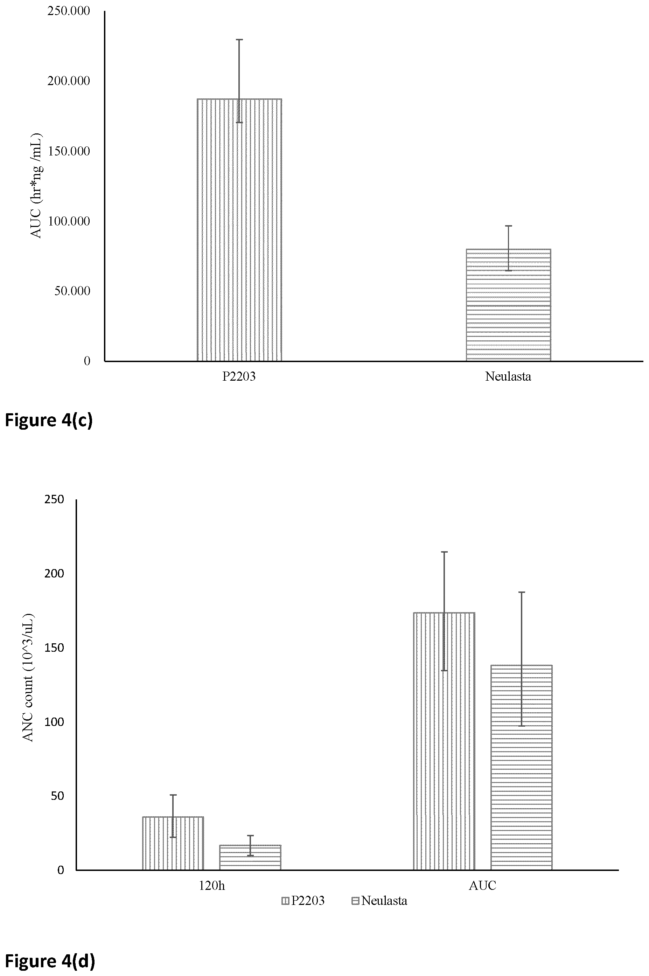

A graphical summary of P2203 and Pegfilgrastim (Neulasta, trademarked) can be seen in Figure 4, where the PK in rats, PD in rats, and PD in rats and monkey comparison are plotted out. In particular, Figure 6(a) denotes comparison of PK study in rats between P2203 and Neulasta (trademarked). Figure 4(b) denotes comparison of PD study in rats between P2203 and Neulasta (trademarked). Figure 4(c) denotes comparison of PK study in monkeys between P2203 and Neulasta (trademarked) using dosage at 300 µg/kg. Figure 4(d) denotes comparison of PD study in monkeys between P2203 and Neulasta (trademarked) using dosage at 300µg/kg.

### Example 10

### P2203 Clinical Evaluation

The safety variables evaluated include adverse events (AEs), serious adverse events (SAEs), clinical laboratory data, electrocardiogram (ECG) changes, physical examination findings, and lab examinations.

Characterizations of the pharmacokinetics (PK), pharmacodynamics (PD), and immunogenicity of P2203 are conducted. The PK parameters include, but not limited to Cₘₐₓ, Tₘₐₓ, λ_{z} (Kₑ), T_{½}, AUC₀₋ₜ, AUC. In addition, the PD profile of P2203 is to be evaluated by the measurement of white blood cell counts (WBC) and WBC differential (%) overtime in the blood. Further, the proportion of patients with positive result in the anti-drug antibody (ADA) measurement randomized, open-label, single-site, dose escalation study is to be evaluated to see the MTD, safety, tolerability, and PK/PD profile of P2203.

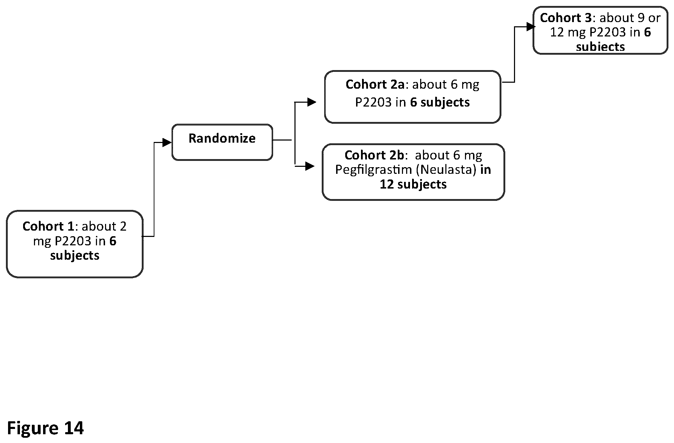

As denoted in the design of Figure 14, a total of 30 healthy volunteers are enrolled. Subjects or subjects in need are screened within 28 days before study dosing. Eligible subjects or subjects in need are sequentially enrolled into 3 escalating-dose cohorts (Cohort 1 to 3) to receive a single injection of P2203 at a pre-determined specific dose or to receive a single injection of 6 mg pegfilgrastim.

Patients are sequentially enrolled into the following inter-patient dose escalation cohorts: Cohort 1, Cohort 2, and Cohort 3. Subjects or subjects in need are to be treated with a single injection of P2203 or pegfilgrastim at Day 1 and receive a 28-day safety monitoring (from Day 1 to Day 29). Escalation to the next dose level follows the review by a Scientific Review Committee (SRC) on safety data from the lower doses during the 28-day safety monitoring period. A total of 6 subjects or subjects in need are to be enrolled in Cohort 1 to receive a single injection of P2203 at 2 mg. Of the 6 subjects or subjects in need in Cohort 1, two sentinel subjects or subjects in need are to be dosed at least 72 hours prior to the remaining members of this cohort. Procedures for sentinel subjects or subjects in need are to be the same for the other subjects.

Enrollment of Cohort 2 are to be initiated if no safety concerns are identified by the SRC review based on 28-day safety data of all Cohort 1 subjects or subjects in need. For Cohort 2, a total of 18 subjects or subjects in need are to be enrolled and randomized in a 1:2 ratio to receive a single injection of 6 mg P2203 (Cohort 2a, 6 subjects) or a single injection of 6 mg pegfilgrastim (Cohort 2b, 12 subjects). Enrollment of Cohort 3 is to be initiated if no safety concerns are identified by the SRC review based on 28-day safety data of Cohort 2a. A total of 6 subjects or subjects in need are to be enrolled in Cohort 3 to receive a single a single injection of about 9 or about 12 mg P2203. Subjects or subjects in need withdrawn prior to the completion of 28-day safety monitoring (prior to Day 29) are to be replaced. Participants are to visit the clinical sites as needed for screening evaluations. For each cohort, subjects or subjects in need are to be clinically confined from Day 1 until collection of the 72-hour blood sample at Day 4. Prolonged confinement due to non-safety factors is allowable and will not be classified as a SAE. Subjects or subjects in need are to return to the clinic for the 96, 120, 144, 168, 192, 240, 288, 336, 504, and 672 hours blood sampling and safety monitoring, and are to return for a safety follow-up visit at 35 days post drug administration.

### Pharmacokinetic Assessments

Sufficient samples of blood are to be taken in order to adequately define the maximum (Cₘₐₓ) and minimum (Cₘᵢₙ) blood concentrations. Samples of venous blood are to be obtained within one hour pre-dose administration (pre-dose), and at 1, 2, 3, 4, 6, 8, 12, 16, 24, 36, 48, 72, 96, 120, 144, 168, 192, 240, 288, 336, 504 and 672 hours after dose administration.

The following pharmacokinetic parameters are to be calculated: Cₘₐₓ, Tₘₐₓ, λz (Ke), T½, AUC₀₋ₜ, AUC.

### Pharmacodynamic Assessments

Blood samples for assessment of WBC and WBC differential (%) are to be obtained within one hour pre-dose and at 1, 2, 3, 4, 6, 8, 12, 16, 24, 36, 48, 72, 96, 120, 144, 168, 192, 240, 288, 336, 504 and 672 hours after dose administration.

### Immunogenic Assessments

Blood samples for immunogenicity testing are to be obtained within one hour pre-dose, and at 144, 336, and 672 hours after dose administration. A multi-tiered ADA testing approach are to include assays for the following: anti-P2203 binding antibodies and a confirmatory assay, antibody titers, and P2203 neutralizing antibodies. Anti-polyethylene glycol (PEG) are also to be measured.

Statistical analyses are to be reported using summary tables, graphs, and data listings. Continuous variables are to be summarized using mean, standard deviation, median, minimum, and maximum. Categorical variables are to be summarized by counts and by percentage of subjects or subjects in need in corresponding categories.

Results are to show that P2203 is safe and effective to accelerate recovery from chemotherapy induced neutropenia, for subjects or subjects in need with Chemotherapy-induced Febrile Neutropenia, Acute Myeloid Leukemia, subjects or subjects in need who Receive Bone Marrow Transplant, Peripheral Blood Progenitor Cell Collection and Engraftment, and/or subjects or subjects in need with Severe Chronic Neutropenia. P2203 can decrease the incidence of a subject's or subject in need's infection of any kind, as manifested by febrile neutropenia, in patients with non-myeloid malignancies receiving myelosuppressive anti-cancer drugs associated with a clinically significant incidence of febrile neutropenia; increase survival in subjects or subjects in need acutely exposed to myelosuppressive doses of radiation (Hematopoietic Subsyndrome of Acute Radiation Syndrome); mobilize hematopoietic stem cells; treat subjects or subjects in need who underwent transplantation of hematopoietic stem and/or progenitor cells (e.g., HSCT); treat neutropenia after chemotherapy, Acute myeloid leukemia, Myelodysplastic syndrome (MDS), and/or Aplastic anemia; treat persistent neutropenia in advanced HIV infection, and/or subjects or subjects in need with radiation-induced myelosuppression following a radiological/nuclear incident (H-ARS). P2203 can decrease the incidence of infection, as compared to FDA approved pegfilgrastim and/or its biosimilars, as manifested by febrile neutropenia, in individuals with non-myeloid malignancies receiving myelosuppressive anti-cancer drugs in one or more of the following categories: 1. Cancer individuals receiving myelosuppressive chemotherapy, 2. individuals with acute myeloid leukemia (AML) receiving induction or consolidation chemotherapy, 3. Cancer individuals receiving bone marrow transplantation, 4. individuals undergoing Peripheral Blood Progenitor Cell Collection and Therapy, 5. individuals with Severe Chronic Neutropenia, and/or 6. individuals who undergoes high-risk MDS (Myelodysplastic syndrome) and haematopoietic stem cell (HSC) transplantation (also known as post HSCT nertrophil engraftments).P2203 can also be used for cancer subjects or subjects in need using Myelosuppressive Chemotherapy, Acute Myeloid Leukemia, Bone Marrow Transplant, Progenitor Cell Collection, Severe Chronic Neutropenia, and/or Acute Radiation Syndrome, all associated with febrile neutropenia, and has surprisingly fewer, lesser, or subtler side effect as compared to FDA approved pegfilgrastim or any of its biosimilars. P2203 can also be used to treat neutropenia, a condition where the body makes too few white blood cells, decrease the incidence of infection, as manifested by febrile neutropenia, in subjects or subjects in need with non-myeloid malignancies receiving myelosuppressive anti-neoplastic drugs with surprisingly higher efficacy as compared to FDA approved pegfilgrastim and/or its biosimilars, decrease the incidence of infection, as manifested by febrile neutropenia, in subjects or subjects in need with non-myeloid malignancies receiving myelosuppressive anti-cancer drugs associated with a clinically significant incidence of febrile neutropenia, to increase survival in subjects or subjects in need acutely exposed to myelosuppressive doses of radiation (Hematopoietic Subsyndrome of Acute Radiation Syndrome) as compared to other FDA approved pegfilgrastim and/or its biosimilars. P2203 can also be provided in a single dose during a course of chemotherapy, and/or provided as multiple doses over the course of chemotherapy, or provide much greater dosing flexibility than is the case with the commercially available FDA approved pegfilgrastim and/or any of its biosimilars.

It is contemplated that any embodiment discussed in this specification can be implemented with respect to any method, kit, reagent, or composition of the disclosure, and vice versa. Furthermore, compositions of the disclosure can be used to achieve methods of the disclosure.

It will be understood that particular embodiments described herein are shown by way of illustration and not as limitations of the disclosure. The principal features of this disclosure can be employed in various embodiments without departing from the scope of the disclosure. Those skilled in the art will recognize, or be able to ascertain using no more than routine experimentation, numerous equivalents to the specific procedures described herein. Such equivalents are considered to be within the scope of this disclosure and are covered by the claims.

All publications and patent applications mentioned in the specification are indicative of the level of skill of those skilled in the art to which this disclosure pertains. All publications and patent applications are herein incorporated by reference to the same extent as if each individual publication or patent application was specifically and individually indicated to be incorporated by reference.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

All of the compositions and/or methods disclosed and claimed herein can be made and executed without undue experimentation in light of the present disclosure. While the compositions and methods of this disclosure have been described in terms of preferred embodiments, it will be apparent to those of skill in the art that variations may be applied to the compositions and/or methods and in the steps or in the sequence of steps of the method described herein without departing from the concept, spirit and scope of the disclosure. All such similar substitutes and modifications apparent to those skilled in the art are deemed to be within the spirit, scope and concept of the disclosure as defined by the appended claims.

Additional aspects and embodiments of the invention are disclosed below:
Embodiment 1. A substantially homogenous composition comprising a mPEG-MetHuG-CSF having a structure shown in formula I: 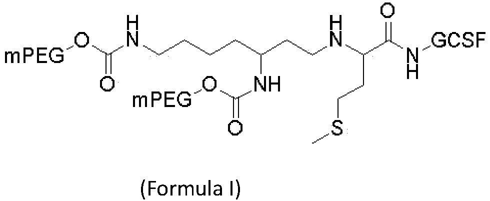
   wherein each mPEG includes a molecular weight of between about 19-23KDa; and
   wherein said composition includes a mixture of D- and L- form isomers of the mPEG-MetHuG-CSF.
Embodiment 2. The substantially homogenous composition according to embodiment 1, wherein said mixture includes at least about 95% D- form isomers.
Embodiment 3. The substantially homogenous composition according to embodiment 1, wherein said mixture includes at least about 98% D- form isomers.
Embodiment 4. The substantially homogenous composition according to embodiment 1, wherein said mixture includes at least about 99% D- form isomers.
Embodiment 5. The substantially homogenous composition according to embodiment 1, wherein said mixture includes at least about 99.5% or more D- form isomers.
Embodiment 6. A pharmaceutical formulation comprising
   the substantially homogenous composition according to embodiment 1; and
   a pharmaceutically acceptable carrier.
Embodiment 7. The substantially homogenous composition of embodiment 1, wherein the mPEG-MetHuG-CSF exhibits improved pharmacodynamics, pharmacokinetics, and/or toxicokinetic profile in *in vitro, in vivo,* and/or *ex vivo* as compared to filgrastim, pegfilgrastim, and/or their biosimilars.
Embodiment 8. The substantially homogenous composition of embodiment 7, wherein the biosimilars comprises pegfilgrastim-jmdb, pegfilgrastim-pbbk, pegfilgrastim-apgf, pegfilgrastim-cbqv, pegfilgrastim-bmez, pegfilgrastim-fpgk, and/or eflapegrastim-xnst.
Embodiment 9. A method of increasing white cell count in a subject or subject in need, or treating a condition in said subject or the subject in need thereof, wherein the condition includes compromised white blood cell production in said subject or said subject in need, the method comprising
   administering an effective amount of the substantially homogenous composition or pharmaceutical formulation according to any of the embodiments 1-8 to the subject or the subject in need.
Embodiment 10. The method according to embodiment 9, wherein the subject or the subject in need is at risk of and/or is suffering from neutropenia.
Embodiment 11. The method according to embodiment 9, wherein the subject or the subject in need is being treated with an agent, and/or is undergoing radiation and/or chemotherapy treatment that decreases white blood cell count.
Embodiment 12. The method according to embodiment 11, wherein administration of the substantially homogenous composition or the pharmaceutical formulation occurs on the same day or about the same time as the treatment with the agent, radiation and/or chemotherapy treatment.
Embodiment 13. The method according to embodiment 9, wherein the subject or the subject in need has a decreased endogenous level of GCSF.
Embodiment 14. The method according to embodiment 9, wherein the subject or the subject in need is suffering from one or more diseases including lung cancer, lymphoma, breast cancer, bone marrow transplantation, testicular cancer, AIDS-related malignancies, myleodysplastic disorders, myeloid cancer, acute leukemia, congenital and cyclic neutropenias, aplastic anemia, head and neck cancer, lung cancer, stomach cancer, colon cancer, pancreatic cancer, prostate cancer, breast cancer, kidney cancer, bladder cancer, ovary cancer, cervical cancer, melanoma, glioblastoma, myeloid leukemia, myeloma, lymphoma, and leukemia.
Embodiment 15. The method according to embodiment 9, wherein the subject or the subject in need is suffering from chronic and/or severe neutropenia, severe congenital neutropenia, cyclic neutropenia, febrile neutropenia, and/or severe combined neutropenia.
Embodiment 16. The method according to embodiment 9, the subject or the subject in need is at risk of and/or is suffering from an infection.
Embodiment 17. The method according to embodiments 9, wherein the substantially homogenous composition and/or pharmaceutical formulation is administered via an injection.
Embodiment 18. The method according to embodiment 17, wherein the injection is a subcutaneous injection.
Embodiment 19. A method of treating a hematopoietic disorder in a subject or the subject in need thereof, the method comprising:
   administering a therapeutically effective dose of the substantially homogenous composition or pharmaceutical formulation according to any of the embodiments 1-8 to the subject or the subject in need, wherein the method
   (a) alleviates, slows down, lessen symptoms of, and/or halts progression of the subject or the subject in need for the hematopoietic disorder including chronic, severe neutropenia, severe congenital neutropenia, cyclic neutropenia, febrile neutropenia, and/or severe combined neutropenia, as compared to one or more other pharmaceutical drugs; and/or
   (b) alleviates, slows down, lessen symptoms of, and/or halts progression of the subject or the subject in need who is at risk of and/or is suffering from neutropenia, as compared to one or more other pharmaceutical drugs.
Embodiment 20. The method according to embodiment 19, wherein said substantially homogenous composition or pharmaceutical formulation exhibits more potent efficacy of
   (a) alleviating, slowing down, lessening symptoms of, and/or halting progression of the hematopoietic disorder in the subject or the subject in need, as compared to one or more other pharmaceutical drugs; and/or
   (b) alleviates, slows down, lessen symptoms of, and/or halts progression of the subject or the subject in need who is at risk of and/or is suffering from neutropenia, as compared to one or more other pharmaceutical drugs.
Embodiment 21. The method according to embodiment 20, wherein said other pharmaceutical drugs comprise filgrastim, pegfilgrastim, pegfilgrastim-jmdb, pegfilgrastim-pbbk, pegfilgrastim-apgf, pegfilgrastim-cbqv, pegfilgrastim-bmez, pegfilgrastim-fpgk, and/or eflapegrastim-xnst.
Embodiment 22. The method according to embodiment 19, wherein the substantially homogenous composition or pharmaceutical formulation is administered as an injection.
Embodiment 23. The method according to embodiment 22, wherein the injection is injected subcutaneously.
Embodiment 24. A method for preparing a mPEG-MetHuG-CSF having a structure shown in Formula I 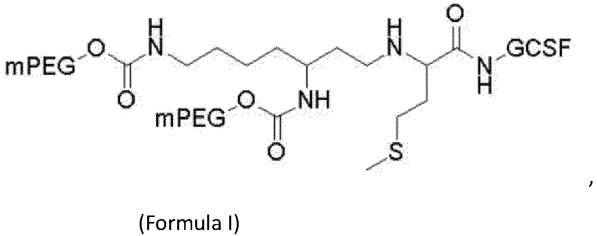 the method comprising:
   attaching covalently a branched aldehyde polymer including a pair of methoxy poly(ethylene glycol) (mPEG) molecules to the N-terminus of a MetHuG-CSF via amine linkage, wherein each mPEG molecule includes a molecular weight between about 19 to about 23 KDa, thereby obtaining mPEG-MetHuG-CSF.
Embodiment 25. The method according to embodiment 24, wherein each mPEG includes a molecular weight about 20 KDa.
Embodiment 27. The method of embodiment 24, wherein the step of attaching comprises
   covalently coupling the branched aldehyde polymer to the N-terminus of MetHuG-CSF under reducing alkylation conditions at a pH sufficiently acidic to activate the carbonyl group at the N terminus, thereby forming an amine bond; and the step further optionally comprises purifying and/or separating the mPEG-MetHuG-CSF from an unreacted MetHuG-CSF or any impurities.
Embodiment 28. A method of treating chemotherapy induced thrombocytopenia and/or chemotherapy induced neutropenia in a subject or a subject in need, the method comprising
   administering a therapeutically effective dose of the substantially homogenous composition or pharmaceutical formulation according to any of the embodiments 1-8 to the subject or the subject in need.
Embodiment 29. The method of embodiment 28, wherein said subject or the subject in need is suffering from myelosuppressive chemotherapy - induced thrombocytopenia and/or chemotherapy induced neutropenia.
Embodiment 30. The method of embodiment 29, wherein the myelosuppressive chemotherapy-induced thrombocytopenia is caused by a drug comprising Ziv-aflibercept, Brentuximab vedotin, Pralatrexate, Ganciclovir, Valganciclovir, Romidepsin, Ruxolitinib, Decitabine, Imatinib, Topotecan, Lenalidomide, Irinotecan, Interferons, Phenylhydrazine, Tamoxifen, Lipopolysaccharide, Anthracycline antibiotics, daunorubicin, doxorubicin, Gemcitabine, Cytoxan, Paclitaxel, Alkylating antineoplastic agent, DNA intercalating agent, Alkylating agent, bendamustin, mustard, Topoisomerase inhibitor, Bortezomib, Temsirolimus, Vorinostat, Ifosfamide, and/or Ixabepilone.
Embodiment 31. The method of embodiment 28, wherein the subject or the subject in need receives a chemotherapeutic agent comprising one or more of cyclophosphamide, doxorubicin, etoposide, ifosfamide, mesna, cisplatin, gemcitabine, tamoxifen and/or lenalidomide.
Embodiment 32. The method of embodiment 28, wherein the subject or the subject in need has multiple myeloma, chronic myelogenous leukemia (CML), acute myeloid leukemia, and/or myelodysplastic syndrome.
Embodiment 33. Use of the substantially homogenous composition or pharmaceutical formulation according to any of the embodiments 1-8 for the preparation of a medicament for the treatment of one or more conditions including compromised white blood cell production, decreased white blood cell count, decreased endogenous levels of GCSF, neutropenia, chronic and/or severe neutropenia, severe congenital neutropenia, cyclic neutropenia, febrile neutropenia, severe combined neutropenia, myelosuppressive chemotherapy induced thrombocytopenia, chemotherapy induced neutropenia, chemically induced neutropenia, multiple myeloma, chronic myelogenous leukemia (CML), acute myeloid leukemia, myelodysplastic syndrome, and/or alcoholic hepatitis.
Embodiment 34. The use of embodiment 33, wherein the one or more conditions is induced by a cancer therapy or another condition, wherein said cancer of cancer therapy or another condition comprises lung cancer, lymphoma, breast cancer, hematopoietic stem cell transplantation, bone marrow transplantation, testicular cancer, AIDS-related malignancies, myelodysplastic disorders, myeloid cancer, acute leukemia, aplastic anemia, head and neck cancer, lung cancer, stomach cancer, colon cancer, pancreatic cancer, prostate cancer, breast cancer, kidney cancer, bladder cancer, ovary cancer, cervical cancer, melanoma, glioblastoma, myeloid leukemia, myeloma, lymphoma, leukemia, myelosuppressive chemotherapy, acute myeloid leukemia, bone marrow transplant, progenitor cell collection, and/or acute radiation syndrome.
Embodiment 35. A substantially homogenous composition or pharmaceutical formulation according to any of the embodiments 1-8, for use in treating one or more of condition including compromised white blood cell production, decreased white blood cell count, decreased endogenous levels of GCSF, neutropenia, chronic and/or severe neutropenia, severe congenital neutropenia, cyclic neutropenia, febrile neutropenia, severe combined neutropenia, myelosuppressive chemotherapy induced thrombocytopenia, chemotherapy induced neutropenia, chemically induced neutropenia, multiple myeloma, chronic myelogenous leukemia (CML), acute myeloid leukemia, myelodysplastic syndrome, and/or alcoholic hepatitis.
Embodiment 36. The substantially homogenous composition or pharmaceutical formulation of embodiment 35, wherein the one or more of conditions is induced by a cancer therapy or another condition, wherein said cancer of cancer therapy or another condition includes lung cancer, lymphoma, breast cancer, hematopoietic stem cell transplantation, bone marrow transplantation, testicular cancer, AIDS-related malignancies, myelodysplastic disorders, myeloid cancer, acute leukemia, aplastic anemia, head and neck cancer, lung cancer, stomach cancer, colon cancer, pancreatic cancer, prostate cancer, breast cancer, kidney cancer, bladder cancer, ovary cancer, cervical cancer, melanoma, glioblastoma, myeloid leukemia, myeloma, lymphoma, leukemia, myelosuppressive chemotherapy, acute myeloid leukemia, bone marrow transplant, progenitor cell collection, and/or acute radiation syndrome.

## Claims

1. A substantially homogenous composition comprising a mPEG-MetHuG-CSF having a structure shown in formula I: 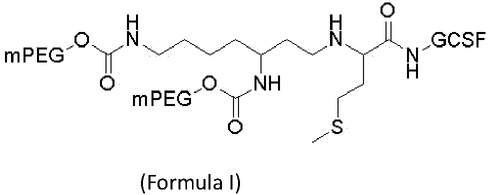 wherein each mPEG includes a molecular weight of between about 19-23KDa.

2. The substantially homogenous composition according to claim 1,
wherein said composition includes a mixture of D- and L- form isomers of the mPEG-MetHuG-CSF.

3. The substantially homogenous composition according to claim 2, wherein said composition comprises between at least about 95% to 99.5%, or more D- and L- form isomers.

4. A pharmaceutical formulation comprising
the substantially homogenous composition according to claim 1; and
a pharmaceutically acceptable carrier.

5. The substantially homogenous composition of claim 1, wherein the mPEG-MetHuG-CSF exhibits improved pharmacodynamics, pharmacokinetics, and/or toxicokinetic profile in *in vitro, in vivo,* and/or *ex vivo* as compared to filgrastim, pegfilgrastim, and/or their biosimilars.

6. The substantially homogenous composition of claim 5, wherein the biosimilars comprises pegfilgrastim-jmdb, pegfilgrastim-pbbk, pegfilgrastim-apgf, pegfilgrastim-cbqv, pegfilgrastim-bmez, pegfilgrastim-fpgk, and/or eflapegrastim-xnst.

7. A method for preparing a mPEG-MetHuG-CSF having a structure shown in Formula I 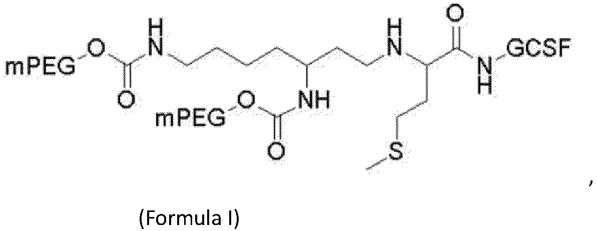 the method comprising:
(a) attaching covalently a branched aldehyde polymer including a pair of methoxy poly(ethylene glycol) (mPEG) molecules to the N-terminus of a MetHuG-CSF via amine linkage, wherein each mPEG molecule includes a molecular weight between about 19 to about 23 KDa, thereby obtaining mPEG-MetHuG-CSF; and
(b) covalently coupling the branched aldehyde polymer to the N-terminus of MetHuG-CSF under reducing alkylation conditions at a pH sufficiently acidic to activate the carbonyl group at the N terminus, thereby forming an amine bond.

8. The method according to claim 7, further comprising purifying and/or separating the mPEG-MetHuG-CSF from an unreacted MetHuG-CSF or any impurities.

9. A substantially homogenous composition according to claim 1, for use in treating one or more conditions comprising: compromised white blood cell production, decreased white blood cell count, decreased endogenous levels of GCSF, neutropenia, chronic and/or severe neutropenia, severe congenital neutropenia, cyclic neutropenia, febrile neutropenia, severe combined neutropenia, myelosuppressive chemotherapy induced thrombocytopenia, chemotherapy induced neutropenia, chemically induced neutropenia, multiple myeloma, chronic myelogenous leukemia (CML), acute myeloid leukemia, myelodysplastic syndrome, and/or alcoholic hepatitis.

10. The substantially homogenous composition for use according to claim 9, wherein the one or more conditions is induced by a cancer, a cancer therapy of said cancer, or another condition,
wherein said cancer, cancer therapy of said cancer, or another condition comprises: lung cancer, lymphoma, breast cancer, hematopoietic stem cell transplantation, bone marrow transplantation, testicular cancer, AIDS-related malignancies, myelodysplastic disorders, myeloid cancer, acute leukemia, aplastic anemia, head and neck cancer, lung cancer, stomach cancer, colon cancer, pancreatic cancer, prostate cancer, breast cancer, kidney cancer, bladder cancer, ovary cancer, cervical cancer, melanoma, glioblastoma, myeloid leukemia, myeloma, lymphoma, leukemia, myelosuppressive chemotherapy, acute myeloid leukemia, bone marrow transplant, progenitor cell collection, infection, and/or acute radiation syndrome.

11. The substantially homogenous composition for use according to claim 10, wherein the myelosuppressive chemotherapy-induced thrombocytopenia is caused by one or more drug comprising Ziv-aflibercept, Brentuximab vedotin, Pralatrexate, Ganciclovir, Valganciclovir, Romidepsin, Ruxolitinib, Decitabine, Imatinib, Topotecan, Lenalidomide, Irinotecan, Interferons, Phenylhydrazine, Tamoxifen, Lipopolysaccharide, Anthracycline antibiotics, daunorubicin, doxorubicin, Gemcitabine, Cytoxan, Paclitaxel, Alkylating antineoplastic agent, DNA intercalating agent, Alkylating agent, bendamustin, mustard, Topoisomerase inhibitor, Bortezomib, Temsirolimus, Vorinostat, Ifosfamide, and/or Ixabepilone.

12. The substantially homogenous composition for use according to claim 10, wherein said another condition comprises side effects of receiving one or more chemotherapeutic agent including one or more of: cyclophosphamide, doxorubicin, etoposide, ifosfamide, mesna, cisplatin, gemcitabine, tamoxifen and/or lenalidomide.

13. A pharmaceutical formulation according to claim 4, for use in treating one or more conditions comprising: compromised white blood cell production, decreased white blood cell count, decreased endogenous levels of GCSF, neutropenia, chronic and/or severe neutropenia, severe congenital neutropenia, cyclic neutropenia, febrile neutropenia, severe combined neutropenia, myelosuppressive chemotherapy induced thrombocytopenia, chemotherapy induced neutropenia, chemically induced neutropenia, multiple myeloma, chronic myelogenous leukemia (CML), acute myeloid leukemia, myelodysplastic syndrome, and/or alcoholic hepatitis.

14. The pharmaceutical formulation for use according to claim 13, wherein the myelosuppressive chemotherapy-induced thrombocytopenia is caused by one or more drug comprising Ziv-aflibercept, Brentuximab vedotin, Pralatrexate, Ganciclovir, Valganciclovir, Romidepsin, Ruxolitinib, Decitabine, Imatinib, Topotecan, Lenalidomide, Irinotecan, Interferons, Phenylhydrazine, Tamoxifen, Lipopolysaccharide, Anthracycline antibiotics, daunorubicin, doxorubicin, Gemcitabine, Cytoxan, Paclitaxel, Alkylating antineoplastic agent, DNA intercalating agent, Alkylating agent, bendamustin, mustard, Topoisomerase inhibitor, Bortezomib, Temsirolimus, Vorinostat, Ifosfamide, and/or Ixabepilone.

15. The pharmaceutical formulation for use according to claim 13, wherein the one or more conditions is induced by a caner, a cancer therapy of said cancer, or another condition,
wherein said cancer, cancer therapy of said cancer, or another condition comprises: lung cancer, lymphoma, breast cancer, hematopoietic stem cell transplantation, bone marrow transplantation, testicular cancer, AIDS-related malignancies, myelodysplastic disorders, myeloid cancer, acute leukemia, aplastic anemia, head and neck cancer, lung cancer, stomach cancer, colon cancer, pancreatic cancer, prostate cancer, breast cancer, kidney cancer, bladder cancer, ovary cancer, cervical cancer, melanoma, glioblastoma, myeloid leukemia, myeloma, lymphoma, leukemia, myelosuppressive chemotherapy, acute myeloid leukemia, bone marrow transplant, progenitor cell collection, infection, and/or acute radiation syndrome.

16. The pharmaceutical formulation for use according to claim 15, wherein said another condition comprises side effects of receiving one or more chemotherapeutic agent including: cyclophosphamide, doxorubicin, etoposide, ifosfamide, mesna, cisplatin, gemcitabine, tamoxifen and/or lenalidomide.
